# EUROPEAN PATENT APPLICATION

(11) **EP 1 746 102 A1**
(43) Date of publication of application: **24.01.2007**
(21) Application number: 05076632.8
(22) Date of filing: 15.07.2005
(51) Int. Cl.: C07K 14/085, C12N 15/41, C12N 7/00, A61K 39/125, C12N 15/11, G01N 33/53

(54) **Parechovirus, vaccines, medicaments and diagnostic kits**

(71) Applicant: PrimaGen Holding B.V., 1105 BA Amsterdam (NL)
(72) Inventor: Benschop, Kimberley, Samantha, Meriaha, 1183 AH Amstelveen (NL); Schinkel, Cornelia Johanna, 1011 LG Amsterdam (NL); Luken, Manon Elisabeth Maria, 1716 VL Opmeer (NL); Van den Broek, Petrus Johannes Maria, 3951 WJ Maarn (NL)
(74) Representative: Winckels, Johannes Hubertus F.

(57) **Abstract**

The invention provides a new parechoviral serotype called HPEV 251176, and functional parts, derivatives and analogues of said virus. Compounds capable of specifically binding HPEV 251176, such as isolated or recombinant antibodies and T cells, are also herewith provided. Immunogenic compositions, vaccines, pharmaceutical compositions and diagnostic kits comprising said virus, amino acid sequence or compound are also provided, as well as methods for producing same. A diagnostic kit of the invention may as well comprise a primer/probe capable of amplifying and/or hybridising at least part of the genome of HPEV 251176.

### Annex to the application documents - subsequently filed sequences listing

SEQUENCE LISTING

<110>  PrimaGen Holding B.V.

<120>  Parechovirus, vaccines, medicaments and diagnostic kits

<130>  P73570EP00

<140>  EP 05076632.8
<141>  2005-07-15

<160>  146

<170>  PatentIn version 3.3

<210>  1
<211>  24
<212>  DNA
<213>  Artificial

<220>
<223>  Primer A0013

<400>  1
gcyacacagc catcctctag taag                                              24

<210>  2
<211>  20
<212>  DNA
<213>  Artificial

<220>
<223>  Primer A0014

<400>  2
ggyttggccc rctagacgtt                                                   20

<210>  3
<211>  20
<212>  DNA
<213>  Artificial

<220>
<223>  Primer A0015

<220>
<221>  misc_feature
<222>  (10)..(10)
<223>  "N" stands for unknown nucleic acid

<400>  3
agcatccyan tgccagcgga                                                   20

<210>  4
<211>  22
<212>  DNA
<213>  Artificial

<220>
<223>  Primer A0016

<400>  4
gccccagatc agatccayag tg                                                22

<210>  5
<211>  18
<212>  DNA
<213>  Artificial

<220>
<223>  Primer VP1_echoF1

<400>  5
ccaaaattcr tggggttc                                                    18


<210>  6
<211>  20
<212>  DNA
<213>  Artificial

<220>
<223>  Primer VP1_echoR1

<400>  6
aaaccyctrt ctaaatawgc                                                  20


<210>  7
<211>  23
<212>  DNA
<213>  Artificial

<220>
<223>  Primer VP1_echoF0

<400>  7
ttytcmacwt ggatgaggaa rac                                              23


<210>  8
<211>  26
<212>  DNA
<213>  Artificial

<220>
<223>  Primer VP1_echoR0

<400>  8
ccwgtyaawg ctgtyttraa aatrtc                                           26


<210>  9
<211>  24
<212>  DNA
<213>  Artificial

<220>
<223>  Primer HPEVx 6522F

<400>  9
agattggcac ttyatgatta atgc                                             24


<210>  10
<211>  24
<212>  DNA
<213>  Artificial

<220>
<223>  Primer HPEVx 6545R

<400>  10
gcattaatca traagtgcca atct                                              24


<210>  11
<211>  26
<212>  DNA
<213>  Artificial

<220>
<223>  Primer HPEVx 6556F

<400>  11
tacaattatg aratggayta ytcyca                                            26


<210>  12
<211>  26
<212>  DNA
<213>  Artificial

<220>
<223>  Primer HPEVx 6581R

<400>  12
tgrgartart ccatytcata attgta                                            26


<210>  13
<211>  23
<212>  DNA
<213>  Artificial

<220>
<223>  Primer HPEVx 4951F


<220>
<221>  misc_feature
<222>  (18)..(18)
<223>  "N" stands for unknown nucleic acid

<400>  13
caaatggarg cyttyatnga acc                                               23


<210>  14
<211>  23
<212>  DNA
<213>  Artificial

<220>
<223>  Primer HPEVx 4861F


<220>
<221>  misc_feature
<222>  (9)..(9)
<223>  "N" stands for unknown nucleic acid

<400>  14
gatgcwgtnt cwtacatyaa gca                                               23


<210>  15
<211>  23
<212>  DNA

<213>  Artificial

<220>
<223>  Primer HPEVx 4883R


<220>
<221>  misc_feature
<222>  (15)..(15)
<223>  "N" stands for unknown nucleic acid

<400>  15
tgcttratgt awganacwgc atc                                              23


<210>  16
<211>  25
<212>  DNA
<213>  Artificial

<220>
<223>  Primer GSP1

<400>  16
tcagatccay agtgtcactt gttac                                            25


<210>  17
<211>  25
<212>  DNA
<213>  Artificial

<220>
<223>  Primer GSP2

<400>  17
gcatccttcg tgggccttac aacta                                            25


<210>  18
<211>  37
<212>  DNA
<213>  Artificial

<220>
<223>  Primer JZHoligodT

<400>  18
gctatcatca caatggactt tttttttttt ttttttv                               37


<210>  19
<211>  18
<212>  DNA
<213>  Artificial

<220>
<223>  Primer JZH1

<400>  19
gctatcatca caatggac                                                    18


<210>  20
<211>  617
<212>  DNA
<213>  Parechovirus

<400> 20
tgcactgaag attgcaaaca gactatttcc ccagatgaac tgggtctaac ttcagcccaa 60

gacgatggtc ctctgggagg tgaaaagcca aattacttct tgaattttag agctgtcaat 120

gttgacatat ttactgtgag tcacactaaa gtagacaaca tctttggtag ggcatggttt 180

gcatatgatc atacatatag agatgaagga acctggaggc aggctttgga tttcccaaag 240

aaaggccatg gtgccttaac ccaattattt gcctattact caggagaatt aaatatacat 300

gttttattct tgagtgaaac agggtttctg agagtggcac atacttatga cagtgataca 360

aacaggtctg acttcttctc ttcaaacggc gtcatcactg tgcccgcagg ggaacaaatg 420

accctgtcag taccattcta ctcttcaaag cccttgagga caatcaggga ttcagctgct 480

ctagggtatg tgatgtgtaa accattcatg tctgggacaa caggtggaaa gatagagata 540

tatctgagtt taagatgtcc aaacctattc tttcccttac cagctccgaa acctgcaact 600

agtcgtgctt tgcgggg 617

<210> 21
<211> 205
<212> PRT
<213> Parechovirus

<400> 21

Cys Thr Glu Asp Cys Lys Gln Thr Ile Ser Pro Asp Glu Leu Gly Leu
1               5                   10                  15

Thr Ser Ala Gln Asp Asp Gly Pro Leu Gly Gly Glu Lys Pro Asn Tyr
            20                  25                  30

Phe Leu Asn Phe Arg Ala Val Asn Val Asp Ile Phe Thr Val Ser His
        35                  40                  45

Thr Lys Val Asp Asn Ile Phe Gly Arg Ala Trp Phe Ala Tyr Asp His
    50                  55                  60

Thr Tyr Arg Asp Glu Gly Thr Trp Arg Gln Ala Leu Asp Phe Pro Lys
65                  70                  75                  80

Lys Gly His Gly Ala Leu Thr Gln Leu Phe Ala Tyr Tyr Ser Gly Glu
                85                  90                  95

Leu Asn Ile His Val Leu Phe Leu Ser Glu Thr Gly Phe Leu Arg Val
            100                 105                 110

Ala His Thr Tyr Asp Ser Asp Thr Asn Arg Ser Asp Phe Phe Ser Ser
        115                 120                 125

Asn Gly Val Ile Thr Val Pro Ala Gly Glu Gln Met Thr Leu Ser Val
    130                 135                 140

Pro Phe Tyr Ser Ser Lys Pro Leu Arg Thr Ile Arg Asp Ser Ala Ala

145                 150                 155                 160

Leu Gly Tyr Val Met Cys Lys Pro Phe Met Ser Gly Thr Thr Gly Gly
                165                 170                 175

Lys Ile Glu Ile Tyr Leu Ser Leu Arg Cys Pro Asn Leu Phe Phe Pro
            180                 185                 190

Leu Pro Ala Pro Lys Pro Ala Thr Ser Arg Ala Leu Arg
        195                 200                 205

<210>  22
<211>  848
<212>  DNA
<213>  Parechovirus

<400>  22
agattggcac tttatgatta atgccctgaa tgactacaat tatgaaatgg attactctca      60

gtatgatggt tcccttagtt caatgttact gtgggaggct gtggaggttt tggcctattg     120

tcatgattca cctgatcttg ttatgcaatt acacaaacca gtaattgact cggaccatgt     180

ggtcttcaat gagagatggt taattcatgg cggtatgcca tcaggatctc catgtaccac     240

tgtgttaaat tcattgtgta atttgatgat gtgcatatat acaactaatt taattagccc     300

agggattgac tgtttaccaa ttgtttatgg tgatgatgtc atcttgtcac ttgataaaga     360

aatagaccca gagaaactgc agagtatcat ggcagattca tttggtgctg aagtaactgg     420

ttcccgcaag gatgagcctc cctcactaaa acccagattg gaggtggaat tcctgaagcg     480

taaacctggt tacttcccag agtccacatt catagtagga aaattggaca ctgaaaatat     540

gatacaacat ttaatgtgga tgaaaaattt cagcacattc aagcagcaac tccagtccta     600

cttaatggag ttatgcctcc atggaaaaga cacttactta cactacatca aaattttgga     660

tccatacctg aaagaatgga atataacagt ggatgattat gatgttgtca ttgctaagtt     720

gatgcccatg gtgtttgact aaaattaatg ttttggtttt tctttgttat ggactatatc     780

attagaacac taattaaatt agaacactaa tttggaatat tggacatacg aaaaaaaaaa     840

aaaaaaaa                                                              848

<210>  23
<211>  246
<212>  PRT
<213>  Parechovirus

<400>  23

Asp Trp His Phe Met Ile Asn Ala Leu Asn Asp Tyr Asn Tyr Glu Met
1               5                   10                  15

Asp Tyr Ser Gln Tyr Asp Gly Ser Leu Ser Ser Met Leu Leu Trp Glu
            20                  25                  30

Ala Val Glu Val Leu Ala Tyr Cys His Asp Ser Pro Asp Leu Val Met

35                  40                  45

Gln Leu His Lys Pro Val Ile Asp Ser Asp His Val Val Phe Asn Glu
    50                  55                  60

Arg Trp Leu Ile His Gly Gly Met Pro Ser Gly Ser Pro Cys Thr Thr
65                  70                  75                  80

Val Leu Asn Ser Leu Cys Asn Leu Met Met Cys Ile Tyr Thr Thr Asn
                85                  90                  95

Leu Ile Ser Pro Gly Ile Asp Cys Leu Pro Ile Val Tyr Gly Asp Asp
            100                 105                 110

Val Ile Leu Ser Leu Asp Lys Glu Ile Asp Pro Glu Lys Leu Gln Ser
        115                 120                 125

Ile Met Ala Asp Ser Phe Gly Ala Glu Val Thr Gly Ser Arg Lys Asp
    130                 135                 140

Glu Pro Pro Ser Leu Lys Pro Arg Leu Glu Val Glu Phe Leu Lys Arg
145                 150                 155                 160

Lys Pro Gly Tyr Phe Pro Glu Ser Thr Phe Ile Val Gly Lys Leu Asp
                165                 170                 175

Thr Glu Asn Met Ile Gln His Leu Met Trp Met Lys Asn Phe Ser Thr
            180                 185                 190

Phe Lys Gln Gln Leu Gln Ser Tyr Leu Met Glu Leu Cys Leu His Gly
        195                 200                 205

Lys Asp Thr Tyr Leu His Tyr Ile Lys Ile Leu Asp Pro Tyr Leu Lys
    210                 215                 220

Glu Trp Asn Ile Thr Val Asp Asp Tyr Asp Val Val Ile Ala Lys Leu
225                 230                 235                 240

Met Pro Met Val Phe Asp
                245

<210>  24
<211>  866
<212>  DNA
<213>  Parechovirus

<400>  24
atggagacaa tcaagggtat tgcagatatg gcaactggta ttaccaagac tattgattct      60

acagttaaca ctgttaatga gaccattacc aaccccaaca atgctgctgg tggggatatg     120

cttacaaaag tggctgatga tgcttcaaat gtgttaggac caaactgttt tgcaayaaca     180

tcaacaccgg agaacaagga cgttgtccaa gcaacgacta ctgttaatac aacaaatttg     240

acacaacacc cttctgcccc aactatgcct tttaccccag atttccgtaa tgtagacatg 300

tttcactcaa tggcttatga cgtcaccaca ggagacaaga atcctagtaa attgattagg 360

ttaaggacag aattctggga acgcacatat gcaagacaac atttgatagt ggatgtagaa 420

ctccctcatg ccttttggga caatactaac aaaccagcat atggacaatc ccggtatttc 480

gcagccgtca ggtgcggttt ccattttcaa gtacaggtga atgttaacca aggaacagca 540

ggaagtgctt tagttgtgta tgaaccaaaa ccagctgtta cccacagtga taagatggag 600

tttggagcct ttaccaattt accacatgta ctcatgaatt tggctgaaac aacgcaagct 660

gatttatgta tcccctatgt gtcagacaca aactatgtta aaacagattc gtctgaccta 720

gggcgtttaa gagtgtatgt gtgggctcca ctaacaatac catctggagc aactagtact 780

gttgatgtga ctgtgttagg aagcttgtta caattagatt tccaaaaccc cagagtctat 840

aacacccaag ttgatatata cgacaa 866

<210> 25
<211> 289
<212> PRT
<213> Parechovirus

<400> 25

Met Glu Thr Ile Lys Gly Ile Ala Asp Met Ala Thr Gly Ile Thr Lys
1               5                   10                  15

Thr Ile Asp Ser Thr Val Asn Thr Val Asn Glu Thr Ile Thr Asn Pro
            20                  25                  30

Asn Asn Ala Ala Gly Gly Asp Met Leu Thr Lys Val Ala Asp Asp Ala
        35                  40                  45

Ser Asn Val Leu Gly Pro Asn Cys Phe Ala Thr Thr Ser Thr Pro Glu
    50                  55                  60

Asn Lys Asp Val Val Gln Ala Thr Thr Thr Val Asn Thr Thr Asn Leu
65                  70                  75                  80

Thr Gln His Pro Ser Ala Pro Thr Met Pro Phe Thr Pro Asp Phe Arg
                85                  90                  95

Asn Val Asp Met Phe His Ser Met Ala Tyr Asp Val Thr Thr Gly Asp
            100                 105                 110

Lys Asn Pro Ser Lys Leu Ile Arg Leu Arg Thr Glu Phe Trp Glu Arg
        115                 120                 125

Thr Tyr Ala Arg Gln His Leu Ile Val Asp Val Glu Leu Pro His Ala
    130                 135                 140

Phe Trp Asp Asn Thr Asn Lys Pro Ala Tyr Gly Gln Ser Arg Tyr Phe

145                 150                 155                 160

Ala Ala Val Arg Cys Gly Phe His Phe Gln Val Gln Val Asn Val Asn
                165                 170                 175

Gln Gly Thr Ala Gly Ser Ala Leu Val Val Tyr Glu Pro Lys Pro Ala
            180                 185                 190

Val Thr His Ser Asp Lys Met Glu Phe Gly Ala Phe Thr Asn Leu Pro
        195                 200                 205

His Val Leu Met Asn Leu Ala Glu Thr Thr Gln Ala Asp Leu Cys Ile
    210                 215                 220

Pro Tyr Val Ser Asp Thr Asn Tyr Val Lys Thr Asp Ser Ser Asp Leu
225                 230                 235                 240

Gly Arg Leu Arg Val Tyr Val Trp Ala Pro Leu Thr Ile Pro Ser Gly
                245                 250                 255

Ala Thr Ser Thr Val Asp Val Thr Val Leu Gly Ser Leu Leu Gln Leu
            260                 265                 270

Asp Phe Gln Asn Pro Arg Val Tyr Asn Thr Gln Val Asp Ile Tyr Asp
        275                 280                 285

Asn

<210>  26
<211>  397
<212>  DNA
<213>  Parechovirus

<400>  26
gcycacacag ccatcctcta gtaagtttgt gagacgtctg gtgacgtgtg ggaacttatt      60

ggaaacaaca ttttgctgca aagcatccta ctgccagcgg aacaacacct ggtaacaggt     120

gcctctgggg ccaaaagcca aggtttaata gaccctttag gattggttct aaacctgaga     180

tgttgtggaa aatatttagt acctgctgac ctggtagtaa tacaaacact agttgtaagg     240

cccacgaagg atgcccagaa ggtacccgta gtaacaagtg acactatgga tctgatctgg     300

ggccaaccac ctctatcttg gtgggttggt taaaaaacgt ctagtgggcc aaacccaggg     360

gggatccctg gtttcctttt attttatcat atttaat                              397

<210>  27
<211>  289
<212>  PRT
<213>  Parechovirus

<400>  27

Met Glu Thr Ile Lys Gly Ile Ala Asp Met Ala Thr Gly Ile Thr Lys

1               5                   10                  15

Thr Ile Asp Ser Thr Val Asn Thr Val Asn Glu Thr Ile Thr Asn Pro
            20                  25                  30

Asn Asn Ala Ala Gly Gly Asp Met Leu Thr Lys Val Ala Asp Asp Ala
        35                  40                  45

Ser Asn Val Leu Gly Pro Asn Cys Phe Ala Thr Thr Ser Thr Pro Glu
    50                  55                  60

Asn Lys Asp Val Val Gln Ala Thr Thr Thr Val Asn Thr Thr Asn Leu
65                  70                  75                  80

Thr Gln His Pro Ser Ala Pro Thr Met Pro Phe Thr Pro Asp Phe Arg
                85                  90                  95

Asn Val Asp Met Phe His Ser Met Ala Tyr Asp Val Thr Thr Gly Asp
            100                 105                 110

Lys Asn Pro Ser Lys Leu Ile Arg Leu Arg Thr Glu Phe Trp Glu Arg
        115                 120                 125

Thr Tyr Ala Arg Gln His Leu Ile Val Asp Val Glu Leu Pro His Ala
    130                 135                 140

Phe Trp Asp Asn Thr Asn Lys Pro Ala Tyr Gly Gln Ser Arg Tyr Phe
145                 150                 155                 160

Ala Ala Val Arg Cys Gly Phe His Phe Gln Val Gln Val Asn Val Asn
                165                 170                 175

Gln Gly Thr Ala Gly Ser Ala Leu Val Val Tyr Glu Pro Lys Pro Ala
            180                 185                 190

Val Thr His Ser Asp Lys Met Glu Phe Gly Ala Phe Thr Asn Leu Pro
        195                 200                 205

His Val Leu Met Asn Leu Ala Glu Thr Thr Gln Ala Asp Leu Cys Ile
    210                 215                 220

Pro Tyr Val Ser Asp Thr Asn Tyr Val Lys Thr Asp Ser Ser Asp Leu
225                 230                 235                 240

Gly Arg Leu Arg Val Tyr Val Trp Ala Pro Leu Thr Ile Pro Ser Gly
                245                 250                 255

Ala Thr Ser Thr Val Asp Val Thr Val Leu Gly Ser Leu Leu Gln Leu
            260                 265                 270

Asp Phe Gln Asn Pro Arg Val Tyr Asn Thr Gln Val Asp Ile Tyr Asp

275                 280                 285

Asn

<210>  28
<211>  290
<212>  PRT
<213>  Parechovirus

<400>  28

Met Glu Thr Ile Lys Ser Ile Ala Asp Met Ala Thr Gly Phe Thr Asn
1               5                   10                  15

Thr Ile Asp Ser Thr Val Asn Ala Val Thr Glu Gly Val Ser Lys Ile
            20                  25                  30

Gly Asn Asp Ser Gly Gly Glu Ile Leu Thr Lys Val Ala Asp Asp Ala
        35                  40                  45

Ser Asn Leu Leu Gly Pro Asn Cys Val Ala Ser Thr Ser Gln Pro Glu
    50                  55                  60

Asn Lys Asp Val Val Gln Ala Thr Thr Thr Val Asn Thr Leu Thr Asn
65                  70                  75                  80

Leu Thr Gln His Pro Ser Ala Pro Thr Met Pro Phe Thr Pro Asp Phe
                85                  90                  95

Ser Asn Val Asp Val Phe His Ser Met Ala Tyr Asp Ile Thr Thr Gly
            100                 105                 110

Asp Lys Asn Pro Ser Lys Leu Ile Arg Leu Asp Thr Thr Thr Trp Gln
        115                 120                 125

His Thr Trp Pro Arg Gln His Leu Ile Asn Asp Val Glu Leu Pro Lys
    130                 135                 140

Ala Phe Trp Asp Lys Asn Ser Lys Pro Ala Tyr Gly Gln Ser Arg Tyr
145                 150                 155                 160

Phe Ala Ala Val Arg Cys Gly Phe His Phe Gln Val Gln Ile Asn Val
                165                 170                 175

Asn Gln Gly Thr Ala Gly Cys Ala Leu Val Val Tyr Glu Pro Lys Pro
            180                 185                 190

Ile Val Thr His Gly Gly His Leu Glu Phe Gly Ser Tyr Thr Asn Leu
        195                 200                 205

Pro His Val Leu Met Asn Leu Ala Glu Thr Thr Gln Ala Asp Leu Cys
    210                 215                 220

Ile Pro Tyr Val Ser Asp Thr Asn Tyr Val Lys Thr Asp Ser Ser Asp
225                 230                 235                 240

Leu Gly Arg Leu Arg Val Tyr Val Trp Thr Pro Leu Thr Ile Pro Ser
                245                 250                 255

Ser Ala Thr Asn Asp Val Asp Val Thr Val Leu Gly Ser Leu Leu Gln
            260                 265                 270

Leu Asp Phe Gln Asn Pro Arg Thr Tyr Asp Thr Asp Val Asn Ile Tyr
        275                 280                 285

Asp Asn
    290

<210>  29
<211>  289
<212>  PRT
<213>  Parechovirus

<400>  29

Met Glu Thr Ile Lys Ser Ile Ala Asp Met Ala Thr Gly Val Thr Lys
1               5                   10                  15

Thr Ile Asp Ala Thr Ile Asn Ser Val Asn Glu Ile Ile Thr Asn Thr
            20                  25                  30

Asp Asn Ala Ser Gly Gly Asp Ile Leu Thr Lys Val Ala Asp Asp Ala
        35                  40                  45

Ser Asn Ile Leu Gly Pro Asn Cys Tyr Ala Thr Thr Ser Glu Pro Glu
    50                  55                  60

Asn Lys Asp Val Val Gln Ala Thr Thr Thr Val Asn Thr Thr Asn Leu
65                  70                  75                  80

Thr Gln His Pro Ser Ala Pro Thr Leu Pro Phe Thr Pro Asp Phe Ser
                85                  90                  95

Asn Val Asp Thr Phe His Ser Met Ala Tyr Asp Thr Thr Thr Gly Ser
            100                 105                 110

Lys Asn Pro Asn Lys Leu Val Arg Leu Thr Thr His Ala Trp Ala Ser
        115                 120                 125

Thr Leu Gln Arg Gly His Gln Ile Asp His Val Asn Leu Pro Val Asp
    130                 135                 140

Phe Trp Asp Glu Gln Arg Lys Pro Ala Tyr Gly His Ala Lys Tyr Phe
145                 150                 155                 160

Ala Ala Val Arg Cys Gly Phe His Phe Gln Val Gln Val Asn Val Asn
                165                 170                 175

Gln Gly Thr Ala Gly Ser Ala Leu Val Val Tyr Glu Pro Lys Pro Val
            180                 185                 190

Val Asp Tyr Asp Lys Asp Leu Glu Phe Gly Ala Phe Thr Asn Leu Pro
        195                 200                 205

His Val Leu Met Asn Leu Ala Glu Thr Thr Gln Ala Asp Leu Cys Ile
    210                 215                 220

Pro Tyr Val Ala Asp Thr Asn Tyr Val Lys Thr Asp Ser Ser Asp Leu
225                 230                 235                 240

Gly Gln Leu Lys Val Tyr Val Trp Thr Pro Leu Ser Ile Pro Ser Gly
                245                 250                 255

Ser Ser Asn Gln Val Asp Val Thr Ile Leu Gly Ser Leu Leu Gln Leu
            260                 265                 270

Asp Phe Gln Asn Pro Arg Val Tyr Gly Gln Asn Val Asp Ile Tyr Asp
        275                 280                 285

Thr

<210>  30
<211>  289
<212>  PRT
<213>  Parechovirus

<400>  30

Met Glu Thr Ile Lys Ser Ile Ala Asp Met Ala Thr Gly Val Val Ser
1               5                   10                  15

Ser Val Asp Ser Thr Ile Asn Ala Val Asn Glu Lys Val Glu Ser Val
            20                  25                  30

Gly Asn Glu Ile Gly Gly Asn Leu Leu Thr Lys Val Ala Asp Asp Ala
        35                  40                  45

Ser Asn Ile Leu Gly Pro Asn Cys Phe Ala Thr Thr Ala Glu Pro Glu
    50                  55                  60

Asn Lys Asn Val Val Gln Ala Thr Thr Thr Val Asn Thr Thr Asn Leu
65                  70                  75                  80

Thr Gln His Pro Ser Ala Pro Thr Met Pro Phe Ser Pro Asp Phe Ser
                85                  90                  95

Asn Val Asp Asn Phe His Ser Met Ala Tyr Asp Ile Thr Thr Gly Asp
            100                 105                 110

Lys Asn Pro Ser Lys Leu Val Arg Leu Glu Thr His Glu Trp Thr Pro
        115                 120                 125

Ser Trp Ala Arg Gly Tyr Gln Ile Thr His Val Glu Leu Pro Lys Val
    130                 135                 140

Phe Trp Asp His Gln Asp Lys Pro Ala Tyr Gly Gln Ser Arg Tyr Phe
145                 150                 155                 160

Ala Ala Val Arg Cys Gly Phe His Phe Gln Val Gln Val Asn Val Asn
                165                 170                 175

Gln Gly Thr Ala Gly Ser Ala Leu Val Val Tyr Glu Pro Lys Pro Val
            180                 185                 190

Val Thr Tyr Asp Ser Lys Leu Glu Phe Gly Ala Phe Thr Asn Leu Pro
        195                 200                 205

His Val Leu Met Asn Leu Ala Glu Thr Thr Gln Ala Asp Leu Cys Ile
    210                 215                 220

Pro Tyr Val Ala Asp Thr Asn Tyr Val Lys Thr Asp Ser Ser Asp Leu
225                 230                 235                 240

Gly Gln Leu Lys Val Tyr Val Trp Thr Pro Leu Ser Ile Pro Thr Gly
                245                 250                 255

Ser Ala Asn Gln Val Asp Val Thr Ile Leu Gly Ser Leu Leu Gln Leu
            260                 265                 270

Asp Phe Gln Asn Pro Arg Val Phe Ala Gln Asp Val Asn Ile Tyr Asp
        275                 280                 285

Asn

<210>  31
<211>  289
<212>  PRT
<213>  Parechovirus

<400>  31

Met Glu Ser Ile Lys Asp Leu Val Asn Val Ala Thr Gly Ala Met Asp
1               5                   10                  15

Thr Leu Ser Leu Ser Asn Val Glu Thr Glu Val Asn Asn Ile Ile Ser
            20                  25                  30

Gly Asn Glu Val Gly Gly Glu Ile Ile Thr Lys Val Ala Asp Asp Ala

35                  40                  45

Ser Asn Leu Leu Gly Pro Asn Ser Phe Ala Thr Thr Ala Gln Pro Glu
    50                  55                  60

Asn Lys Asp Val Val Gln Ala Thr Thr Thr Val Asn Thr Thr Asn Leu
65                  70                  75                  80

Thr Gln His Pro Ser Ala Pro Thr Ile Pro Phe Thr Pro Asp Phe Lys
                85                  90                  95

Asn Val Asp Asn Phe His Ser Met Ala Tyr Asp Ile Thr Thr Gly Asp
            100                 105                 110

Lys Asn Pro Ser Lys Leu Ile Arg Leu Asp Thr Ala Ser Trp Gln Thr
        115                 120                 125

Ser Tyr Ser Arg Gln Tyr Glu Ile Thr Thr Val Glu Leu Pro Lys Ser
    130                 135                 140

Phe Trp Asp Asp Thr Arg Lys Pro Ala Tyr Gly Gln Ala Lys Tyr Phe
145                 150                 155                 160

Ala Ala Val Arg Cys Gly Phe His Phe Gln Val Gln Val Asn Val Asn
                165                 170                 175

Gln Gly Thr Ala Gly Ser Ala Leu Val Val Tyr Glu Pro Lys Pro Val
            180                 185                 190

Ile Asp Ser Arg Gln Tyr Leu Glu Phe Gly Ser Leu Thr Asn Leu Pro
        195                 200                 205

His Val Leu Met Asn Leu Ala Glu Thr Thr Gln Ala Asp Leu Cys Ile
    210                 215                 220

Pro Tyr Val Ala Asp Thr Asn Tyr Val Lys Thr Asp Ser Ser Asp Leu
225                 230                 235                 240

Gly Gln Leu Arg Val Tyr Val Trp Thr Pro Leu Ser Val Pro Thr Gly
                245                 250                 255

Ala Ser Asn Glu Val Asp Val Thr Val Met Gly Ser Leu Leu Gln Leu
            260                 265                 270

Asp Phe Gln Asn Pro Arg Pro Tyr Gly Glu Asp Val Asp Ile Tyr Asp
        275                 280                 285

Asn

<210>  32

<211>  259
<212>  PRT
<213>  Parechovirus

<400>  32

Met Ala Ala Ser Lys Met Asn Pro Val Gly Asn Leu Leu Ser Thr Val
1               5                   10                  15

Ser Ser Thr Val Gly Ser Leu Leu Gln Asn Pro Ser Val Glu Glu Lys
            20                  25                  30

Glu Met Asp Ser Asp Arg Val Ala Ala Ser Thr Thr Thr Asn Ala Gly
        35                  40                  45

Asn Leu Val Gln Ala Ser Val Ala Pro Thr Met Pro Val Lys Pro Asp
    50                  55                  60

Phe Lys Asn Thr Asp Asp Phe Leu Ser Met Ser Tyr Arg Ser Thr Thr
65                  70                  75                  80

Ala Pro Thr Asn Pro Thr Lys Met Val His Leu Ala His Gly Thr Trp
                85                  90                  95

Thr Thr Asn Gln His Arg Gln Ala Leu Val Ala Ser Ile Thr Leu Pro
            100                 105                 110

Gln Ala Phe Trp Pro Asn Gln Asp Phe Pro Ala Trp Gly Gln Ser Arg
        115                 120                 125

Tyr Phe Ala Ala Val Arg Cys Gly Phe His Ile Gln Val Gln Leu Asn
    130                 135                 140

Val Asn Ile Gly Ser Ala Gly Cys Leu Ile Ala Ala Tyr Met Pro Lys
145                 150                 155                 160

Thr Ala His Asp His Met Asn Thr Tyr Thr Phe Gly Ser Tyr Thr Asn
                165                 170                 175

Leu Pro His Val Leu Met Asn Ala Ala Thr Thr Ser Gln Ala Asp Leu
            180                 185                 190

Tyr Ile Pro Tyr Val Phe Asn His Asn Tyr Ala Arg Thr Asp Ser Asp
        195                 200                 205

Asp Leu Gly Gly Ile Tyr Ile Trp Val Trp Ser Ala Leu Thr Val Pro
    210                 215                 220

Ser Gly Ser Pro Thr Thr Val Asp Val Thr Ile Phe Gly Ser Leu Leu
225                 230                 235                 240

Asp Leu Asp Phe Gln Cys Pro Arg Pro Pro Gly Ala Asp Thr Val Ile
                245                 250                 255

Tyr Thr Gln

<210> 33
<211> 867
<212> DNA
<213> Parechovirus

<400> 33
atggagacaa tcaagggtat tgcagatatg gcaactggta ttaccaagac tattgattct 60

acagttaaca ctgttaatga gaccattacc aaccccaaca atgctgctgg tggggatatg 120

cttacaaaag tggctgatga tgcttcaaat gtgttaggac caaactgttt tgcaayaaca 180

tcaacaccgg agaacaagga cgttgtccaa gcaacgacta ctgttaatac aacaaatttg 240

acacaacacc cttctgcccc aactatgcct tttaccccag atttccgtaa tgtagacatg 300

tttcactcaa tggcttatga cgtcaccaca ggagacaaga atcctagtaa attgattagg 360

ttaaggacag aattctggga acgcacatat gcaagacaac atttgatagt ggatgtagaa 420

ctccctcatg ccttttggga caatactaac aaaccagcat atggacaatc ccggtatttc 480

gcagccgtca ggtgcggttt ccattttcaa gtacaggtga atgttaacca aggaacagca 540

ggaagtgctt tagttgtgta tgaaccaaaa ccagctgtta cccacagtga taagatggag 600

tttggagcct ttaccaattt accacatgta ctcatgaatt tggctgaaac aacgcaagct 660

gatttatgta tcccctatgt gtcagacaca aactatgtta aaacagattc gtctgaccta 720

gggcgtttaa gagtgtatgt gtgggctcca ctaacaatac catctggagc aactagtact 780

gttgatgtga ctgtgttagg aagcttgtta caattagatt tccaaaaccc cagagtctat 840

aacacccaag ttgatatata cgacaac 867

<210> 34
<211> 870
<212> DNA
<213> Parechovirus

<400> 34
atggagacaa tcaagagcat tgcagatatg gccactggct tcactaacac aattgattca 60

actgtcaacg ctgtaactga aggtgtatct aagattggaa atgattcggg tggtgagatt 120

cttacaaagg tagcagatga tgcttcaaac cttcttggac caaattgtgt tgcatccacc 180

tcacagcctg aaaataagga tgtagtacaa gcaactacca cagtcaacac actcacaaat 240

ttgacacaac atccttctgc tcccactatg cctttcactc cagatttttc caatgttgac 300

gtttttcact ccatggctta tgatataacc actggtgata agaaccctag taaacttatt 360

aggctagaca ccacaacctg gcaacacact tggcctagac agcatttaat aaatgacgta 420

gaattaccta aggcattctg ggacaaaaat agtaaacctg catacgggca gtccagatat 480

tttgctgctg tgaggtgtgg attccatttc caagtacaaa ttaatgtcaa tcaaggaaca 540

gctggttgtg cactggtagt ttatgaaccc aagcctatag taacccatgg tggccatcta 600

gaatttggat cgtatactaa cttaccacat gtattgatga atttggctga aaccactcaa 660

gctgacctat gtatccccta tgtttctgac acaaactatg ttaagactga ttcgtcagac 720

ctagggcgtt tgagggttta tgtttggaca ccactgacaa ttccatccag tgcgactaat 780

gatgtggatg tgacagtttt aggtagctta ttacaacttg atttccaaaa tccaagaact 840

tatgacacag atgtcaatat ctatgataac 870

<210> 35
<211> 867
<212> DNA
<213> Parechovirus

<400> 35
atggagacaa tcaagagcat tgcagatatg gcgaccggtg taactaaaac cattgatgcc 60

acaatcaatt ctgttaatga gatcatcact aacacagata atgcttcagg tggagatata 120

ttgactaaag ttgctgatga tgcttcaaat attttagggc ccaactgtta tgcgacaaca 180

tctgagccag aaaacaagga tgtggtgcaa gcaaccacca ctgtgaacac cactaatctg 240

acacagcacc catcagcacc aacgttacca tttacaccag acttttcgaa tgttgacacg 300

tttcattcaa tggcttatga tactacaact ggtagtaaga accctaataa gttagttagg 360

ttaacgacac atgcttgggc tagtacccta cagaggggtc atcagattga tcatgttaat 420

ctaccagttg acttctggga tgaacagagg aaaccagctt atggccatgc taaatatttt 480

gcagctgttc ggtgtggatt tcattttcaa gtacaggtca atgtgaatca gggaactgct 540

gggagtgctt tggtagtgta tgaaccaaag ccagtagttg attatgataa ggatttggaa 600

tttggagcat ttaccaattt accacatgtg ttaatgaact tggccgagac tacccaggcc 660

gacttatgta tcccctatgt tgcagataca aactatgtga agactgattc atctgactta 720

gggcaattga aagtttatgt gtggactccc cttagcattc catcaggctc atctaaccaa 780

gtggacgtga ctatattggg tagcttatta caattggatt tccaaaaccc aagggtgtat 840

gggcaaaatg ttgacattta cgataca 867

<210> 36
<211> 867
<212> DNA
<213> Parechovirus

<400> 36
atggagtcta tcaaggattt agtcaatgtt gctactggtg ctatggatac tctctccctg 60

tctaacgtgg aaactgaagt taataatatt atttctggta atgaagttgg tggggagata 120

ataactaaag tagcagatga tgcctccaat ttacttgggc caaacagttt tgcaaccaca 180

gctcaaccag agaacaagga tgtagtgcaa gctacaacca ctgtaaacac tacaaatttg 240

acccaacacc catcagctcc aactatccca ttcacaccag atttaaaaaa tgtggacaac 300

tttcattcaa tggcttatga tataactact ggagacaaga accccagtaa gcttatacga 360

ctggacacag cttcatggca gaccagttat tctaggcagt acgaaataac cactgtggaa 420

ttgcccaaat ctttttggga tgataccaga aagcccgcct atggccaagc caaatatttt 480

gctgcagtaa ggtgtggttt tcatttccaa gtccaggtta acgtcaacca aggaactgca 540

ggaagtgcat tggtggttta tgagcctaag ccggttatag attcaaggca atatttggaa 600

tttggttctt taaccaatct cccccatgtg ctgatgaatc tagctgagac cacccaggct 660

gacctatgca tcccctatgt tgcagacaca aactatgtta aaactgattc gtctgatcta 720

gggcaattgc gggtctatgt ttggacacct ctttcagtac ctacaggggc atctaatgag 780

gttgatgtca cagttatggg tagcttattg caattggatt ttcaaaaccc acgcccttat 840

ggtgaagatg tggatattta tgataat 867

<210> 37
<211> 867
<212> DNA
<213> Parechovirus

<400> 37
atggagtcta tcaaggattt agtcaatgtt gctactggtg ctatggatac tctttccttg 60

tctaatgtgg aaactgaagc taataatatt atttctggta atgaagttgg tggggagata 120

ataaccaaag tagcagatga tgcttccaat ttacttgggc caaacagttt tgcaaccaca 180

gctcaaccag agaacaaaga tgtagtgcaa gctacaacca ctgtaaacac cacgaatttg 240

acccaacacc catcagctcc aactatcccg ttcacaccag attttagaaa tgtggacaac 300

tttcattcaa tggcttatga tataactact ggagataaaa accccagtaa gcttataaga 360

ctggacacaa cttcatggca gactagttat actaggcagt acaaaataac cactgtggaa 420

ttgcccaaat ctttttggga tgatactaga aagcccgcct atggccaagc caaatatttt 480

gctgcggtaa ggtgtggttt tcatttccaa gtccaggtta acgtcaacca aggaactgca 540

ggaagtgcat tggtggttta tgagcctaag ccagttatag attcaaggca atatttggaa 600

tttggttctt taactaatct cccccatgtg ttgatgaatc tggctgagac cacccaggct 660

gacctatgca tcccctatgt tgcagacaca aactatgtta aaaccgattc gtctgatcta 720

gggcaattgc gggtctatgt ttggacacct ctttcagtac ctacaggggc ttctaatgag 780

gttgatgtca cagttatggg tagcttatta caattggatt ttcaaaaccc acgcccttat 840

ggtgaagatg tagaaattta tgataat 867

<210> 38
<211> 867
<212> DNA
<213> Parechovirus

<400> 38
atggagacaa ttaagagtat tgcagatatg gctactggtg ttgtaagttc tgttgattct 60

accatcaatg cagtcaatga aaaggtagaa agcgtcggta atgaaattgg aggtaatttg 120

ttaactaaag tagctgatga tgcttccaat atcctaggac caaattgttt tgcaacgact 180

gctgaaccag agaacaagaa tgtagttcaa gccactacta ctgttaatac taccaacttg 240

actcaacatc catcggcacc tacgatgccc ttttcaccag atttctccaa tgtcgacaat 300

tttcactcta tggcttatga catcacaact ggggacaaaa atcccagtaa attggttcgt 360

cttgaaaccc atgagtggac gccatcttgg gcaagaggat accagattac acatgtagaa 420

ttacctaaag ttttttggga tcaccaagac aagcccgcat atggacaatc acgctacttt 480

gcggctgtaa gatgtggctt tcactttcaa gtacaagtta atgtgaatca aggtactgca 540

ggcagtgcct tggtagtata tgaacctaaa ccagttgtca cttatgattc aaaactggaa 600

tttggagcct ttacaaatct tccacatgtt ttgatgaatt tggctgaaac cacacaggct 660

gacttatgta tcccctatgt tgctgacaca aactatgtca agaccgattc gtcagactta 720

gggcagttaa aagtctatgt ctggactcca ttgtcaatcc ccactggatc tgctaaccaa 780

gttgatgtca ccatactggg tagcttgttg caattagact tccaaaatcc aagggtattt 840

gctcaagatg tcaacatata tgataat 867

<210> 39
<211> 867
<212> DNA
<213> Parechovirus

<400> 39
atggagacaa tcaagggtat tgcagatatg gcaactggta ttaccaagac tattgattct 60

acagttaaca ctgttaatga gaccattacc aaccccaaca atgctgctgg tggggatatg 120

cttacaaaag tggctgatga tgcttcaaat gtgttaggac caaactgttt tgcaayaaca 180

tcaacaccgg agaacaagga cgttgtccaa gcaacgacta ctgttaatac aacaaatttg 240

acacaacacc cttctgcccc aactatgcct tttaccccag atttccgtaa tgtagacatg 300

tttcactcaa tggcttatga cgtcaccaca ggagacaaga atcctagtaa attgattagg 360

ttaaggacag aattctggga acgcacatat gcaagacaac atttgatagt ggatgtagaa 420

ctccctcatg ccttttggga caatactaac aaaccagcat atggacaatc ccggtatttc 480

gcagccgtca ggtgcggttt ccattttcaa gtacaggtga atgttaacca aggaacagca 540

ggaagtgctt tagttgtgta tgaaccaaaa ccagctgtta cccacagtga taagatggag 600

tttggagcct ttaccaattt accacatgta ctcatgaatt tggctgaaac aacgcaagct 660

gatttatgta tcccctatgt gtcagacaca aactatgtta aaacagattc gtctgaccta 720

gggcgtttaa gagtgtatgt gtgggctcca ctaacaatac catctggagc aactagtact 780

gttgatgtga ctgtgttagg aagcttgtta caattagatt tccaaaaccc cagagtctat 840

aacacccaag ttgatatata cgacaac 867

<210> 40
<211> 870
<212> DNA
<213> Parechovirus

<400> 40
atggagacaa tcaagagcat tgcagatatg gccactggct tcactaacac aattgattca 60

actgtcaacg ctgtaactga aggtgtatct aagattggaa atgattcggg tggtgagatt 120

cttacaaagg tagcagatga tgcttcaaac cttcttggac caaattgtgt tgcatccacc 180

tcacagcctg aaaataagga tgtagtacaa gcaactacca cagtcaacac actcacaaat 240

ttgacacaac atccttctgc tcccactatg cctttcactc cagatttttc caatgttgac 300

gtttttcact ccatggctta tgatataacc actggtgata agaaccctag taaacttatt 360

aggctagaca ccacaacctg gcaacacact tggcctagac agcatttaat aaatgacgta 420

gaattaccta aggcattctg ggacaaaaat agtaaacctg catacgggca gtccagatat 480

tttgctgctg tgaggtgtgg attccatttc caagtacaaa ttaatgtcaa tcaaggaaca 540

gctggttgtg cactggtagt ttatgaaccc aagcctatag taacccatgg tggccatcta 600

gaatttggat cgtatactaa cttaccacat gtattgatga atttggctga aaccactcaa 660

gctgacctat gtatccccta tgtttctgac acaaactatg ttaagactga ttcgtcagac 720

ctagggcgtt tgagggttta tgtttggaca ccactgacaa ttccatccag tgcgactaat 780

gatgtggatg tgacagtttt aggtagctta ttacaacttg atttccaaaa tccaagaact 840

tatgacacag atgtcaatat ctatgataac 870

<210> 41
<211> 654
<212> DNA
<213> Artificial

<220>
<223> Consensus VP0 cDNA sequence

<400> 41
atggagacaa tcaaggattg cagatatggc actggtacaa acattgattc acgtaacctg 60

tatgatcaaa atgtcggtgg gaatcttaca aagtgcgatg atgcttcaaa ttggaccaaa 120

tgtttgcaac tcaccgaaaa aggagtgtca agcaacacac gtaaacacaa atttgacaca 180

acaccttctg cccactatgc ctttacccag atttaatgtg acttttcact catggcttat 240

gataccacgg gaaagaacct agtaaatatt aggtaacact ggaaccactc agacacattt 300

atagagtaga atcctagctt tgggacaaat aaaaccgcat aggcatccgt atttgcgcgt 360

aggtgggttc catttcaagt acataatgta acaaggaaca gcgggtgctg tgttatgaac 420

caaccgtacc cagtgatgat ttggacttac aattaccaca tgtatatgaa tttggctgaa 480

acaccaagct gatatgtatc ccctatgttc gacacaaact atgttaaacg attcgtcgac 540

ctagggcgtt taggttatgt tggcccacta caatccatcg gcactattgt gatgtgacgt 600

ttaggagctt ttacaatgat ttccaaaacc agatatacac agtatattag aaac 654

<210> 42
<211> 203
<212> PRT
<213> Parechovirus

<400> 42

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Arg Ala Ala
        195                 200

<210>  43
<211>  203
<212>  PRT
<213>  Parechovirus

<400>  43

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Val
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Val His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Leu Met His Gly Thr Thr Arg Thr Thr
                165                 170                 175

Ala Glu Val Tyr Val Ser Leu Arg Cys Pro Asn Phe Phe Phe Pro Val
            180                 185                 190

Pro Ala Pro Lys Pro Thr Gly Ser Arg Ala Ile
        195                 200

<210>  44
<211>  204
<212>  PRT
<213>  Parechovirus

<400>  44

Ser Val Glu Asp Cys Lys Gln Thr Ile Thr Pro Thr Glu Leu Gly Leu
1               5                   10                  15

Thr Ser Ala Gln Asp Asp Gly Pro Leu Gly Asn Asp Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Ile Phe Gly Arg Ala Trp Phe Ala His Val His
    50                  55                  60

Asp Ile Ile Thr Val Pro Ala Gly Glu Gln Met Thr Leu Ser Val Pro

65                  70                  75                  80

Phe Tyr Ser Asn Lys Pro Leu Arg Thr Ile Arg Ser Ser Asn Ala Leu
                85                  90                  95

Gly Tyr Leu Leu Cys Lys Pro Leu Leu Thr Gly Thr Ser Ser Gly Arg
            100                 105                 110

Ile Glu Ile Phe Leu Ser Leu Arg Cys Pro Asn Phe Phe Phe Pro Leu
        115                 120                 125

Pro Ala Phe Thr Asn Asp Gly Leu Trp Arg Gln Glu Leu Lys Phe Pro
    130                 135                 140

Lys Glu Gly His Gly Ala Leu Ser Leu Leu Phe Ala Tyr Phe Thr Gly
145                 150                 155                 160

Glu Leu Asn Ile His Val Leu Phe Leu Ser Asp Arg Gly Phe Leu Arg
                165                 170                 175

Val Gly His Thr Tyr Asp Thr Glu Thr Asn Arg Thr Asn Phe Leu Ser
            180                 185                 190

Ser Ser Gly Pro Lys Pro Ala Thr Ser Lys Tyr Arg
        195                 200

<210>  45
<211>  203
<212>  PRT
<213>  Parechovirus

<400>  45

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Leu Met His Gly Thr Thr Arg Thr Thr
                165                 170                 175

Ala Glu Val Tyr Val Ser Leu Arg Cys Pro Asn Phe Phe Phe Pro Val
            180                 185                 190

Pro Ala Pro Lys Pro Thr Gly Ser Arg Ala Thr .
        195                 200

<210>  46
<211>  203
<212>  PRT
<213>  Parechovirus

<400>  46

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Leu Met His Gly Thr Thr Arg Thr Thr
                165                 170                 175

Ala Glu Val Tyr Val Ser Leu Arg Cys Pro Asn Phe Phe Phe Pro Val
            180                 185                 190

Pro Ala Pro Lys Pro Thr Gly Ser Arg Ala Thr
        195                 200

<210>   47
<211>   205
<212>   PRT
<213>   Parechovirus

<400>   47

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210> 48
<211> 205
<212> PRT
<213> Parechovirus

<400> 48

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Gly Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210> 49

<211>  205
<212>  PRT
<213>  Parechovirus

<400>  49

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>  50
<211>  205
<212>  PRT
<213>  Parechovirus

<400>  50

Asp Thr Glu Asn Cys Lys Gln Thr Met Ser Pro Asn Glu Leu Gly Leu
1               5                   10                  15

Thr Ser Ala Gln Asp Asp Gly Pro Leu Gly Gln Glu Lys Pro Asn Tyr
            20                  25                  30

Phe Leu Asn Phe Arg Ser Met Asn Val Asp Ile Phe Thr Val Ser His
        35                  40                  45

Thr Lys Val Asp Asn Leu Phe Gly Arg Ala Trp Phe Phe Met Glu His
    50                  55                  60

Thr Phe Thr Asn Glu Gly Gln Trp Arg Val Pro Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ser Glu Arg Gly Phe Leu Arg Val
            100                 105                 110

Ala His Thr Tyr Asp Thr Ser Asn Asp Arg Val Asn Phe Leu Ser Ser
        115                 120                 125

Asn Gly Val Ile Thr Val Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Ser Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Ser Ser Arg Ala Leu Arg
        195                 200                 205

<210> 51
<211> 203
<212> PRT
<213> Parechovirus

<400> 51

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Lys Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155.                160

Gly Phe Leu Met Cys Arg Pro Ser Met His Gly Thr Thr Arg Thr Thr
                165                 170                 175

Val Glu Val Tyr Val Ser Leu Arg Cys Pro Asn Phe Phe Phe Pro Val
            180                 185                 190

Pro Ala Pro Lys Pro Thr Gly Ser Arg Ala Thr
        195                 200

<210>   52
<211>   203
<212>   PRT
<213>   Parechovirus

<400>   52

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25          .       30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Ala Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile

85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Arg Ala Ala
        195                 200

<210>  53
<211>  205
<212>  PRT
<213>  Parechovirus

<400>  53

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>   54
<211>   205
<212>   PRT
<213>   Parechovirus

<400>   54

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  55
<211>  205
<212>  PRT
<213>  Parechovirus

<400>  55

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Ser Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Gly Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Ser Arg Ala Leu Arg
        195                 200                 205

<210> 56
<211> 205
<212> PRT
<213> Parechovirus

<400> 56

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210> 57
<211> 203
<212> PRT
<213> Parechovirus

<400> 57

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Ala
        195                 200

<210> 58
<211> 203
<212> PRT
<213> Parechovirus

<400> 58

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Arg Ala Ala
        195                 200

<210>   59
<211>   203
<212>   PRT
<213>   Parechovirus

<400>   59

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Ala
        195                 200

<210>  60
<211>  203
<212>  PRT
<213>  Parechovirus

<400>  60

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala

            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Val
        195                 200

<210> 61
<211> 203
<212> PRT
<213> Parechovirus

<400> 61

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Val
        195                 200

<210>   62
<211>   203
<212>   PRT
<213>   Parechovirus

<400>   62

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Ala
        195                 200

<210>  63
<211>  203
<212>  PRT
<213>  Parechovirus

<400>  63

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Arg Ala Val
        195                 200

<210>  64
<211>  203
<212>  PRT
<213>  Parechovirus

<400>  64

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Asp Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Ala
        195                 200

<210>  65
<211>  203
<212>  PRT
<213>  Parechovirus

<400>  65

Met Glu Asn Cys Lys Gln Ser Ile Ser Pro Asn Glu Leu Gly Leu Thr
1               5                   10                  15

Ser Ala Gln Asp Asp Gly Pro Leu Gly Asn Glu Lys Pro Asn Tyr Phe
            20                  25                  30

Leu Asn Phe Arg Thr Met Asn Val Asp Ile Phe Thr Val Ser His Thr
        35                  40                  45

Lys Val Asp Asn Ile Phe Gly Arg Ala Trp Tyr Val Thr Ser His Asp
    50                  55                  60

Phe Asn Asn Gly Asp Thr Trp Arg Gln Lys Leu Thr Phe Pro Lys Glu
65                  70                  75                  80

Gly His Gly Met Leu Ser Gln Phe Phe Ala Tyr Phe Thr Gly Glu Ile
                85                  90                  95

Asn Ile His Ile Leu Tyr Met Ala Glu Gln Gly Phe Leu Arg Val Ala
            100                 105                 110

His Thr Tyr Asp Thr Glu Asp Asn Arg Lys Thr Phe Leu Ser Ser Asn
        115                 120                 125

Gly Val Ile Thr Ile Pro Ala Gly Glu Gln Met Thr Leu Ser Val Pro
    130                 135                 140

Phe Tyr Ser Asn Lys Pro Leu Arg Thr Val Arg His Glu Ser Ala Leu
145                 150                 155                 160

Gly Phe Leu Met Cys Arg Pro Met Met His Gly Thr Thr Arg Thr Thr
                165                 170                 175

Ala Glu Val Tyr Ile Ser Leu Arg Cys Pro Asn Phe Phe Phe Pro Val
            180                 185                 190

Pro Ala Pro Lys Pro Thr Gly Ser Gly Ala Ala
        195                 200

<210>   66
<211>   205
<212>   PRT
<213>   Parechovirus

<400>   66

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Val Leu Arg
        195                 200                 205

<210>  67
<211>  205
<212>  PRT
<213>  Parechovirus

<400>  67

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>   68
<211>   205
<212>   PRT
<213>   Parechovirus

<400>   68

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser

115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Ser Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>   69
<211>   205
<212>   PRT
<213>   Parechovirus

<400>   69

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Phe Gln Glu His
    50                  55                  60

Thr Phe Thr Asn Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Ser Arg Ala Leu Arg
        195                 200                 205

<210>  70
<211>  205
<212>  PRT
<213>  Parechovirus

<400>  70

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210> 71
<211> 205
<212> PRT
<213> Parechovirus

<400> 71

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210> 72
<211> 205
<212> PRT
<213> Parechovirus

<400>  72

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Ile Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  73
<211>  205
<212>  PRT
<213>  Parechovirus

<400>  73

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Arg Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asn Arg Val Asp Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  74
<211>  205
<212>  PRT
<213>  Parechovirus

<400>  74

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Phe Lys Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>  75
<211>  205
<212>  PRT
<213>  Parechovirus

<400>  75

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Thr Leu Arg
        195                 200                 205

<210> 76
<211> 205
<212> PRT
<213> Parechovirus

<400> 76

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala

130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>  77
<211>  205
<212>  PRT
<213>  Parechovirus

<400>  77

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Asn Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210> 78
<211> 205
<212> PRT
<213> Parechovirus

<400> 78

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asp Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Ile Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>  79
<211>  205
<212>  PRT
<213>  Parechovirus

<400>  79

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Ser Arg Ala Leu Arg
        195                 200                 205

<210>  80
<211>  205
<212>  PRT
<213>  Parechovirus

<400>  80

Ser Ala Glu Glu Cys Lys Gln Thr Ile Ser Pro Asn Glu Leu Gly Leu

1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Arg Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  81
<211>  205
<212>  PRT
<213>  Parechovirus

<400>  81

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Arg Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  82
<211>  205
<212>  PRT
<213>  Parechovirus

<400>  82

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>   83
<211>   205
<212>   PRT
<213>   Parechovirus

<400>   83

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>  84
<211>  205
<212>  PRT
<213>  Parechovirus

<400>  84

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Ser Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser

145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Ser Arg Ala Leu Arg
        195                 200                 205

<210>  85
<211>  205
<212>  PRT
<213>  Parechovirus

<400>  85

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Ser Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asp Glu Gly Gln Trp Arg Val Ser Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Ala Asp Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Ser Arg Ala Leu Arg
        195                 200                 205

<210>  86
<211>  205
<212>  PRT
<213>  Parechovirus

<400>  86

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Arg Gly Phe Leu Arg Val
            100                 105                 110

Ala His Thr Tyr Asp Thr Ser Ala Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  87
<211>  205
<212>  PRT

<213>  Parechovirus

<400>  87

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Glu Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Ser Thr Ser Arg Ala Leu Arg
        195                 200                 205

<210>  88
<211>  205
<212>  PRT
<213>  Parechovirus

<400>  88

Ser Ala Glu Glu Cys Lys Gln Thr Ile Ser Pro Asn Glu Leu Gly Leu
1               5                   10                  15

Thr Ser Ala Gln Asp Asp Gly Pro Leu Gly Asp Asn Lys Pro Asn Tyr

20                  25                  30

Phe Leu Asn Phe Lys Ser Met Asn Val Asp Ile Phe Thr Val Ser His
        35                  40                  45

Thr Lys Val Asp Asn Leu Phe Gly Arg Ala Trp Phe Tyr Gln Glu His
    50                  55                  60

Thr Phe Thr Asn Glu Gly Gln Trp Arg Val Asn Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ala Gly Lys Gly Phe Leu Arg Val
            100                 105                 110

Ala His Thr Tyr Asp Thr Ser Glu Asn Arg Val Asn Phe Leu Ser Ser
        115                 120                 125

Asn Gly Val Ile Thr Ile Pro Ala Gly Glu Gln Met Thr Leu Ser Ala
    130                 135                 140

Pro Tyr Tyr Ser Asn Lys Pro Leu Arg Thr Val Arg Asp Ser Asn Ser
145                 150                 155                 160

Leu Gly Tyr Leu Met Cys Lys Pro Phe Leu Thr Gly Thr Thr Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Thr Gly Arg Ala Leu Arg
        195                 200                 205

<210>   89
<211>   205
<212>   PRT
<213>   Parechovirus

<400>   89

Asp Thr Glu Asn Cys Lys Gln Thr Met Ser Pro Asn Glu Leu Gly Leu
1               5                   10                  15

Thr Ser Ala Gln Asp Asp Gly Pro Leu Gly Gln Glu Lys Pro Asn Tyr
            20                  25                  30

Phe Leu Asn Phe Arg Ser Met Asn Val Asp Ile Phe Thr Val Ser His
        35                  40                  45

Thr Lys Val Asp Asn Leu Phe Gly Arg Ala Trp Phe Phe Met Glu His
    50                  55                  60

Thr Phe Thr Asn Glu Gly Gln Trp Arg Val Pro Leu Glu Phe Pro Lys
65                  70                  75                  80

Gln Gly His Gly Ser Leu Ser Leu Leu Phe Ala Tyr Phe Thr Gly Glu
                85                  90                  95

Leu Asn Ile His Val Leu Phe Leu Ser Glu Arg Gly Phe Leu Arg Val
            100                 105                 110

Ala His Thr Tyr Asp Thr Ser Asn Asp Arg Val Asn Phe Leu Ser Ser
        115                 120                 125

Asn Gly Val Ile Thr Val Pro Ala Gly Glu Gln Met Thr Leu Ser Ala
    130                 135                 140

Pro Tyr Tyr Ser Asn Lys Pro Leu Arg Thr Val Arg Asp Asn Asn Ser
145                 150                 155                 160

Leu Gly Tyr Leu Met Cys Lys Pro Phe Leu Thr Gly Thr Ser Thr Gly
                165                 170                 175

Lys Ile Glu Val Tyr Leu Ser Leu Arg Cys Pro Asn Phe Phe Phe Pro
            180                 185                 190

Leu Pro Ala Pro Lys Val Thr Ser Ser Arg Ala Leu Arg
        195                 200                 205

<210>   90
<211>   204
<212>   PRT
<213>   Parechovirus

<400>   90

Ser Val Glu Asp Cys Lys Gln Thr Ile Thr Pro Thr Glu Leu Gly Leu
1               5                   10                  15

Thr Ser Ala Gln Asp Asp Gly Pro Leu Gly Asn Asp Lys Pro Asn Tyr
            20                  25                  30

Phe Leu Asn Phe Lys Ser Met Asn Val Asp Ile Phe Thr Val Ser His
        35                  40                  45

Thr Lys Val Asp Asn Ile Phe Gly Arg Ala Trp Phe Ala His Val His
    50                  55                  60

Asp Phe Thr Asn Asp Gly Leu Trp Arg Gln Gly Leu Glu Phe Pro Lys
65                  70                  75                  80

Glu Gly His Gly Ala Leu Ser Leu Leu Phe Ala Tyr Phe Thr Gly Glu
                85                  90                  95

Leu Asn Ile His Val Leu Phe Leu Ser Asp Arg Gly Phe Leu Arg Val
            100                 105                 110

Gly His Thr Tyr Asp Thr Glu Thr Asn Arg Thr Asn Phe Leu Ser Ser
        115                 120                 125

Ser Gly Ile Ile Thr Val Pro Ala Gly Glu Gln Met Thr Leu Ser Val
    130                 135                 140

Pro Ser Tyr Ser Asn Lys Pro Leu Arg Thr Val Arg Ser Ser Asn Ala
145                 150                 155                 160

Leu Gly Tyr Leu Leu Cys Lys Pro Leu Leu Thr Gly Thr Ser Ser Gly
                165                 170                 175

Arg Ile Glu Ile Phe Leu Ser Leu Arg Cys Pro Asn Phe Phe Phe Pro
            180                 185                 190

Leu Pro Ala Pro Lys Pro Ala Thr Arg Lys Tyr Arg
        195                 200

<210> 91
<211> 205
<212> PRT
<213> Parechovirus

<400> 91

Glu Ala Glu Asp Cys Lys Gln Thr Ile Ser Pro Asp Glu Leu Gly Leu
1               5                   10                  15

Thr Ser Ala Gln Asp Asp Gly Pro Leu Gly Val Glu Lys Pro Asn Tyr
            20                  25                  30

Phe Leu Asn Phe Arg Ala Ile Asn Val Asp Ile Phe Thr Val Ser His
        35                  40                  45

Thr Lys Val Asp Asn Ile Phe Gly Arg Ala Trp Leu Ala Leu Glu His
    50                  55                  60

Thr Phe Ala Asp Asp Gly Thr Trp Arg Ala Asp Leu Asn Phe Pro Thr
65                  70                  75                  80

Gln Gly His Gly Thr Leu Thr Arg Leu Phe Thr Tyr Tyr Ser Gly Glu
                85                  90                  95

Leu Asn Val His Val Leu Tyr Leu Ser Asp Asn Gly Phe Leu Arg Val
            100                 105                 110

Thr His Ala Tyr Asp His Asp Asn Asp Arg Ser Asn Phe Leu Ser Ser
        115                 120                 125

Asn Gly Val Ile Thr Val Pro Ala Gly Glu Gln Met Thr Leu Ser Val
    130                 135                 140

Pro Phe Tyr Ser Ser Lys Pro Leu Arg Thr Ile Arg Glu Thr Gly Ala
145                 150                 155                 160

Leu Gly Lys Leu Ile Cys Lys Pro Leu Leu Ser Gly Thr His Ser Gly
                165                 170                 175

Lys Ile Glu Val Tyr Leu Ser Leu Arg Cys Pro Asn Leu Phe Phe Pro
            180                 185                 190

Ser Pro Ala Pro Lys Glu Lys Thr Ser Arg Ala Leu Arg
        195                 200                 205

<210>  92
<211>  205
<212>  PRT
<213>  Parechovirus

<400>  92

Cys Thr Glu Asp Cys Lys Gln Thr Ile Ser Pro Asp Glu Leu Gly Leu
1               5                   10                  15

Thr Ser Ala Gln Asp Asp Gly Pro Leu Gly Gly Glu Lys Pro Asn Tyr
            20                  25                  30

Phe Leu Asn Phe Arg Ala Val Asn Val Asp Ile Phe Thr Val Ser His
        35                  40                  45

Thr Lys Val Asp Asn Ile Phe Gly Arg Ala Trp Phe Ala Tyr Asp His
    50                  55                  60

Thr Tyr Arg Asp Glu Gly Thr Trp Arg Gln Ala Leu Asp Phe Pro Lys
65                  70                  75                  80

Lys Gly His Gly Ala Leu Thr Gln Leu Phe Ala Tyr Tyr Ser Gly Glu
                85                  90                  95

Leu Asn Ile His Val Leu Phe Leu Ser Glu Thr Gly Phe Leu Arg Val
            100                 105                 110

Ala His Thr Tyr Asp Ser Asp Thr Asn Arg Ser Asp Phe Phe Ser Ser
        115                 120                 125

Asn Gly Val Ile Thr Val Pro Ala Gly Glu Gln Met Thr Leu Ser Val
    130                 135                 140

Pro Phe Tyr Ser Ser Lys Pro Leu Arg Thr Ile Arg Asp Ser Ala Ala
145                 150                 155                 160

Leu Gly Tyr Val Met Cys Lys Pro Phe Met Ser Gly Thr Thr Gly Gly

165                 170                 175

Lys Ile Glu Ile Tyr Leu Ser Leu Arg Cys Pro Asn Leu Phe Phe Pro
            180                 185                 190

Leu Pro Ala Pro Lys Pro Ala Thr Ser Arg Ala Leu Arg
        195                 200                 205

<210>  93
<211>  115
<212>  PRT
<213>  Artificial

<220>
<223>  Consensus Parechovirus VP1 amino acid sequence

<400>  93

Glu Cys Lys Gln Pro Glu Leu Gly Leu Thr Ser Ala Gln Asp Asp Gly
1               5                   10                  15

Pro Leu Gly Lys Pro Asn Tyr Phe Leu Asn Phe Asn Val Asp Ile Phe
            20                  25                  30

Thr Val Ser His Thr Lys Val Asp Asn Phe Gly Arg Ala Trp His Trp
        35                  40                  45

Arg Leu Phe Pro Gly His Gly Leu Phe Tyr Gly Glu Asn His Leu Gly
    50                  55                  60

Phe Leu Arg Val His Tyr Asp Arg Phe Ser Ser Gly Ile Thr Pro Ala
65                  70                  75                  80

Gly Glu Gln Met Leu Ser Pro Tyr Ser Lys Pro Leu Arg Thr Arg Leu
                85                  90                  95

Gly Cys Pro Gly Thr Glu Ser Leu Arg Cys Pro Asn Phe Phe Pro Pro
            100                 105                 110

Ala Pro Lys
        115

<210>  94
<211>  611
<212>  DNA
<213>  Artificial

<220>
<223>  VP1 encoding PCR product of HPEV3CAN82853-01

<400>  94
atggaaaatt gtaaacagtc catatcacca aatgaattgg gtttgacttc agctcaagat      60

gatgggcctt tgggtaatga gaaaccaaat tattttttaa acttcagaac tatgaatgtt     120

gacattttta cagttagtca taccaaagtt gacaacatct ttggtagagc ttggtatgtg     180

acttctcatg attttaacaa tggagatacc tggaggcaga aattaacatt tccaaaagag 240

ggtcacggca tgttatcaca gttttttgct tattttacag gagaaataaa tattcatatc 300

ctatatatgg cagagcaggg gtttctcaga gtggctcata catatgacac tgaagataat 360

aggaaaacat tcttgtcttc aaatggggta ataactatcc ctgctggtga gcagatgaca 420

ctctcagtac ctttttattc aaacaagcct ctgaggacag tgcgccatga ttcagcatta 480

ggtttcttta tgtgtagacc aatgatgcac gggactactc gaactactgc agaagtttac 540

attagtttaa ggtgccccaa tttctttttc cctgtaccag ctcctaaacc aactggttca 600

agggctgcag c 611

<210> 95
<211> 611
<212> DNA
<213> Artificial

<220>
<223> VP1 encoding PCR product of HPEV3A628-99

<400> 95
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagtaagtca taccaaagtt gacaacatct ttggtagagc ttggtatgtg 180

acgtctcatg tctttaataa tggagatacc tggaggcaga aattaacatt tccaaaagag 240

ggtcatggta tgttatcaca gttttttgct tattttacag gagaaataaa tgttcatatc 300

ctatatatgg cagagcaggg gttccttaga gtggctcata catatgacac tgaagataat 360

aggaaaactt tcttgtcttc aaatggggta ataactatcc ctgctggtga gcagatgaca 420

ctctcagtac ctttttattc aaacaagcct ctgaggacag tgcgccatga ttcagcatta 480

gggtttctta tgtgtagacc attgatgcac gggactacac gaactactgc agaagtttat 540

gttagtttaa ggtgccccaa tttctttttc cctgtaccag ctcctaaacc aactggttca 600

agggctatag c 611

<210> 96
<211> 614
<212> DNA
<213> Artificial

<220>
<223> VP1 encoding PCR product of ECHO23INHOUSE

<400> 96
tcagtagaag attgtaagca aaccattaca ccaacagaat tgggactaac ctcagcacaa 60

gatgatggcc ctttaggaaa tgacaaacca aattattttc ttaactttaa gtctatgaat 120

gttgatatct ttactgtcag tcacaccaaa gtagacaata tttttggacg tgcttggttt 180

gcccatgttc atgacttcac taatgatggc ctatggagac aggaattgaa atttccaaag 240

gaagggcacg gtgccctatc acttctgttt gcctacttta ctggtgaatt aaacatccat 300

gttctatttc ttagtgatag gggttttctc agagttggac atacatatga cactgagaca 360

aacagaacca attttttatc atccagtggc ataattacag taccagcagg agaacagatg 420

acactatctg tcccctttta ttccaacaag ccattacgga caattagatc atccaatgct 480

ttaggttatt tactgtgtaa accattgcta actggtacca gctctggtag aatagagata 540

ttccttagct tgagatgtcc aaatttcttc tttcccttac cagcaccaaa accagcaaca 600

agtaaatata gagg 614

<210> 97
<211> 611
<212> DNA
<213> Artificial

<220>
<223> VP1 encoding PCR product of HPEV3A317-99

<400> 97
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagtaagtca taccaaagtt gacaacatct ttggtagagc ttggtatgtg 180

acgtctcatg actttaataa tggagatacc tggaggcaga aattaacatt tccaaaagag 240

ggtcatggta tgttatcaca gttttttgct tattttacag gagaaataaa tattcatatc 300

ctatatatgg cagagcaggg gttccttaga gtggctcata catatgacac tgaagataat 360

aggaaaactt tcttgtcttc aaatggggta ataactatcc ctgctggtga gcagatgaca 420

ctctcagtac ctttttattc aaacaagcct ctgaggacag tgcgccatga ttcagcatta 480

ggtttcttta tgtgtagacc attgatgcac gggactacac gaactactgc agaagtttat 540

gttagtttaa ggtgccccaa tttctttttc cctgtaccag ctcctaaacc aactggttca 600

agggctacag c 611

<210> 98
<211> 611
<212> DNA
<213> Artificial

<220>
<223> VP1 encoding PCR product of HPEV3A354-99

<400> 98
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagtaagtca taccaaagtt gacaacatct ttggtagagc ttggtatgtg 180

acgtctcatg actttaataa tggagatacc tggaggcaga aattaacatt tccaaaagag 240

ggtcatggta tgttatcaca gttttttgct tattttacag gagaaataaa tattcatatc 300

ctatatatgg cagagcaggg gttccttaga gtggctcata catatgacac tgaagataat 360

aggaaaactt tcttgtcttc aaatggggta ataactatcc ctgctggtga gcagatgaca 420

ctctcagtac ctttttattc aaacaagcct ctgaggacag tgcgccatga ttcagcatta 480

ggtttcttta tgtgtagacc attgatgcac gggactacac gaactactgc agaagtttat 540

gttagtttaa ggtgccccaa tttctttttc cctgtaccag ctcctaaacc aactggttca 600

agggctacag c 611

<210> 99
<211> 617
<212> DNA
<213> Artificial

<220>
<223> VP1 encoding PCR product of 054330

<400> 99
tctgcagagg aatgcaaaca gaccatatcg ccaaatgaat tgggcttaac atcagctcag 60

gatgatggac cattgggcga taacaagcca aattatttcc taaatttcaa gtccatgaat 120

gtggatatct tcactgtttc tcacactaag gtagacaacc tatttggaag agcttggttt 180

taccaggaac acactttcac cgatgaaggg cagtggagag tcagtttaga gttcccgaaa 240

caaggccatg gttcgctttc attgttattc gcttacttca caggtgaact aaatattcat 300

gttctgttcc tagctgaaag aggatttctt agagtagccc acacctatga cacatcagca 360

aacagagtta acttcctgtc atccaatggc gtcatcacaa tcccagctgg agaacaaatg 420

acattatctg caccctacta ctcaaataaa cccctcagaa cagtcagaga taacaatagt 480

cttgggtatc taatgtgtaa accattcctc actggaacga caacagggaa gatagaggtt 540

tatcttagtt tgagatgtcc aaattttttc tttcctcttc ccgcacctaa ggtttcaact 600

agccgtgctt tgcgggg 617

<210> 100
<211> 617
<212> DNA
<213> Artificial

<220>
<223> VP1 encoding PCR product of HPEV1A10987-00

<400> 100
tctgcagagg aatgtaaaca gaccatatca ccaaacgaat tgggcttgac atcagctcag 60

gatgatggac cattgggcga taacaagcca aattatttcc tgaatttcaa atccatgaat 120

gtggacattt tcactgtttc tcacactaag gtagacaacc tatttggaag agcttggttt 180

taccaggaac acactttcac caatgaagga cagtggagag ttaatttaga gtttccaaaa 240

caaggccatg gttcgctttc cttgctattc gcttatttca caggtgaatt aaatattcat 300

gttttgttcc tggctggaaa gggattcctt agagtagctc acacctacga cacatcagaa 360

aacagggtta acttcttgtc atccaatggt gttatcacaa tcccagcagg agaacaaatg 420

acactatctg caccttacta ctcaaataaa cccctcagaa cagttaggga cagcaatagt 480

cttgggtatc taatgtgtaa accattcctc actggaacaa caacagggaa aatagaggtc 540

tatcttagtt tgagatgtcc aaatttcttc tttcctcttc ccgcacctaa agttacaact 600

ggtcgtgctt tgcgggg 617

<210> 101
<211> 617
<212> DNA
<213> Artificial

<220>
<223> VP1 encoding PCR product of HPEV1A1086-99

<400> 101
tctgcagagg aatgtaaaca aaccatatca ccaaatgaat tgggtttaac gtcagctcag 60

gatgatggac cactgggcga taacaagcca aattatttcc taaattttaa atccatgaat 120

gtggacattt tcactgtttc tcacactaag gtagacaacc tatttggaag agcttggttt 180

taccaggaac acactttcac caatgaagga cagtggagag ttaatttaga gtttccaaaa 240

caaggccatg gttcgctttc cttactgttc gcttatttca caggtgaatt gaatatccat 300

gttttgttcc tggctgaaaa gggattcctt agagtagctc acacctacga cacatcagaa 360

aacagggtta actttttgtc atccaatggt gttatcacaa tcccagcagg agaacaaatg 420

acactatctg caccatacta ctcaaataaa cccctcagaa cagttaggga cagcaatagt 480

cttgggtatc tgatgtgtaa accattcctc actggaacaa caacagggaa aatagaagtt 540

tatcttagtt tgaggtgtcc aaattttttc tttcctcttc ccgcacctaa agttaccact 600

ggtcgtgctt tgcgggg 617

<210> 102
<211> 617
<212> DNA
<213> Artificial

<220>
<223> VP1 encoding PCR product of HPEV1HARRIS

<400> 102
gacacagaaa attgcaaaca aacaatgtct ccaaatgaac taggactcac ttcagcccaa 60

gatgatggcc cacttggtca agaaaagcca aattattttc tcaattttag gtcgatgaat 120

gtggacattt ttactgtatc acatactaaa gtagataacc tatttgggcg ggcatggttt 180

tttatggagc atactttcac caatgaggga caatggagag tgccattgga atttccaaaa 240

caaggtcatg ggtccttatc actgttgttt gcttatttta ctggtgaact gaatatccat 300

gttctgttcc taagtgagag ggggtttctg agggttgcac acacatatga cactagtaat 360

gatcgagtca attttctgtc atcgaacggt gtaataactg taccagccgg agagcagatg 420

acactttcag ctccctacta ttcaaacaaa ccattaagaa ctgtcagaga taacaatagt 480

cttggttatt tgatgtgcaa gcccttcttg actggaacct ctactggtaa aattgaggtt 540

tatcttagcc tgagatgtcc aaatttcttt ttccctcttc ctgcccctaa ggttacgagt 600

agtcgtgcac tacgggg 617

<210> 103
<211> 614
<212> DNA
<213> Artificial

<220>
<223> VP1 encoding PCR product of HPEV2AWILLIAMSON

<400> 103
tcagtagaag attgtaagca aaccattaca ccaacagaat tgggactaac ctcagcacaa 60

gatgatggcc ctttaggaaa tgacaaacca aattattttc ttaactttaa gtctatgaat 120

gttgatatct ttactgtcag tcacaccaaa gtagacaata tttttggacg tgcttggttt 180

gcccatgttc atgacttcac taatgatggc ctatggagac agggattgga atttccaaag 240

gaagggcacg gtgccctatc acttctgttt gcctacttta ctggtgaatt aaacatccat 300

gttctatttc ttagtgatag gggttttctc agagttggac atacatatga cactgagaca 360

aacagaacca attttttatc atccagtggc ataattacag taccagcagg agaacagatg 420

acactatctg tcccctctta ttccaacaag ccattacgga cagttagatc atccaatgct 480

ttaggttatt tactgtgtaa accattgcta actggtacca gctctggtag aatagagata 540

ttccttagct tgagatgtcc aaatttcttc tttcccttac cagcaccaaa accagcaaca 600

cgtaaatata gagg 614

<210> 104
<211> 611
<212> DNA
<213> Artificial

<220>
<223> VP1 encoding PCR product of HPEV3A308-99

<400> 104
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagtaagtca taccaaagtt gacaacatct ttggtagagc ttggtatgtg 180

acgtctcatg actttaataa tggagatacc tggaggcaga aattaacatt tccaaaagag 240

ggtcatggta tgttatcaca gttttttgct tattttacag gagaaataaa tattcatatc 300

ctatatatgg caaagcaggg gttccttaga gtggctcata catatgacac tgaagataat 360

aggaaaactt tcttgtcttc aaatggggta ataactatcc ctgctggtga gcagatgaca 420

ctctcagtac ctttttattc aaacaagcct ctgaggacag tgcgccatga ttcagcatta 480

ggttttctta tgtgtagacc atcgatgcac gggactacac gaactactgt agaagtttat 540

gttagtttaa ggtgccccaa tttctttttc cctgtaccag ctcctaaacc aactggttca 600

agggctacag c 611

<210> 105
<211> 611
<212> DNA

<213>  Artificial

<220>
<223>  VP1 encoding PCR product of 152037

<400>  105
atggaaaatt gtaaacagtc catatcacca aatgaattgg gtttgacttc agctcaagat      60

gatgggcctt tgggtaatga gaaaccaaat tattttttaa acttcagaac tatgaatgtt      120

gacattttta cagttagtca taccaaggtt gacaacatct ttggtagagc ttggtatgtg      180

gcttctcatg attttaacaa tggagatacc tggaggcaaa aattaacatt tccaaaagag      240

ggtcatggca tgttatcaca gttttttgct tattttacag gagaaataaa tatccatatc      300

ctatatatgg cagagcaggg gtttcttaga gtggctcata catatgacac tgaagacaat      360

aggaaaacat tcttgtcttc aaatggagta ataactatcc ctgctggtga gcagatgaca      420

ctctcagtac ctttttattc aaacaagcct ctgaggacag tgcgccatga ttcagcatta      480

ggttttctta tgtgtagacc aatgatgcac gggactactc gaactactgc agaagtttat      540

attagtttaa ggtgccccaa tttctttttc cctgtaccag ctcctaaacc aactggttca      600

agggctgcag c                                                          611

<210>  106
<211>  617
<212>  DNA
<213>  Artificial

<220>
<223>  VP1 encoding PCR product of 450976

<400>  106
tctgcagaag aatgtaaaca gaccatatca ccaaatgaat tgggattaac atcagctcag      60

gatgatggac cattgggcga taacaagcca aattatttcc taaatttcaa gtctatgaat      120

gtagacatct tcactgtttc ccacactaag gtggacaact tatttggaag agcatggttc      180

taccaggaac acactttcac aaacgaagga cagtggagag ttaatttgga gttcccaaaa      240

caaggtcatg gttcactttc tttgctattt gcttatttta caggtgaatt aaacattcat      300

gttctgttct tagctgaaaa aggatttctt agagtagccc atacatacga cacatcagaa      360

aatagagtta acttcttgtc atctaatggt gttatcacaa tcccagcggg agaacaaatg      420

acattatctg caccttacta ctcaaacaag ccccttagga cagttaggga cagcaatagt      480

cttgggtatc tgatgtgcaa accattcctc actggaacaa caacagggaa aatagaggtc      540

taccttagcc tgagatgtcc aaatttcttc tttcctctcc ccgcacctaa agtgacaact      600

ggtcgtgctt tgcgggg                                                    617

<210>  107
<211>  617
<212>  DNA
<213>  Artificial

<220>
<223>  VP1 encoding PCR product of 152212

<400>  107
tctgcagagg aatgcaaaca gaccatatcg ccaaatgaat tgggcttaac atcagctcag      60

gatgatggac cattgggcga taacaagcca aattatttcc taaatttcaa gtccatgaat     120

gtggatatct tcactgtttc tcacactaag gtagacaacc tatttggaag agcttggttt     180

taccaggaac acactttcac caatgaaggg cagtggagag tcagtttaga gttcccgaaa     240

caaggccatg gttcgctttc attgttattc gcttacttca caggtgaact aaatattcat     300

gttctgttcc tagctgaaag aggatttctt agagtagccc acacctatga cacatcagca     360

aacagagtta acttcctgtc atccaatggc gtcatcacaa tcccagctgg agaacaaatg     420

acattatctg caccctacta ctcaaataaa cccctcagaa cagtcagaga taacaatagt     480

cttgggtatc taatgtgtaa accattcctc actggaacga caacagggaa gatagaggtt     540

tatcttagtt tgagatgtcc aaattttttc tttcctcttc ccgcacctaa ggtttcaact     600

agccgtgctt tgcgggg                                                    617

<210>  108
<211>  617
<212>  DNA
<213>  Artificial

<220>
<223>  VP1 encoding PCR product of 152329

<400>  108
tctgcagagg aatgcaaaca gaccatatcg ccaaatgaat tgggcttaac atcagctcag      60

gatgatggac cattgggcga taacaagcca aattatttcc taaatttcaa gtccatgaat     120

gtggatatct tcactgtttc tcacactaag gtagacaacc tatttggaag agcttggttt     180

taccaggaac acactttcac cgatgaaggg cagtggagag tcagtttaga gttcccgaaa     240

caaggccatg gttcgctttc attgttattc gcttacttca caggtgaact aaatattcat     300

gttctgttcc tagctgaaag aggatttctt agagtagccc acacctatga cacatcagca     360

aacagagtta acttcctgtc atccaatggc gtcatcacaa tcccagctgg agaacaaatg     420

acattatctg caccctacta ctcaaataaa cccctcagaa cagtcagaga taacaatagt     480

cttgggtatc taatgtgtaa accattcctc actggaacga caacagggaa gatagaggtt     540

tatcttagtt tgagatgtcc aaattttttc tttcctcttc ccgcacctaa ggtttcaact     600

agccgtgctt tgcgggg                                                    617

<210>  109
<211>  617
<212>  DNA
<213>  Artificial

<220>
<223>  VP1 encoding PCR product of 152478

<400>  109
tctgcagagg aatgtaaaca gactatatca ccaaacgaat tgggcttaac atcagcacaa      60

gatgacgggc cattgggtga tagcaagcca aattatttcc taaatttcaa gtccatgaat 120

gtggacatct tcactgtttc tcacactaaa gtagacaact tatttgggag agcctggttt 180

tatcaggaac acactttcac caatgaggga cagtggagag tcaatttaga gtttccaaaa 240

caaggccatg gttcgctttc cttactattt gcttatttca caggtgaatt aaatattcat 300

gttttattcc tagctggaaa aggatttctt agagtagccc acacctacga cacatcagaa 360

aacagggtta acttcctgtc gtccaatggt gttattacaa ttccagcagg agaacaaatg 420

acattgtctg caccttacta ttcaaataag cccctcagaa cagttagaga cagtaatagc 480

cttgggtatc taatgtgcaa accattcctc actggaacaa ctacagggaa aatagaagtc 540

tatcttagtt tgagatgtcc aaatttcttc tttcctcttc ctgcacctaa agttacaact 600

agtcgtgctt tgcgggg 617

<210> 110
<211> 617
<212> DNA
<213> Artificial

<220>
<223> VP1 encoding PCR product of 153231

<400> 110
tctgcagagg aatgcaaaca gactatatca ccaaatgaat tgggcttaac atcagctcag 60

gatgatggac cattgggcga taacaagcca aattatttcc taaatttcaa gtccatgaat 120

gtggacatct tcactgtttc tcacactaag gtagacaacc tatttggaag agcttggttt 180

taccaggaac acactttcac tgatgaaggg cagtggagag ttagtttaga gttcccgaag 240

caaggccatg gttcgctttc gttgttattt gcttacttca caggtgaact aaatattcat 300

gtactgtttc tagctgaaaa gggatttctt agagtagccc acacctacga cacatcagaa 360

aacagggtta acttcctgtc gtccaatggt gtcatcacaa tcccagctgg agaacaaatg 420

acattatctg caccctacta ctcaaataaa cccctcagaa cagtcagaga caacaatagt 480

cttgggtacc taatgtgtaa accattcctc actggaacga caacagggaa gatagaggtt 540

tatcttagtt tgagatgtcc aaatttcttc tttcctcttc ccgcacctaa ggtttcaact 600

agtcgtgctt tgcgggg 617

<210> 111
<211> 611
<212> DNA
<213> Artificial

<220>
<223> VP1 encoding PCR product of 251181

<400> 111
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ctatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggttttctta tgtgtagacc aatgatgcac gggactaccc gaactactgc agaagtttat 540

attagtttaa ggtgccccaa tttctttttc cctgtaccag ctcctaaacc aactggttca 600

ggggctgcag c 611

<210> 112
<211> 611
<212> DNA
<213> Artificial

<220>
<223> VP1 encoding PCR product of 251360

<400> 112
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ttatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggttttctta tgtgtagacc aatgatgcac gggactaccc gaactactgc agaagtttat 540

attagtttaa ggtgccccaa tttctttttc cctgtaccag ctcctaaacc aactggttca 600

agggctgcag c 611

<210> 113
<211> 611
<212> DNA
<213> Artificial

<220>
<223> VP1 encoding PCR product of 251407

<400> 113
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ctatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggtttcttta tgtgtagacc aatgatgcac gggactaccc gaactactgc agaagtttat 540

attagtttaa ggtgccccaa tttctttttc cctgtaccag ctcctaaacc aactggttca 600

ggggctgcag c 611

<210> 114
<211> 611
<212> DNA
<213> Artificial

<220>
<223> VP1 encoding PCR product of 450936

<400> 114
atggaaaact gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagggc ttggtatgtg 180

acttctcatg attttaacaa tggagacacc tggaggcaga aattaacatt tccaaaagag 240

ggtcatggca tgttgtcaca gttttttgct tattttacag gagaaataaa tattcatatc 300

ctatatatgg cagagcaggg gttccttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggtttcttta tgtgtagacc aatgatgcat gggaccaccc gaactactgc agaagtttat 540

attagtttaa ggtgccccaa tttctttttc cctgtaccag ctcctaaacc aactggttca 600

ggggctgtag c 611

<210> 115
<211> 611
<212> DNA
<213> Artificial

<220>
<223> VP1 encoding PCR product of 451517

<400> 115
atggaaaact gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagggc ttggtatgtg 180

acttctcatg attttaacaa tggagacacc tggaggcaga aattaacatt tccaaaagag 240

ggtcatggca tgttgtcaca gttttttgct tattttacag gagaaataaa tattcatatc 300

ctatatatgg cagagcaggg gttccttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcattg 480

ggttttctta tgtgtagacc aatgatgcat gggaccaccc gaactactgc agaagtttat 540

attagtttaa ggtgccccaa tttctttttc cctgtaccag ctcctaaacc aactggttca 600

ggggctgtag c 611

<210> 116
<211> 611
<212> DNA
<213> Artificial

<220>
<223> VP1 encoding PCR product of 251393

<400> 116
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ctatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggttttctta tgtgtagacc aatgatgcac gggactaccc gaactactgc agaagtttat 540

attagtttaa ggtgccccaa tttctttttc cctgtaccag ctcctaaacc aactggttca 600

ggggctgcag c 611

<210> 117
<211> 611
<212> DNA
<213> Artificial

<220>
<223> VP1 encoding PCR product of 451371

<400> 117
atggaaaact gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagggc ttggtatgtg 180

acttctcatg attttaacaa tggagacacc tggaggcaga aattaacatt tccaaaagag 240

ggtcatggca tgttgtcaca gttttttgct tattttacag gagaaataaa tattcatatc 300

ctatatatgg cagagcaggg gttccttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggtttcttta tgtgtagacc aatgatgcat gggaccaccc gaactactgc agaagtttat 540

attagtttaa ggtgccccaa tttctttttc cctgtaccag ctcctaaacc aactggttca 600

agggctgtag c 611

<210> 118
<211> 611
<212> DNA
<213> Artificial

<220>
<223> VP1 encoding PCR product of 250956

<400> 118
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ctatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga ttcagcatta 480

ggtttcttta tgtgtagacc aatgatgcac gggactaccc gaactactgc agaagtttat 540

attagtttaa ggtgccccaa tttctttttc cctgtaccag ctcctaaacc aactggttca 600

ggggctgcag c 611

<210> 119
<211> 611
<212> DNA
<213> Artificial

<220>
<223> VP1 encoding PCR product of 252277

<400> 119
atggaaaatt gtaaacagtc catatcacca aatgaattgg gtttgacttc agctcaagat 60

gatgggcctt tgggtaatga gaaaccaaat tattttttaa acttcagaac tatgaatgtt 120

gacattttta cagttagtca taccaaagtt gacaacatct ttggtagagc ttggtatgtg 180

acttctcatg attttaacaa tggagatacc tggaggcaga aattaacatt tccaaaagag 240

ggtcatggca tgttgtcaca gttttttgct tattttacag gagaaataaa tattcatatc 300

ctatatatgg cagagcaggg gtttcttaga gtggctcata catatgacac tgaagataat 360

aggaaaacat tcttgtcttc aaatggggta ataactatcc ccgctggtga gcagatgaca 420

ctctcagtac ctttttattc aaacaagcct ctgaggacag tgcgccatga gtcagcatta 480

ggtttcttta tgtgtagacc aatgatgcac gggactaccc gaactactgc agaagtttat 540

attagtttaa ggtgccccaa tttctttttc cctgtaccag ctcctaaacc aactggttca 600

ggggctgcag c 611

<210>  120
<211>  617
<212>  DNA
<213>  Artificial

<220>
<223>  VP1 encoding PCR product of 252485

<400>  120
tctgcagaag aatgtaaaca gaccatatca ccaaatgaat tgggattaac atcagctcag      60

gatgatggac cattgggtga taacaagcca aattatttcc taaatttcaa gtctatgaat     120

gtggacatct tcactgtttc ccacactaag gtggacaact tatttggaag agcatggttc     180

taccaggaac acactttcac aaatgaagga cagtggagag ttaatttgga gttcccaaaa     240

caaggtcatg gttcactttc tctgctattt gcttatttca caggtgaatt aaacatccat     300

gttttgttcc tagctgaaaa aggatttctt agggtagctc atacatacga cacatcagaa     360

aatagagtta acttcttgtc atctaatggt gttatcacaa tcccggcggg agaacaaatg     420

actttatctg caccttacta ctcaaataaa ccccttagga cagttaggga cagcaatagt     480

cttgggtatc tgatgtgtaa accattcctc actggaacaa caacagggaa aatagaggtc     540

taccttagcc tgagatgtcc aaattttttc tttcctctcc ccgcgcctaa agtgacaact     600

ggtcgtgttt tgcgggg                                                    617

<210>  121
<211>  617
<212>  DNA
<213>  Artificial

<220>
<223>  VP1 encoding PCR product of 350642

<400>  121
tctgcggaag aatgtaagca gaccatatca ccaaatgaat tgggattaac atcagctcag      60

gatgacggac cattgggcga caacaagcca aattatttcc taaatttcaa gtctatgaat     120

gtggacatct tcactgtttc ccacactaag gtggacaact tatttggaag agcatggttc     180

taccaggaac acactttcac aaatgaagga cagtggagag ttaatttgga gttcccaaaa     240

caaggtcatg gttcactttc tctgctattt gcttatttca caggtgaatt aaacatccat     300

gttttgttcc tagctgaaaa aggatttctc agggtagctc atacatacga cacatcagaa     360

aatagagtta acttcttgtc atctaatggt gttatcacaa tcccggcggg agaacaaatg     420

actttatctg caccttacta ctcaaataaa ccccttagga cagttaggga cagcaatagt     480

cttgggtatc tgatgtgcaa accattcctc actggaacaa caacagggaa aatagaggtc     540

taccttagcc tgagatgtcc aaatttcttc tttcctctcc ccgcgcctaa agtgacaact     600

ggtcgtgctc tgcgggg                                                    617

<210>  122
<211>  617
<212>  DNA

<213>  Artificial

<220>
<223>  VP1 encoding PCR product of 350918

<400>  122
tctgcggaag aatgtaagca gaccatatca ccaaatgaat tgggattaac atcagctcag      60

gatgacggac cattgggcga caacaagcca aattatttcc taaatttcaa gtctatgaat     120

gtggacatct tcactgtttc ccacactaag gtggacaact tatttggaag agcatggttc     180

taccaggaac acactttcac aaatgaagga cagtggagag ttaatttgga gttcccaaaa     240

caaggtcatg gttcactttc tctgctattt gcttatttca caggtgaatt aaacatccat     300

gttttgttcc tagctgaaaa aggatttctc agggtagctc atacatacga cacatcagaa     360

aatagagtta acttcttgtc atctaatggt gttatcacaa tcccggcggg agaacaaatg     420

actttatctg caccttacta ctcaaataaa ccccttagga cagttaggga cagcaatagt     480

cttgggtatc tgatgtgcaa accattcctc actggaacaa catcagggaa aatagaggtc     540

taccttagcc tgagatgtcc aaatttcttc tttcctctcc ccgcgcctaa agtgacaact     600

ggtcgtgctc tgcgggg                                                    617

<210>  123
<211>  617
<212>  DNA
<213>  Artificial

<220>
<223>  VP1 encoding PCR product of 550163

<400>  123
tccgcagagg aatgtaaaca gaccatatca ccaaatgaat taggtttgac atcggctcag      60

gatgatggac cattgggtga taacaaacca aattatttcc taaattttaa gtccatgaat     120

gtggacatat ttactgtttc tcacactaaa gtggacaact tatttgggag agcttggttt     180

ttccaagaac acactttcac caatgaagga cagtggagag ttagcttaga attcccaaaa     240

caaggccatg gttcactttc cttgttattt gcttatttta caggtgaatt aaacatccat     300

gttttgtttc tagctgagaa gggattcctc agagtagccc acacttatga cacatcagaa     360

aacagggtca acttcctatc atccaacggt gttatcacaa tcccggcagg agagcaaatg     420

acattatctg caccttacta ttcaaataaa cccctcagaa cagttaggga cagcaatagt     480

cttgggtatt taatgtgtaa accattcctc actggaacaa ccacagggaa aatagaagta     540

tatcttagtt tgagatgtcc aaattttttc tttcctcttc ctgcacctaa agttacaact     600

agccgtgcct tgcgggg                                                    617

<210>  124
<211>  617
<212>  DNA
<213>  Artificial

<220>
<223>  VP1 encoding PCR product of 151442

<400>  124
tctgcagagg aatgcaaaca gaccatatca ccaaatgaat tgggcttaac atcagctcag      60

gatgatggac cattgggcga taataagcca aattatttcc taaatttcaa gtccatgaat     120

gtggatatct tcactgtttc tcacactaag gtagacaacc tatttggaag agcttggttt     180

taccaggaac acactttcac caatgaaggg cagtggagag tcagtttaga gttcccgaaa     240

caaggccatg gttcgctttc attgttattc gcttacttta caggtgaact aaatattcat     300

gttctgtttt tagctgaaaa gggatttctt agagtagccc acacctatga cacatcagca     360

aacagggtta acttcctgtc atccaatggc gtcatcacaa tcccagctgg agaacaaatg     420

acattatctg caccctacta ctcaaataaa cccctcagaa cagttagaga caacaatagt     480

cttgggtatc taatgtgtaa accattcctc actggaacga caacagggaa gatagaggtt     540

tatcttagtt tgagatgtcc aaatttcttc tttcctcttc ccgcacctaa ggtttcaact     600

agccgtgcct tgcgggg                                                    617

<210>  125
<211>  617
<212>  DNA
<213>  Artificial

<220>
<223>  VP1 encoding PCR product of 251949

<400>  125
tctgcagagg aatgcaaaca gaccatatca ccaaatgaat tgggcttaac atcagctcag      60

gatgatggac cattgggcga caacaagcca aattatttcc taaatttcaa gtccatgaat     120

gtggatatct tcactgtttc tcacactaag gttgacaacc tatttggaag agcttggttt     180

taccaggaac acactttcac cgatgaaggg cagtggagag ttagtttaga gttcccgaag     240

caaggtcatg gttcactttc gttgctattc gcttacttca caggtgaact aaatattcat     300

gttctatttc tagctgaaaa gggatttctt agagtagccc acacctatga cacatcagca     360

aacagagtaa acttcctgtc gtccaatggc gtcatcacaa tcccagctgg agaacaaatg     420

acattatctg caccctacta ctcaaataaa cccctcagaa cagtcagaga caacaatagt     480

cttgggtacc taatgtgtaa accattcctc actggaacga caacagggaa gatagaggtt     540

tatctcagct tgagatgtcc aaatttcttc tttcctcttc ccgcacctaa ggtctcaact     600

agccgtgctt tgcgggg                                                    617

<210>  126
<211>  617
<212>  DNA
<213>  Artificial

<220>
<223>  VP1 encoding PCR product of 350014

<400>  126
tctgcagagg aatgcaaaca gaccatatcg ccaaatgaat tgggcttaac atcagctcag      60

gatgatggac cattgggcga taacaagcca aattatttcc taaatttcaa gtccatgaat 120

gtggatatct tcactgtttc tcatacaaag gtagacaacc tatttggaag agcttggttt 180

taccaggaac acactttcac caatgaaggg cagtggagag ttaatttgga gttcccgaaa 240

caaggccatg gttcgctttc attgttattc gcttacttca caggtgagct aaatattcat 300

gttctgttcc tagctgaaaa aggatttctt agagtagccc acacctatga cacatcagaa 360

aacagagtta acttcctgtc atccaatggc atcatcacaa tcccagctgg ggaacaaatg 420

acattatctg caccctacta ctcaaataaa cccctcagaa cagtcagaga taacaacagt 480

cttgggtatc taatgtgcaa accattcctc actggaacga caacagggaa gatagaggtt 540

tatcttagtt tgagatgtcc aaatttcttc tttcctcttc ccgcacctaa ggtttcaact 600

agccgtgctt tgcgggg 617

<210> 127
<211> 617
<212> DNA
<213> Artificial

<220>
<223> VP1 encoding PCR product of 350841

<400> 127
tctgcagagg aatgtaaaca gactatatcg ccaaatgaat tgggcttaac atcagctcag 60

gatgatggac cattgggcga taacaagcca aattatttcc taaatttcaa gtccatgaat 120

gtggatattt tcactgtttc tcacactaag gtagacaacc tatttggaag agcttggttt 180

taccaggaac atactttcac cgatgaaggg cagtggagag ttagtttaga gttcccgaaa 240

cgaggccatg gttcgctttc attgttattc gcttatttca caggtgaact aaatattcat 300

gttctgttcc tagctgaaaa gggatttctt agagtagccc acacctatga cacatcagcg 360

aacagagttg acttcctgtc atccaatggc gtcatcacaa tcccagctgg agaacaaatg 420

acattatctg caccctacta ctcaaataaa cccctcagaa cagtcagaga taacaatagc 480

cttgggtatc taatgtgtaa gccattcctc actggaacga caacagggaa gatagaggtt 540

tatcttagtt tgagatgtcc aaacttcttc tttcctcttc ccgcacctaa ggtttcaact 600

agccgtgctt tgcgggg 617

<210> 128
<211> 617
<212> DNA
<213> Artificial

<220>
<223> VP1 encoding PCR product of 450343

<400> 128
tctgcagagg agtgcaaaca aaccatatca cctaatgaac taggcttaac atcagcacaa 60

gatgatggac cattgggcga caataaacca aattatttcc taaatttcaa gtccatgaat 120

gtggacatct ttactgtttc ccacactaaa gtagacaact tgtttggaag ggcttggttt 180

ttcaaggagc acactttcac cgatgaggga cagtggagag ttagtttaga gttcccaaaa 240

caaggccatg gttcactttc cttattattt gcatacttta caggtgaatt aaatatccat 300

gttttgttct tggctgaaaa gggattcctc agggtggctc acacctatga cacatcggaa 360

aacagggtta acttcttatc atccaatggt gttatcacaa tcccagctgg agagcaaatg 420

acattatctg caccttacta ctcaaataaa ccccttagaa ctgtcaggga tagcaatagc 480

cttgggtatc taatgtgcaa accattcctc acaggaacga caacagggaa aatagaggtc 540

tatcttagtt tgaggtgccc caatttcttc tttcctctac ctgcacctaa agttacaact 600

ggtcgcgctt tgcgggg 617

<210> 129
<211> 617
<212> DNA
<213> Artificial

<220>
<223> VP1 encoding PCR product of 452176

<400> 129
tctgcagaag aatgtaaaca gacaatatca ccaaatgaat tgggattaac atcagcccag 60

gatgatggac cattgggcga taacaagcca aattatttcc taaatttcaa gtctatgaat 120

gtagacatct tcactgtttc ccacactaag gtggacaact tatttggaag agcatggttc 180

taccaggaac acacttttac agacgaagga cagtggagag ttaatttgga gttcccaaaa 240

caaggtcatg gttcactttc tctgctattt gcttatttca caggtgaatt aaacatccat 300

gttttgttct tagctgaaaa aggatttctt agagtagctc atacatacga cacatcagaa 360

aatagagtta acttcttgtc atctaatggt gttatcacaa tcccagcggg agaacaaatg 420

acattatctg caccttacta ctcaaataaa ccccttagga cagttaggga cagcaatagt 480

cttgggtatc tgatgtgcaa gccattcctc actggaacaa caacagggaa aatagaggtc 540

taccttagcc tgaggtgtcc aaatttcttc tttcctctcc ccgcgcctaa agtaacaact 600

ggtcgtactt tgcgggg 617

<210> 130
<211> 617
<212> DNA
<213> Artificial

<220>
<223> VP1 encoding PCR product of ECHO22INHOUSE

<400> 130
gacacagaaa attgcaaaca aacaatgtct ccaaatgaac taggactcac ttcagcccaa 60

gatgatggcc cacttggtca agaaaagcca aattattttc tcaattttag gtcgatgaat 120

gtggacattt ttactgtatc acatactaaa gtagataacc tatttggcgg ggcatggttt 180

tttatggagc atactttcac caatgaggga caatggagag tgccattgga atttccaaaa 240

caaggtcatg ggtccttatc actgttgttt gcttatttta ctggtgaact gaatatccat 300

gttctgttcc taagtgagag ggggtttctg agggttgcac acacatatga cactagtaat 360

gatcgagtca attttctgtc atcgaacggt gtaataactg taccagccgg agagcagatg 420

acactttcag ctccctacta ttcaaacaaa ccattaagaa ctgtcagaga taacaatagt 480

cttggttatt tgatgtgcaa gcccttcttg actggaacct ctactggtaa aattgaggtt 540

tatcttagcc tgagatgtcc aaatttcttt ttccctcttc ctgcccctaa ggttacgagt 600

agtcgtgcac tacgggg 617

<210> 131
<211> 617
<212> DNA
<213> Artificial

<220>
<223> VP1 encoding PCR product of HPEV1A942-99

<400> 131
tctgcagagg aatgtaaaca aaccatatca ccaaacgaat tgggtttaac gtcagctcag 60

gatgatggac cactgggcga taacaagcca aattatttcc taaattttaa atccatgaat 120

gtggacattt tcactgtttc tcatactaag gtagacaacc tatttggaag agcttggttt 180

taccaggaac acactttcac caatgaagga cagtggagag ttaatttaga gtttccaaaa 240

caaggccatg gttcgctttc cttactattc gcttatttca caggtgaatt gaatatccat 300

gttttgttcc tggctggaaa gggatttctg agggttgcac acacatatga cactagtaat 360

gatcgagtca attttctgtc atcgaacggt gttatcacaa tcccagcagg agaacaaatg 420

acactatctg caccatacta ctcaaataaa cccctcagaa cagttagaga cagcaatagt 480

cttgggtatc tgatgtgtaa accattcctc actggaacaa caacagggaa aatagaagtt 540

tatcttagtt tgaggtgtcc aaatttcttc tttcctcttc ccgcacctaa agttacaact 600

ggtcgtgctt tgcgggg 617

<210> 132
<211> 617
<212> DNA
<213> Artificial

<220>
<223> VP1 encoding PCR product of 152824

<400> 132
tctgcagaag aatgtaaaca gaccatatca ccaaatgaat tgggattgac atcagctcag 60

gatgatggac cattgggcga taacaagcca aattatttcc taaatttcaa gtctatgaat 120

gtggacatct tcactgtttc ccacactaag gtggacaact tatttggaag agcatggttc 180

taccaggaac acactttcac agatgaagga cagtggagag ttaatttgga gttcccaaaa 240

caaggtcatg gttcactttc tctgctattt gcttacttca caggtgaatt aaacatccat 300

gttttgttcc tagctgaaaa aggatttctt agggtagctc atacatacga cacatcagaa 360

aatagagtta acttcttgtc atctaatggt gttattacaa tcccggcggg agaacaaatg 420

aatttatctg caccttacta ctcaaacaaa ccccttagga cagttaggga cagcaatagt 480

cttgggtatc tgatgtgcaa accattcctc actggaacaa caacagggaa aatagaggtc 540

taccttagcc tgagatgtcc aaatttcttc tttcctctcc ccgcgcctaa agtgacaact 600

ggtcgtgctt tgcgggg 617

<210> 133
<211> 617
<212> DNA
<213> Artificial

<220>
<223> VP1 encoding PCR product of 252228

<400> 133
tctgcagaag aatgtaagca gaccatatca ccaaatgaat tgggattaac atcagctcag 60

gatgatggac cattgggcga taacaagcca aattatttcc taaatttcaa gtctatgaat 120

gtggacatct tcactgtttc ccacactaag gtggacaact tatttggaag agcatggttc 180

taccaggaac acactttcac aaatgaagga cagtggagag ttaatttgga gttcccaaaa 240

caaggtcatg gttcactttc tctgctattt gcttatttca caggtgaatt aaacatccat 300

gttttgttcc tagctgaaaa aggatttctt agggtagctc atacatacga cacatcagca 360

gatagagtta acttcttgtc atctaatggt gttatcacaa tcccggcggg agaacaaatg 420

actttatctg caccttacta ctcaaataaa ccccttagga cagttaggga cagcaatagt 480

cttgggtatc tgatgtgcaa accattcctc actggaacaa caacagggaa aatagagatc 540

taccttagcc tgagatgtcc aaatttcttc tttcctctcc ccgcgcctaa agtgacaact 600

ggtcgtgctc tgcgggg 617

<210> 134
<211> 617
<212> DNA
<213> Artificial

<220>
<223> VP1 encoding PCR product of 252521

<400> 134
tctgcagaag aatgtaaaca gaccatatca ccaaatgaat tgggattaac atcagctcag 60

gatgatggac cattgggcga taacaagcca aattattttc taaatttcaa atctatgaat 120

gtagatatct tcactgtttc tcacactaag gtggacaact tatttggaag agcatggttc 180

taccaggaac acactttcac aaatgaagga cagtggagag ttagtttgga gttcccaaaa 240

caaggtcatg gttcactttc tctgctattt gcttatttca caggtgaatt aaacatccat 300

gttttgttcc tagctgaaaa aggatttctt agagtagctc atacatacga cacatcagaa 360

aatagggtta atttcttgtc atctaatggt gttatcacaa tcccagcggg agaacaaatg 420

acattatccg caccttacta ctcaaataaa cctcttagga cagttaggga cagcaatagt 480

cttgggtatc tgatgtgcaa accattcctc actggaacaa caacagggaa aatagaagtc 540

taccttagct tgagatgtcc aaatttcttc tttcctctcc ccgcgcctaa agttacaact 600

agccgtgctt tgcgggg 617

<210> 135
<211> 617
<212> DNA
<213> Artificial

<220>
<223> VP1 encoding PCR product of 452473

<400> 135
tctgcagagg aatgcaaaca gaccatatcg ccaaatgaat tgggcttaac atcagctcag 60

gatgatggac cattgggcga taacaagcca aattatttcc taaatttcaa gtccatgaat 120

gtggatatct tcactgtttc tcacactaag gtggacaact tatttggaag agcatggttc 180

taccaggaac acacttttac cgatgaaggg cagtggagag ttaatttaga gttcccgaaa 240

caaggccatg gttcgctttc attgttattc gcttacttca caggtgaact aaacatccat 300

gttctgttcc tagctgaaag gggatttctt agagtagccc acacctatga cacatcagca 360

aacagagtta actttctgtc atccaatggc gtcatcacaa tcccagctgg ggaacaaatg 420

acattatctg caccctacta ctcaaataaa cccctcagaa cagtcagaga taacaatagt 480

cttgggtatc taatgtgtaa accattcctc actggaacga caagagggaa gatagaggtt 540

tatcttagtt tgagatgtcc aaatttcttt tttcctcttc ccgcacctaa ggtttcaact 600

agccgtgctt tgcgggg 617

<210> 136
<211> 617
<212> DNA
<213> Artificial

<220>
<223> VP1 encoding PCR product of 452252

<400> 136
tctgcagagg aatgcaaaca gaccatatcg ccaaatgaat tgggcttaac atcagctcag 60

gatgatggac cattgggcga taacaagcca aattatttcc taaatttcaa gtccatgaat 120

gtggatatct tcactgtttc tcacacaaag gtagataacc tatttggaag agcttggttt 180

taccaggaac acacttttac cgatgaaggg cagtggagag ttaatttaga gttcccgaaa 240

caaggccatg gttcgctttc attgttattc gcttacttca caggtgaact aaacatccat 300

gttctgttcc tagctgaaag gggatttctt agagtagccc acacctatga cacatcagca 360

aacagagtta actttctgtc atccaatggc gtcatcacaa tcccagctgg ggaacaaatg 420

acattatctg caccctacta ctcaaataaa cccctcagaa cagtcagaga taacaatagt 480

cttgggtatc taatgtgtaa accattcctc actggaacga caagagggaa gatagaggtt 540

tatcttagtt tgagatgtcc aaatttcttt tttcctcttc ccgcacctaa ggtttcaact 600

agccgtgctt tgcgggg 617

<210> 137
<211> 617
<212> DNA
<213> Artificial

<220>
<223> VP1 encoding PCR product of 452712

<400> 137
tctgcagaag aatgtaaaca gaccatatca ccaaatgaat tgggattaac atcagctcag 60

gatgatggac cattgggcga taacaagcca aattatttcc taaatttcaa gtccatgaat 120

gtggacatct tcactgtttc ccacactaag gtggacaact tatttggaag agcatggttc 180

taccaggaac atactttcac agatgaagga cagtggagag ttaatttgga gttcccaaaa 240

caaggtcatg gttcactttc tctgttattt gcttatttta caggtgagtt aaacatccat 300

gttttgttcc tagctgaaaa aggattcctt agggtagctc acacatacga cacatcagaa 360

aatagagtta atttcttgtc atctaatggt gttatcacaa tcccggcggg agaacaaatg 420

actttatctg caccttacta ctcaaataaa ccccttagga cagttaggga cagcaatagt 480

cttgggtatc tgatgtgcaa accattcctc actggaacaa caacagggaa aatagaggtc 540

taccttagcc tgaggtgtcc aaatttcttc tttcctctcc ccgcgcctaa agtgacaact 600

ggtcgtgctt tgcgggg 617

<210> 138
<211> 617
<212> DNA
<213> Artificial

<220>
<223> VP1 encoding PCR product of 350757

<400> 138
tctgcggaag aatgtaagca gaccatatca ccaaatgaat tgggattaac atcagctcag 60

gatgacggac cattgggcga caacaagcca aattatttcc taaatttcaa gtctatgaat 120

gtggacatct tcactgtttc ccacactaag gtggacaact tatttggaag agcatggttc 180

taccaggaac acactttcac aaatgaagga cagtggagag ttaatttgga gttcccaaaa 240

caaggtcatg gttcactttc tctgctattt gcttatttca caggtgaatt aaacatccat 300

gttttgttcc tagctgaaaa aggatttctc agggtagctc atacatacga cacatcagaa 360

aatagagtta acttcttgtc atctaatggt gttatcacaa tcccggcggg agaacaaatg 420

actttatctg caccttacta ctcaaataaa ccccttagga cagttaggga cagcaatagt 480

cttgggtatc tgatgtgcaa accattcctc actggaacaa caacagggaa aatagaggtc 540

taccttagcc tgagatgtcc aaatttcttc tttcctctcc ccgcgcctaa agtgacaact 600

ggtcgtgctc tgcgggg 617

<210> 139
<211> 617
<212> DNA

<213>  Artificial

<220>
<223>  VP1 encoding PCR product of 152598

<400>  139
tctgcagagg aatgcaaaca gaccatatca cccaacgaat tgggcttaac atcagcccag      60

gatgatggac cattgggcga cagcaaacca aattattttt taaattttaa gtccatgaat     120

gtagacattt tcactgtatc ccacactaaa gtagacaacc tattcggaag ggcttggttt     180

taccaggaac acacttttac caatgaagga cagtggagag ttaatttgga gttcccaaaa     240

caaggccatg gttcactttc attgctattc gcttacttca caggtgaatt aaatatccat     300

gttttgttct tagctgaaag aggattcctt agagtagctc acacctacga tacatctgaa     360

aacagggtta acttcttgtc atccaatggt gttatcacaa tcccagcagg agaacaaatg     420

acattatctg caccttacta ttcaaataaa cctcttagaa cagttaggga cagcaatagt     480

cttggatatt taatgtgtaa accgttcctc actggaacaa caacagggaa aatagaggtt     540

tatcttagtt tgagatgtcc aaatttcttc tttcctcttc ctgcacctaa agttacaacc     600

agccgagctc tgcgggg                                                    617

<210>  140
<211>  617
<212>  DNA
<213>  Artificial

<220>
<223>  VP1 encoding PCR product of 252581

<400>  140
tctgcagagg aatgtaaaca gactatatca ccaaatgaat tgggcttaac atcagcccag      60

gatgatggac cattgggtga tagtaagcca aattatttcc taaatttcaa gtccatgaat     120

gtggacattt tcactgtttc tcacactaaa gtagacaacc tatttggaag agcttggttt     180

taccaggaac acactttcac cgatgaaggg cagtggagag tcagtttaga gttcccgaaa     240

caaggtcatg gttcactttc cttgctattt gcttatttca caggtgagtt aaatatccat     300

gttttgttcc tagctgaaaa gggattcctt agagtagcac atacttatga cacatcagca     360

gacagggtaa atttcttgtc atccaatggt gttattacaa tcccagccgg agaacaaatg     420

acattatctg caccttacta ctcaaataaa ccccttagaa cagttagaga cagcaatagt     480

cttgggtatc taatgtgtaa accattcctt actggaacaa caacagggaa aatagaggtc     540

tatcttagct tgagatgtcc aaatttcttt tttccacttc ctgcacctaa ggtttcaacc     600

agccgtgctt tgcgtgg                                                    617

<210>  141
<211>  617
<212>  DNA
<213>  Artificial

<220>
<223>  VP1 encoding PCR product of 451294

<400>  141
tctgcagagg aatgcaaaca gaccatatcg ccaaatgaat tgggcttaac atcagctcag      60

gatgatggac cattgggcga taacaagcca aattatttcc taaatttcaa gtccatgaat     120

gtggatatct tcactgtttc tcacacaaag gtagataacc tatttggaag agcttggttt     180

taccaggaac acacttttac caatgaaggg cagtggagag ttaatttaga gttcccgaaa     240

caaggccatg gttcgctttc attgttattc gcttacttca caggtgaact aaacatccat     300

gttctgttcc tagctgaaag gggatttctt agagtagccc acacctatga cacatcagca     360

aacagagtta actttctgtc atccaatggc gtcatcacaa tcccagctgg ggaacaaatg     420

acattatctg caccctacta ctcaaataaa cccctcagaa cagtcagaga taacaatagt     480

cttgggtatc taatgtgtaa accattcctc actggaacga caacagggaa gatagaggtt     540

tatcttagtt tgagatgtcc aaatttcttt tttcctcttc ccgcacctaa ggtttcaact     600

agccgtgctt tgcgggg                                                    617

<210>  142
<211>  617
<212>  DNA
<213>  Artificial

<220>
<223>  VP1 encoding PCR product of 452538

<400>  142
tctgcagaag aatgtaaaca gaccatatca ccaaatgaat tgggattaac atcagctcaa      60

gatgatggac cattgggcga taacaagcca aattatttcc taaatttcaa gtccatgaat     120

gtggacatct tcactgtttc ccacactaag gtggacaact tatttggaag agcatggttc     180

taccaggaac atactttcac aaatgaagga cagtggagag ttaatttgga gttcccaaaa     240

caaggtcatg gttcactttc tctgttattt gcttatttta caggtgagtt aaacatccat     300

gttttgttcc tagctgaaaa aggattcctt agggtagctc acacatacga cacatcagaa     360

aatagagtta atttcttgtc atctaatggt gttatcacaa tcccggcggg agaacaaatg     420

actttatctg caccttacta ctcaaataaa ccccttagga cagttaggga cagcaatagt     480

cttgggtatc tgatgtgcaa accattcctc actggaacaa caacagggaa aatagaggtc     540

taccttagcc tgaggtgtcc aaatttcttc tttcctctcc ccgcgcctaa agtgacaact     600

ggtcgtgctt tgcgggg                                                    617

<210>  143
<211>  617
<212>  DNA
<213>  Artificial

<220>
<223>  VP1 encoding PCR product of HPEV2CT86-6760

<400>  143
gaagctgagg attgtaaaca aactatatct cctgatgaac taggtcttac ctcagcacaa      60

gatgatgggc cccttggagt agagaaacca aattattttc taaattttag agcaattaat 120

gtagatatct ttactgttag tcatactaag gtagataaca tttttgggcg tgcatggttg 180

gcccttgaac acacatttgc agatgatgga acatggaggg cagatttgaa ttttcccaca 240

cagggtcatg gtactctgac aagactcttc acatattact ctggtgaatt aaatgtgcat 300

gtactgtatc ttagtgacaa tgggttcctc cgagtaactc atgcctatga ccatgataat 360

gacagatcca actttttgtc atccaatgga gtaattacag tgccagcagg tgaacagatg 420

acgctttctg tgccattcta ttcttctaaa ccacttagaa caataagaga aactggtgca 480

ttaggcaaat taatctgtaa accattgttg tctggcacac attcagggaa gatcgaagtt 540

tatttgagtc tcagatgccc taatctattc tttccttctc ctgcacctaa agagaaaact 600

tccagagctt tgcgggg 617

<210> 144
<211> 617
<212> DNA
<213> Artificial

<220>
<223> VP1 encoding PCR product of 251176

<400> 144
tgcactgaag attgcaaaca gactatttcc ccagatgaac tgggtctaac ttcagcccaa 60

gacgatggtc ctctgggagg tgaaaagcca aattacttct tgaattttag agctgtcaat 120

gttgacatat ttactgtgag tcacactaaa gtagacaaca tctttggtag ggcatggttt 180

gcatatgatc atacatatag agatgaagga acctggaggc aggctttgga tttcccaaag 240

aaaggccatg gtgccttaac ccaattattt gcctattact caggagaatt aaatatacat 300

gttttattct tgagtgaaac agggtttctg agagtggcac atacttatga cagtgataca 360

aacaggtctg acttcttctc ttcaaacggc gtcatcactg tgcccgcagg ggaacaaatg 420

accctgtcag taccattcta ctcttcaaag cccttgagga caatcaggga ttcagctgct 480

ctagggtatg tgatgtgtaa accattcatg tctgggacaa caggtggaaa gatagagata 540

tatctgagtt taagatgtcc aaacctattc tttcccttac cagctccgaa acctgcaact 600

agtcgtgctt tgcgggg 617

<210> 145
<211> 267
<212> DNA
<213> Artificial

<220>
<223> Consensus VP1 encoding PCR product

<400> 145
gaatgaacac atcccgaatg gtactcgcca gagaggcctg gaaccaaatt atttaattac 60

taatgtgaat ttacgtcaac aagtgaaatt tggggctggt cataggtgga gttttccagg 120

caggtctttc tatcgggata atcatttttg gttctggtca ctagaagttt tctcaggtat 180

actccgcggg acaatgattc gccttatcaa cctgactgtg gtttgacctg gacagattta 240

gtagtgccaa tttttccccg cccaagg 267

<210> 146
<211> 441
<212> DNA
<213> Artificial

<220>
<223> Consensus VP1 encoding cDNA product

<400> 146
gactgagatt gaaacaacta ttcccgatga actggtctac tcagccaaga gatggccctg 60

gaggaaacca aattatttaa ttttagagct aatgtgaatt ttactgtagt caactaagta 120

gaaacatttt ggggcatggt tgctgacaac attagatgag gaactggagg gtttgatttc 180

caaggcatgg tctacatttc tattactcgg gaattaaatt catgttttag tgaagggttc 240

tgagtccatc tatgactgat aacagtcact ttttctcaag ggtatacgtg ccgcagggaa 300

caatgacctt cgtccattct atcttcaacc tagacaatag gacgtgctag gatattgtaa 360

accatttgtc tggacaggaa gatgattatt gagttagatg ccaactattc tttcctccgc 420

ccaaaaactg gctttgcggg g 441

## Description

The invention relates to the fields of virology and medicine. More specifically, the invention relates to the identification of a new parechovirus.

The genus Parechovirus, being the latest addition to the nine genera of the family *Picornaviridae,* now includes three human pathogens HPEV1, HPEV2 and HPEV3 as well as a rodent virus, Ljungan virus. HPEV1 and HPEV2 (previously known as echovirus 22 and 23 respectively) have been designated to the Parechoviral genus in view of their unique molecular and biological properties.

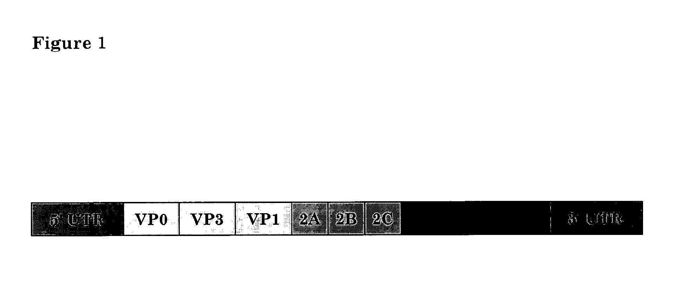

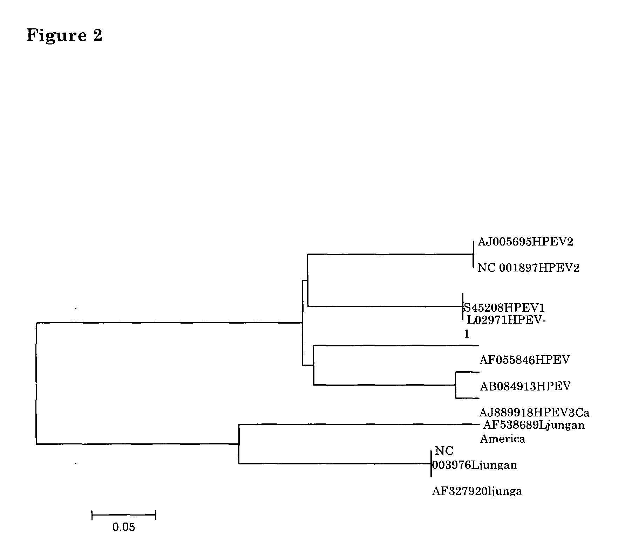

Like other picornaviruses, parechoviruses are small non-enveloped particles containing a monocistronic RNA genome with positive polarity which is packed in a protein capsid consisting of 60 copies of each of the structural proteins VP1 to VP4. However, parechoviruses have distinctive molecular properties compared to other picornaviruses, including differences in capsid protein processing (VP0 is not cleaved to VP2 and VP4, See Fig. 1) and functions of the non-structural proteins¹⁰. A simple phylogenetic tree of the HPEV1, 2 and 3 type strains is shown in Figure 2.

Members of the genus *Parechovirus* are common pathogens with a worldwide distribution and studies have shown that HPEV1 infections occur early in life and the number of seropositive children increases rapidly after one year of age. Thus, the incidence of Parechovirus infection appears to be extremely high and the age distribution is distinct from that of typical enteroviruses. HPEV1 causes, in most cases, mild infections of the gastrointestinal or respiratory tract. When compared, for instance, to enterovirus infections, the involvement of the central nervous system is less frequent, although cases of encephalitis and paralysis have been reported. Myocarditis, necrotizing enterocolitis and hemolytic uremic syndrome have also been associated with PEV1 infection.

Parechovirus 1 infections are very common, by contrast parechovirus 2 infections seem rarer, but are associated with the same clinical symptoms. HPEV-3 has been recently isolated from stool specimens of a young Japanese child with transient paralysis. From Canada, three additional cases of neonatal sepsis associated with HPEV-3 were reported in the fall of 2001 in infants 7-27 days old. All children were hospitalized with high fever, erythematous rash, and tachypnea for a median of 5 days². Recent work by Primagen demonstrated that HPEV-3 circulates as a pathogen in the Netherlands. In 2004, the RIVM and Rijnstate hospital in Arnhem identified Parechovirus 3 as newly emerging pathogens in the Netherlands associated with cases of neonatal sepsis-like syndrome.

Hence, parechoviral infections are common. Characterization of the causative agent is desired in order to provide adequate diagnosis, treatment and/or prevention of infection.

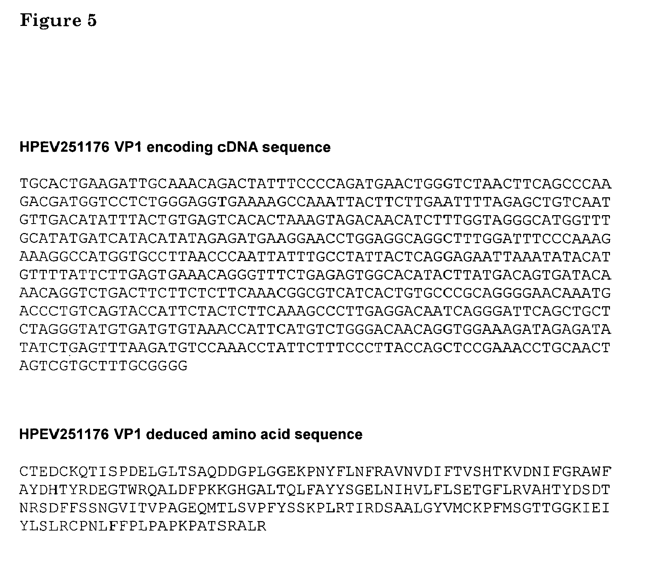

The invention provides a previously unknown member of the Parechovirus genus. This new virus (designated Parechovirus 251176, also called HPEV 251176) was characterized and several of the isolated fragments were sequenced. Sequences of the VP1 domain, the 3' end, the VP0 domain and part of the 5' end are depicted in Figures 5, 6, 7 and 8. The invention therefore, in one aspect, provides an isolated or recombinant virus comprising a nucleic acid sequence as depicted in Figure 5, Figure 6, Figure 7 and/or Figure 8, or a functional part, derivative and/or analogue of said virus. Now that these sequences have been provided, the genome of Parechovirus 251176 is further sequenced. This is done using well known methods. In one embodiment at least one primer is designed based on at least part of a sequence as depicted in figure 5, 6, 7 and/or 8. Preferably, said primer is capable of specifically binding a region that is located at or near the 5' or 3' end of at least one of the sequences depicted in figures 5, 6, 7 and/or 8. Subsequently, an amplification reaction is performed with said at least one primer. An amplificate is obtained which is at least in part sequenced.

One embodiment comprises primer walking on the genome. A primer is directed to a region of which the sequence is known and this primer is used to sequence a flanking region that is as yet unknown. A subsequent primer is preferably generated against a newly identified sequence in order to sequence a further region. This procedure can be repeated until the entire sequence of the virus is elucidated.

In one embodiment at least one consensus primer is used to amplify partially overlapping amplification fragments. Amplified sequences are subsequently sequenced. Said at least one consensus primer is preferably derived from genome alignment of at least two known Parechoviruses. Preferably, said at least one consensus primer is derived from genome alignment of at least four known Parechoviruses. Said at least one consensus primer is most preferably derived from genome alignment of all known Parechoviruses. The 3' end of the HPEV251176 genome is preferably amplified using a combination of JZH and HPEV x6556F, depicted in Table 1, preferably following cDNA synthesis primed with JHZ-tagged oligo-dT oligonucleotides. The 5' end of HPEV 251176 is preferably amplified using primers GSP1 and 2 depicted in Table 1. In one preferred embodiment the 5'-RACE protocol described in the Examples is used.

As a source of the virus the isolate ECACC 05060901 (filed on 9 June 2005 at the European Collection of Cell Cultures, CAMR, Salisbury, Wiltshire, UK) is for instance used.

The reading frames used in the Parechovirus 251176 sequences have been determined using tBLASTn software. The corresponding amino acid sequences are depicted under each nucleotide sequence in figures 5-7. The invention therefore further provides an isolated or recombinant virus comprising an amino acid sequence as depicted in Figure 5, Figure 6 and/or Figure 7, or a functional part, derivative and/or analogue of said virus. These amino acid sequences need not be encoded by exactly the same nucleic acid sequences in each and every HPEV 251176 strain because a given amino acid sequence can be encoded by a variety of codons. Hence, viruses comprising nucleic acid with alternative codons encoding at least one amino acid sequence as depicted in figure 5, 6 and/or 7 are also within the scope of the invention. One embodiment therefore provides an isolated or recombinant virus comprising a nucleic acid sequence encoding an amino acid sequence as depicted in Figure 5, Figure 6 and/or Figure 7, or a functional part, derivative and/or analogue of said virus.

The new Parechovirus of the invention is characterised by the strain ECACC 05060901. However, many natural variants of this strain exist because parechoviruses acquire a plurality of mutations upon spreading of the virus through a subject population and/or during culturing *ex vivo.* The existence of such natural variants is normal for RNA viruses that undergo frequent mutation through for instance the introduction of mistakes by a polymerase that copies the genome. HPEV 251176 viruses that have a slightly divergent nucleic acid sequence are thus also provided by the present invention. Such viruses are considered to be a derivative of a virus comprising a nucleic acid sequence as depicted in figure 5, 6, 7 and/or 8. The variant does not necessarily have to be a natural variant. It is very well possible to generate variants through recombinant means. For instance the genome of the virus is altered through nucleotide substitution, without altering the amino acid sequence of the encoded proteins, if use is made of the redundancy in the triplet genetic code for particular amino acids. It is also possible to introduce amino acid alterations, while retaining the replicating potential of the virus in kind, not necessarily in amount. This is for instance done via conservative amino acid substitution. However, non-conservative amino acid substitutions are also often tolerated without significantly inhibiting the replicating potential of the virus.

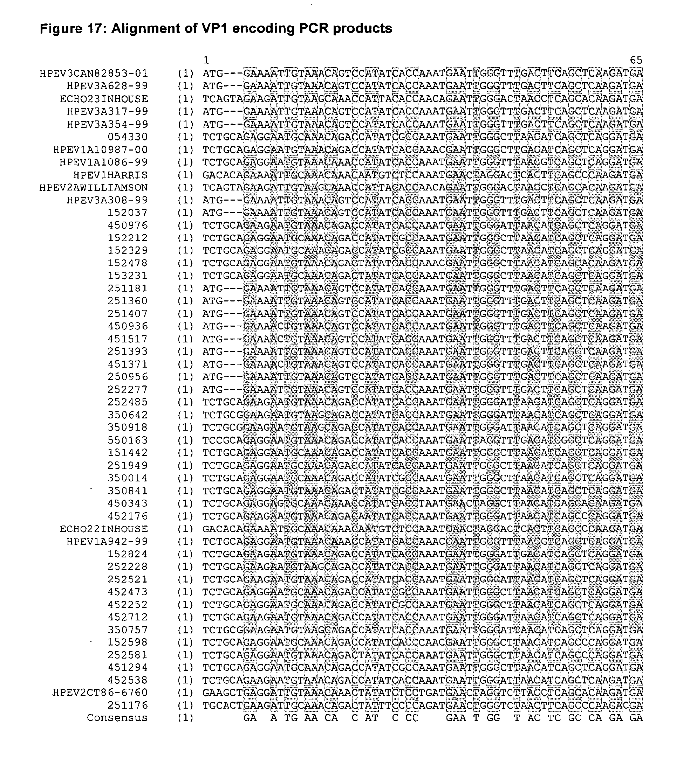

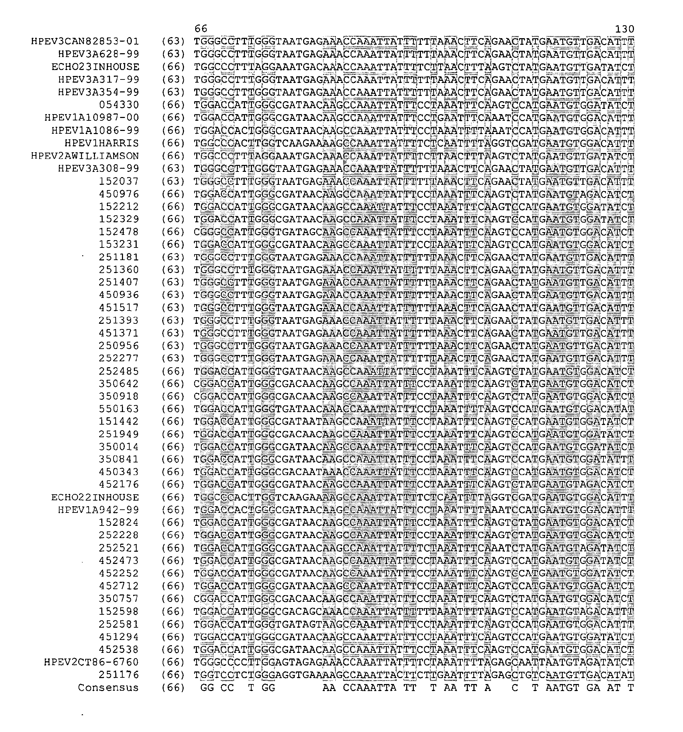

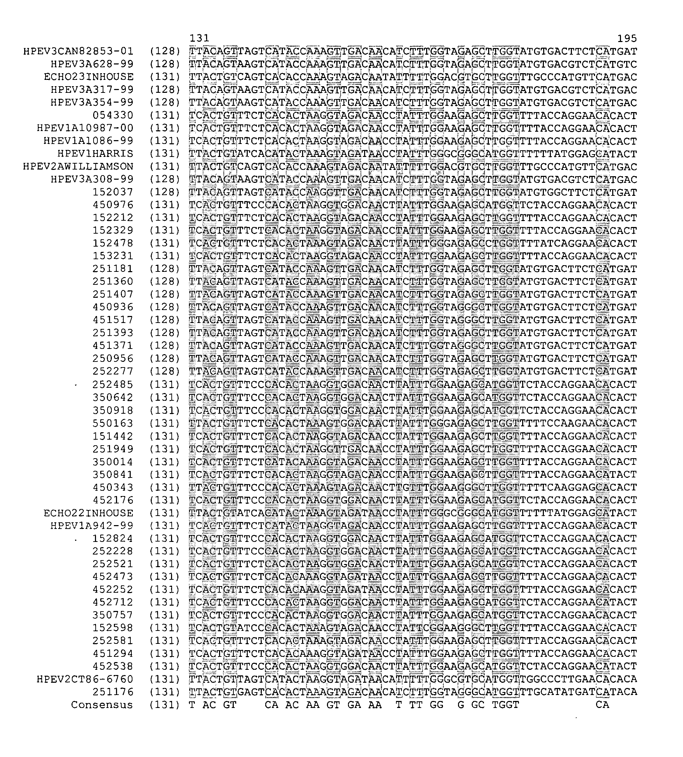

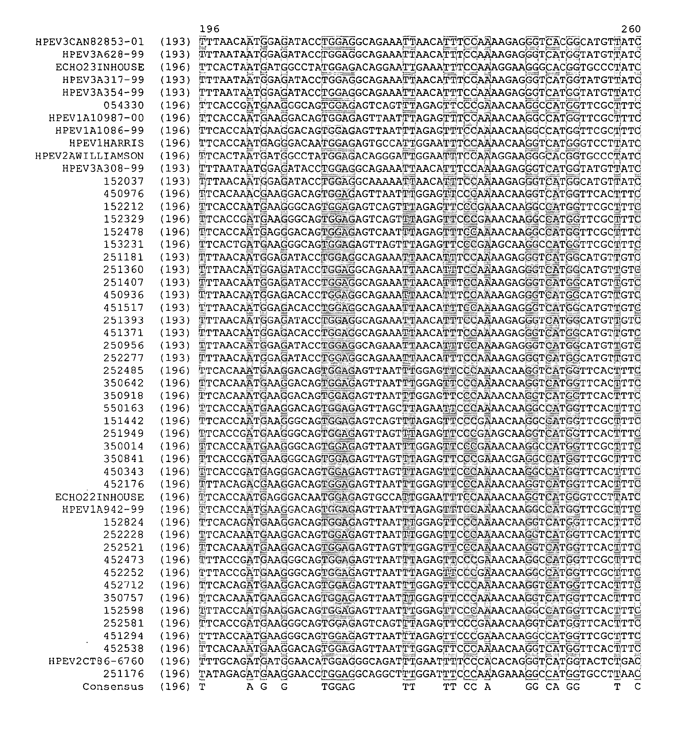

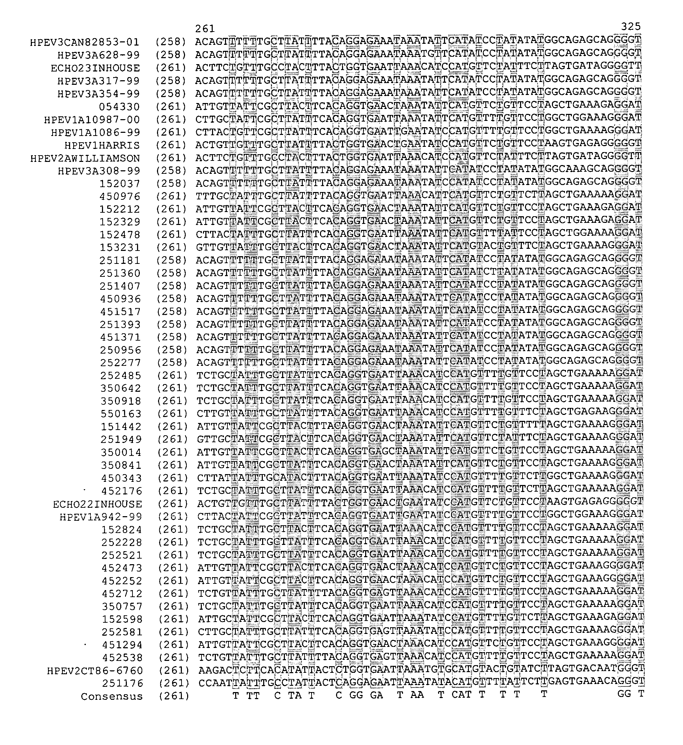

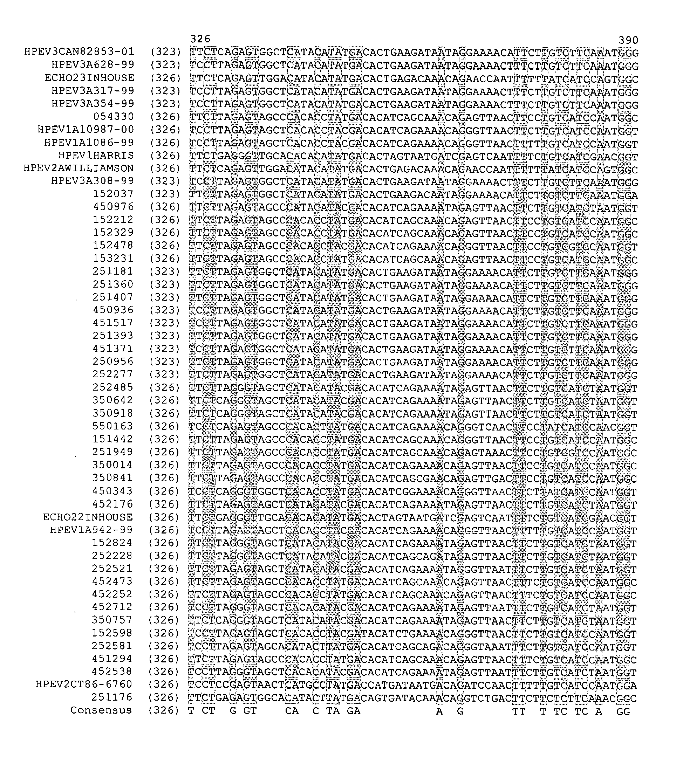

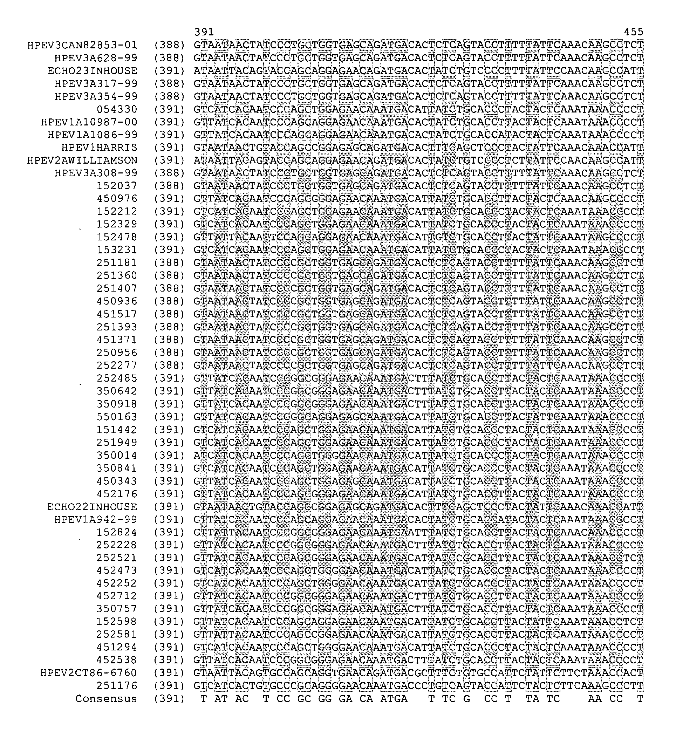

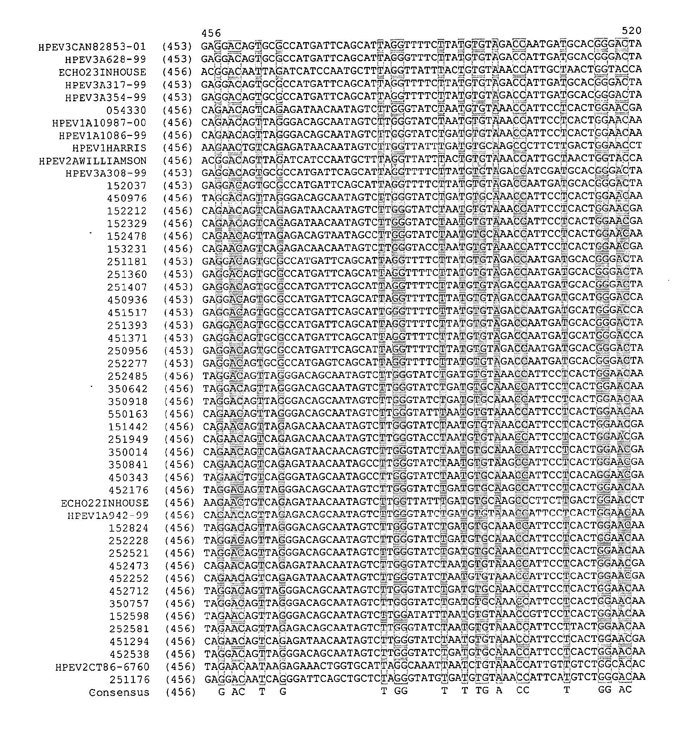

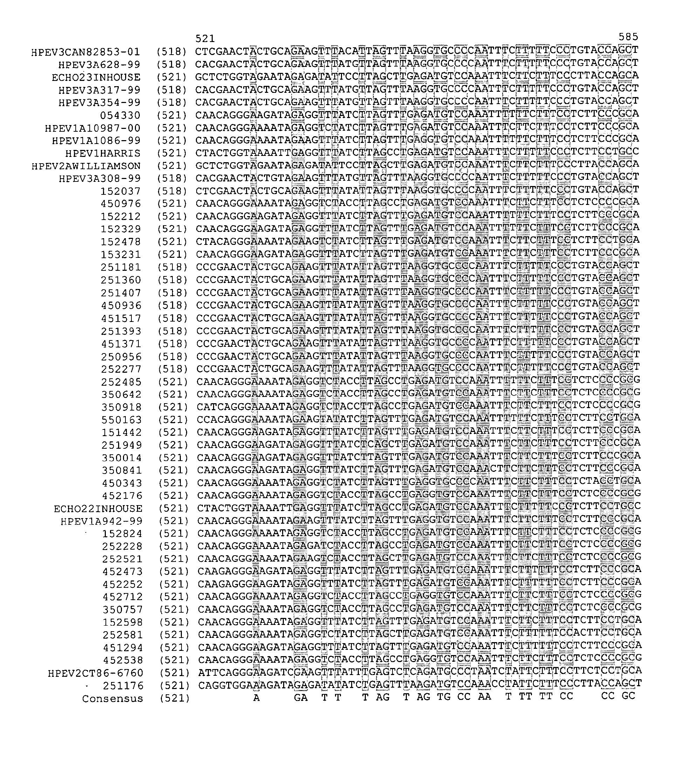

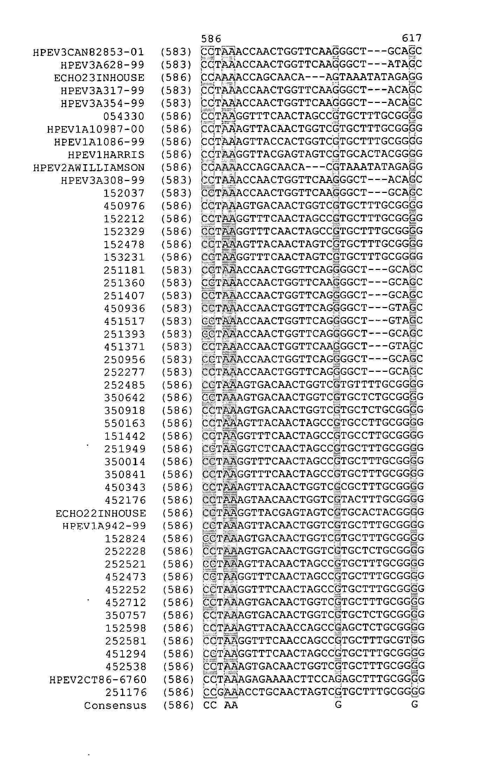

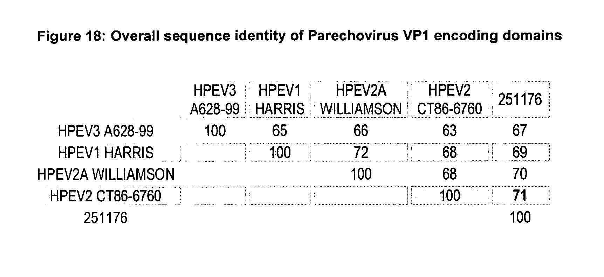

As shown in Figures 17 and 18, the VP1 encoding nucleic acid sequence of HPEV251176 as depicted in Figure 5 differs significantly from other known Parechoviral strains. The most homologous VP1 nucleic acid sequence of another HPEV strain that has been found is present in HPEV2 CT86-6760, which nucleic acid sequence is 71% homologous to the HPEV251176 sequence depicted in Figure 5. It is thus noted that the nucleic acid of Figure 5 does not share extensive homology with any of the currently known Parechoviruses. The invention therefore provides in one embodiment an isolated or recombinant virus comprising a nucleic acid sequence which is more than 71% homologous to a nucleic acid sequence as depicted in Figure 5, or a functional part, derivative and/or analogue of said virus. Said virus, functional part, derivative and/or analogue preferably comprises a nucleic acid sequence which is more than 75% - more preferably more than 80%, more preferably more than 90% - homologous to a nucleic acid sequence as depicted in Figure 5. The higher the amount of homology, the better a strain is classified as belonging to a specific strain of the HPEV 251176 serotype.

Like other Parechoviruses, HPEV 251176 is an RNA virus. In the present application Parechoviral-derived sequences such as HPEV 251176-derived sequences are depicted as cDNA. It is to be understood that these sequences are representatives of the corresponding RNA sequences (comprising uracil instead of thymine) as well. Hence, as used herein, any reference to a Parechoviral nucleic acid sequence comprises a reference to a cDNA sequence as well as a corresponding RNA sequence. Therefore, as used herein, a nucleic acid sequence with a certain percentage of homology to a nucleic acid sequence of the present invention means a nucleic acid sequence that is homologous to a cDNA sequence of the invention and/or a corresponding RNA sequence. Preferably said nucleic acid sequence is homologous to said corresponding RNA sequence.

Further provided is an isolated or recombinant virus comprising a nucleic acid sequence which is more than 88.5% homologous to at least part of a nucleic acid sequence as depicted in Figure 5, said part having at least 25 nucleic acid residues, or a functional part, derivative and/or analogue of said virus. Preferably said homology is at least 90%, more preferably at least 95%. Stretches of 25 consecutive nucleic acid sequences which are more than 88.5% homologous to the VP1 sequence depicted in Figure 5 are specific for HPEV251176.

Further provided is an isolated or recombinant virus comprising a nucleic acid sequence which is more than 87.1% homologous to at least part of a nucleic acid sequence as depicted in Figure 5, said part having at least 30 nucleic acid residues, or a functional part, derivative and/or analogue of said virus. Preferably said homology is at least 90%, more preferably at least 95%. Stretches of 30 consecutive nucleic acid sequences which are more than 87.1% homologous to the VP1 sequence depicted in Figure 5 are specific for HPEV251176.

Further provided is an isolated or recombinant virus comprising a nucleic acid sequence which is more than 83.9% homologous to at least part of a nucleic acid sequence as depicted in Figure 5, said part having at least 50 nucleic acid residues, or a functional part, derivative and/or analogue of said virus. Preferably said homology is at least 85%, more preferably at least 90% and even more preferably at least 95%. Stretches of 50 consecutive nucleic acid sequences which are more than 83.9% homologous to the VP1 sequence depicted in Figure 5 are specific for HPEV251176.

Further provided is an isolated or recombinant virus comprising a nucleic acid sequence which is more than 78.2% homologous to at least part of a nucleic acid sequence as depicted in Figure 5, said part having at least 100 nucleic acid residues, or a functional part, derivative and/or analogue of said virus. Preferably said homology is at least 80%, more preferably at least 85%, more preferably at least 90% and even more preferably at least 95%. Stretches of 100 consecutive nucleic acid sequences which are more than 78.2% homologous to the VP1 sequence depicted in Figure 5 are specific for HPEV251176.

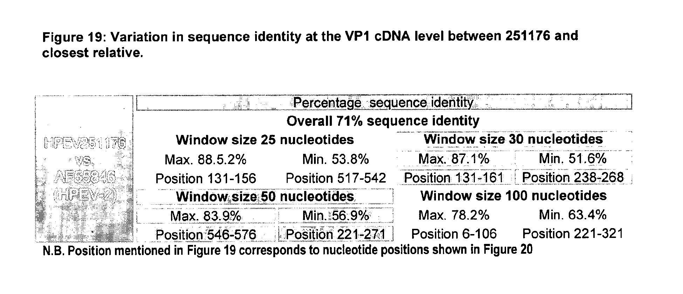

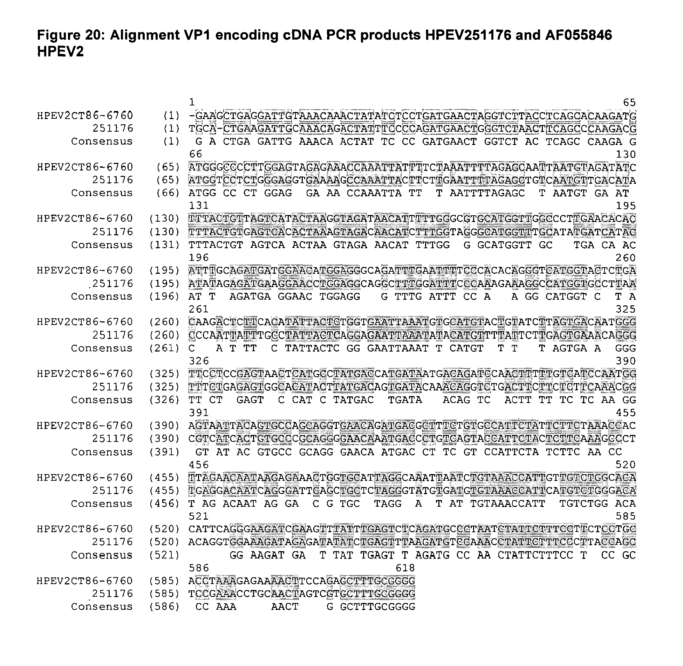

The invention furthermore provides an isolated or recombinant virus comprising a nucleic acid sequence which is more than 53.8% homologous to a nucleic acid sequence located from position 517 to 542 in the HPEV 251176 VP1 nucleic acid sequence as depicted in Figure 20, or a functional part, derivative and/or analogue of said virus. Preferably said homology is at least 60%, more preferably at least 70%, more preferably at least 80% and even more preferably at least 90%.

Furthermore provided is an isolated or recombinant virus comprising a nucleic acid sequence which is more than 51.6% homologous to a nucleic acid sequence located from position 238 to 268 in the HPEV 251176 VP1 nucleic acid sequence as depicted in Figure 5, or a functional part, derivative and/or analogue of said virus. Preferably said homology is at least 55%, more preferably at least 60%, more preferably at least 70%, more preferably at least 80% and even more preferably at least 90%.

Furthermore provided is an isolated or recombinant virus comprising a nucleic acid sequence which is more than 56.9% homologous to a nucleic acid sequence located from position 221 to 271 in the HPEV 251176 VP1 nucleic acid sequence as depicted in Figure 5, or a functional part, derivative and/or analogue of said virus. Preferably said homology is at least 60%, more preferably at least 70%, more preferably at least 80% and even more preferably at least 90%.

Furthermore provided is an isolated or recombinant virus comprising a nucleic acid sequence which is more than 63.4% homologous to a nucleic acid sequence located from position 221 to 321 in the HPEV 251176 VP1 nucleic acid sequence as depicted in Figure 5, or a functional part, derivative and/or analogue of said virus. Preferably said homology is at least 65%, more preferably at least 70%, more preferably at least 80% and even more preferably at least 90%.

A nucleic acid sequence located from position 517 to 542 in the HPEV 251176 VP1 nucleic acid sequence as depicted in Figure 20, a nucleic acid sequence located from position 238 to 268 in the HPEV 251176 VP1 nucleic acid sequence as depicted in Figure 20, a nucleic acid sequence located from position 221 to 271 in the HPEV 251176 VP1 nucleic acid sequence as depicted in Figure 20 and a nucleic acid sequence located from position 221 to 321 in the HPEV 251176 VP1 nucleic acid sequence as depicted in Figure 20 are particularly indicative for HPEV, since these sequences are not conserved between Parechoviral serotypes. Hence, these sequences are particularly suitable for distinguishing HPEV251176 from other Parechoviruses. For instance, a primer and/or probe capable of specifically hybridizing to at least one of said sequences is suitable for selectively amplifying and/or detecting HPEV 251176.

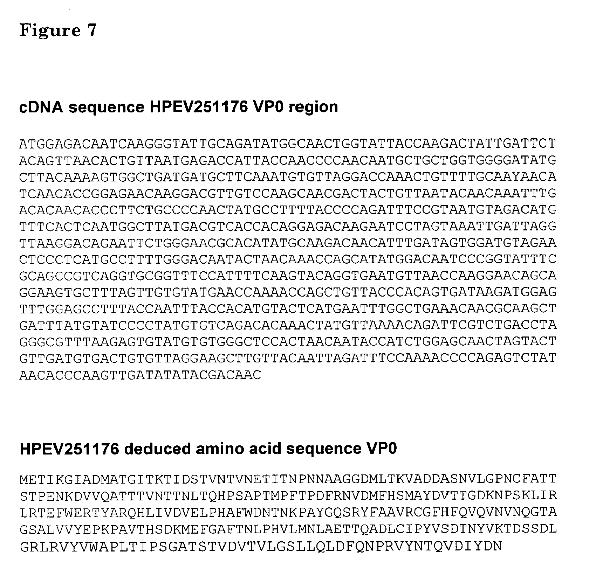

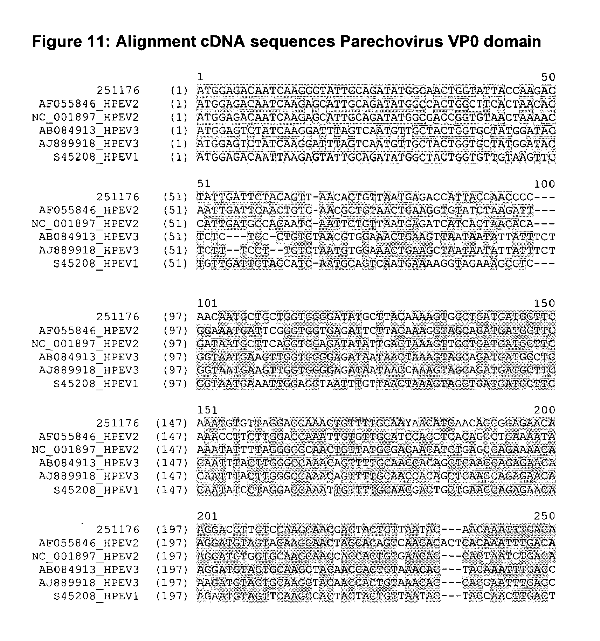

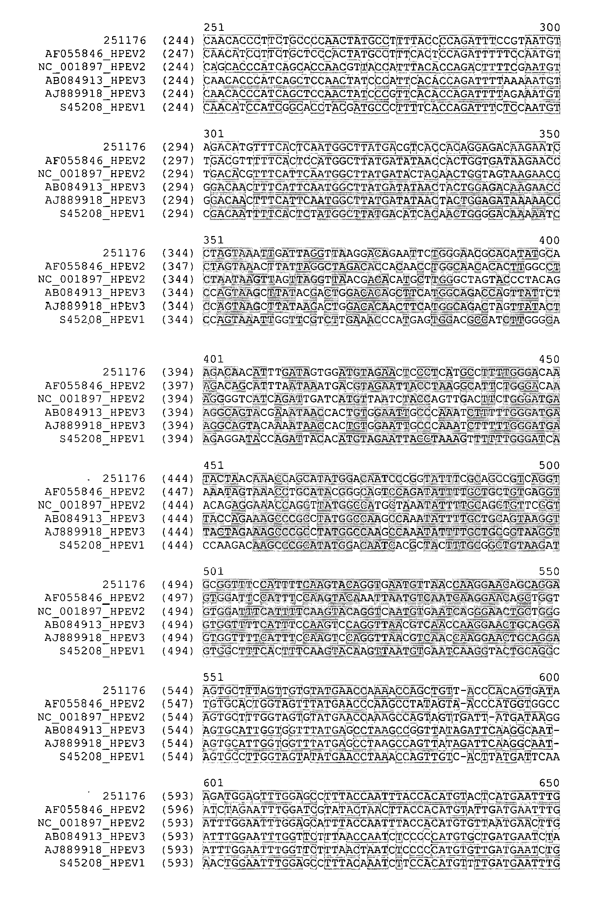

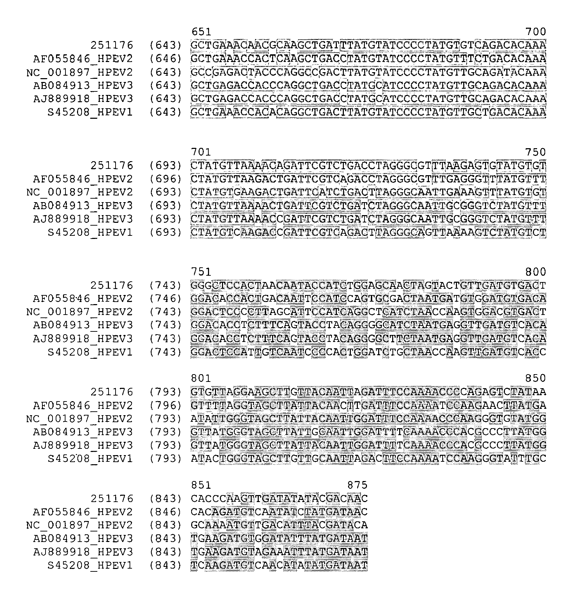

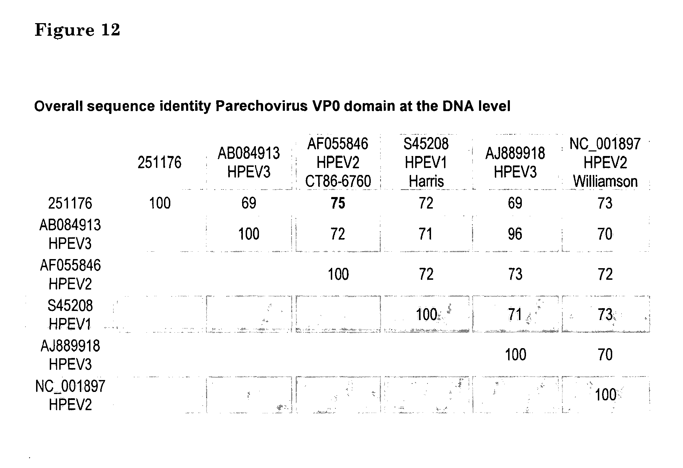

It is furthermore shown in Figures 11 and 12 that the VP0 encoding nucleic acid sequence of HPEV251176 as depicted in Figure 7 differs significantly from other known Parechoviral strains. The most homologous VP0 nucleic acid sequence of another HPEV strain that has been found is present in HPEV2 CT86-6760, which nucleic acid sequence is 75% homologous to the HPEV251176 sequence depicted in Figure 7. It is thus noted that the nucleic acid of Figure 7 does not share extensive homology with any of the currently known Parechoviruses. The invention therefore provides an isolated or recombinant virus comprising a nucleic acid sequence which is more than 75% homologous to a nucleic acid sequence as depicted in Figure 7. Preferably said homology is at least 80%, more preferably at least 85%, more preferably at least 90% and even more preferably at least 95%.

Further provided is an isolated or recombinant virus comprising a nucleic acid sequence which is more than 96.2% homologous to at least part of a nucleic acid sequence as depicted in Figure 7, said part having at least 25 nucleic acid residues, or a functional part, derivative and/or analogue of said virus. Preferably said homology is at least 97%, more preferably at least 98% and even more preferably at least 99%. Stretches of 25 consecutive nucleic acid sequences which are more than 96.2% homologous to the VP0 sequence depicted in Figure 7 are specific for HPEV251176.

Further provided is an isolated or recombinant virus comprising a nucleic acid sequence which is more than 93.3% homologous to at least part of a nucleic acid sequence as depicted in Figure 7, said part having at least 30 nucleic acid residues, or a functional part, derivative and/or analogue of said virus. Preferably said homology is at least 95%, more preferably at least 97% and even more preferably at least 98%. Stretches of 30 consecutive nucleic acid sequences which are more than 93.3% homologous to the VP0 sequence depicted in Figure 7 are specific for HPEV251176.

Further provided is an isolated or recombinant virus comprising a nucleic acid sequence which is more than 90.4% homologous to at least part of a nucleic acid sequence as depicted in Figure 7, said part having at least 50 nucleic acid residues, or a functional part, derivative and/or analogue of said virus. Preferably said homology is at least 95%, more preferably at least 97% and even more preferably at least 98%. Stretches of 50 consecutive nucleic acid sequences which are more than 90.4% homologous to the VP0 sequence depicted in Figure 7 are specific for HPEV251176.

Further provided is an isolated or recombinant virus comprising a nucleic acid sequence which is more than 89.1% homologous to at least part of a nucleic acid sequence as depicted in Figure 7, said part having at least 100 nucleic acid residues, or a functional part, derivative and/or analogue of said virus. Preferably said homology is at least 90%, more preferably at least 95%, more preferably at least 97% and even more preferably at least 98%. Stretches of 100 consecutive nucleic acid sequences which are more than 90.4% homologous to the VP0 sequence depicted in Figure 7 are specific for HPEV251176.

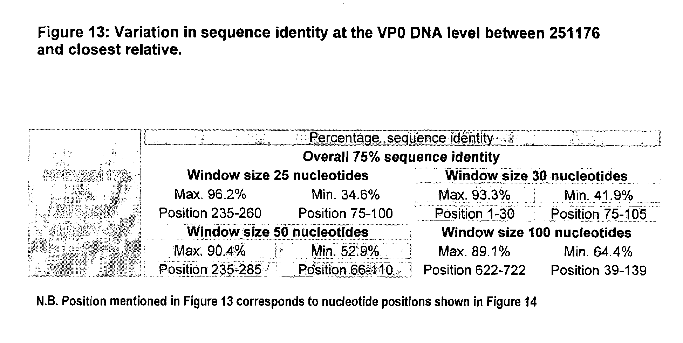

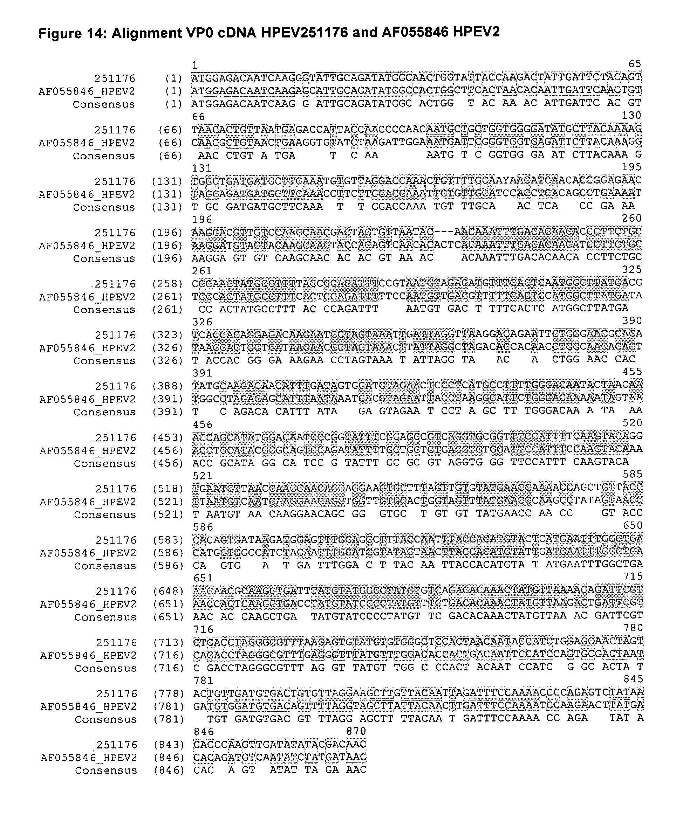

The invention furthermore provides an isolated or recombinant virus comprising a nucleic acid sequence which is more than 34.6% homologous to a nucleic acid sequence located from position 75 to 100 in the HPEV 251176 VP0 nucleic acid sequence as depicted in Figure 14, or a functional part, derivative and/or analogue of said virus. Preferably said homology is at least 40%, more preferably at least 50%, more preferably at least 60%, more preferably at least 70%, more preferably at least 80% and even more preferably at least 90%.

Furthermore provided is an isolated or recombinant virus comprising a nucleic acid sequence which is more than 41.9% homologous to a HPEV 251176 VP0 nucleic acid sequence located from position 75 to 105 in the nucleic acid sequence as depicted in Figure 14, or a functional part, derivative and/or analogue of said virus. Preferably said homology is at least 45%, more preferably at least 50%, more preferably at least 60%, more preferably at least 70%, more preferably at least 80% and even more preferably at least 90%.

Furthermore provided is an isolated or recombinant virus comprising a nucleic acid sequence which is more than 52.9% homologous to a nucleic acid sequence located from position 66 to 110 in the HPEV 251176 VP0 nucleic acid sequence as depicted in Figure 14, or a functional part, derivative and/or analogue of said virus. Preferably said homology is at least 55%, more preferably at least 60%, more preferably at least 70%, more preferably at least 80% and even more preferably at least 90%.

Furthermore provided is an isolated or recombinant virus comprising a nucleic acid sequence which is more than 64.4% homologous to a nucleic acid sequence located from position 39 to 139 in the HPEV 251176 VP0 nucleic acid sequence as depicted in Figure 7, or a functional part, derivative and/or analogue of said virus. Preferably said homology is at least 65%, more preferably at least 70%, more preferably at least 80% and even more preferably at least 90%.

A nucleic acid sequence located from position 75 to 100 in the HPEV 251176 VP0 nucleic acid sequence as depicted in Figure 14, a nucleic acid sequence located from position 75 to 105 in the HPEV 251176 VP0 nucleic acid sequence as depicted in Figure 14, a nucleic acid sequence located from position 66 to 110 in the HPEV 251176 VP0 nucleic acid sequence as depicted in Figure 14 and a nucleic acid sequence located from position 39 to 139 in the HPEV 251176 VP0 nucleic acid sequence as depicted in Figure 14 are particularly indicative for HPEV251176, since these sequences are not conserved between Parechoviral serotypes. Hence, these sequences are particularly suitable for distinguishing HPEV251176 from other Parechoviruses. For instance, a primer and/or probe capable of specifically hybridizing to at least one of said sequences is suitable for selectively amplifying and/or detecting HPEV 251176.

The homology between different parechoviruses in the UTR regions is typically high. For this reason, in this application homology is measured in a region outside the UTR regions. Moreover, the 3D gene of human parechoviruses, encoding the RNA dependent polymerase, is also highly conserved. Therefore, in this application homology is measured in a region outside the 3D gene. Preferably, homology is measured in a protein coding region other than the 3D gene.

Amino acid alterations in the genome of a HPEV 251176 virus are allowed as long as the replicating potential of the virus is not significantly inhibited. According to the invention, an amino acid sequence which is more than 79% homologous to an amino acid sequence as depicted in Figure 5 is indicative for an HPEV 251176 amino acid sequence. A virus comprising such amino acid sequence is therefore a member of the novel HPEV 251176 serotype of the present invention. The invention thus provides an isolated or recombinant virus comprising an amino acid sequence which is more than 79% homologous to an amino acid sequence as depicted in Figure 5, or a functional part, derivative and/or analogue of said virus. Preferably said homology is at least 80%, more preferably at least 85%, more preferably at least 90%, more preferably at least 95% and even more preferably at least 98%. The higher the amount of homology, the better a strain is classified as belonging to a certain strain of the HPEV 251176 serotype.

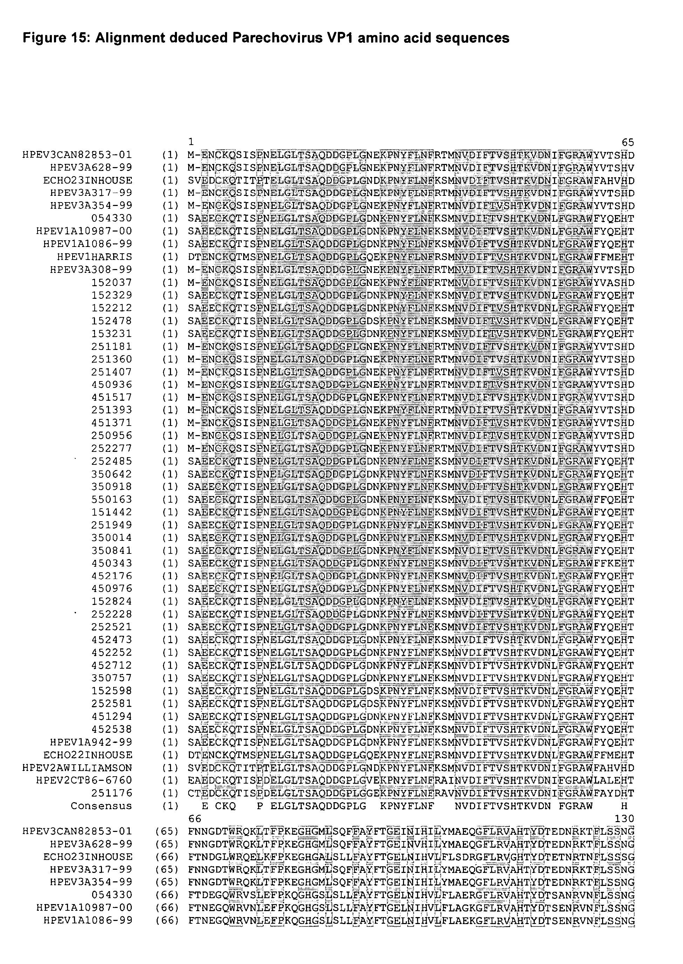

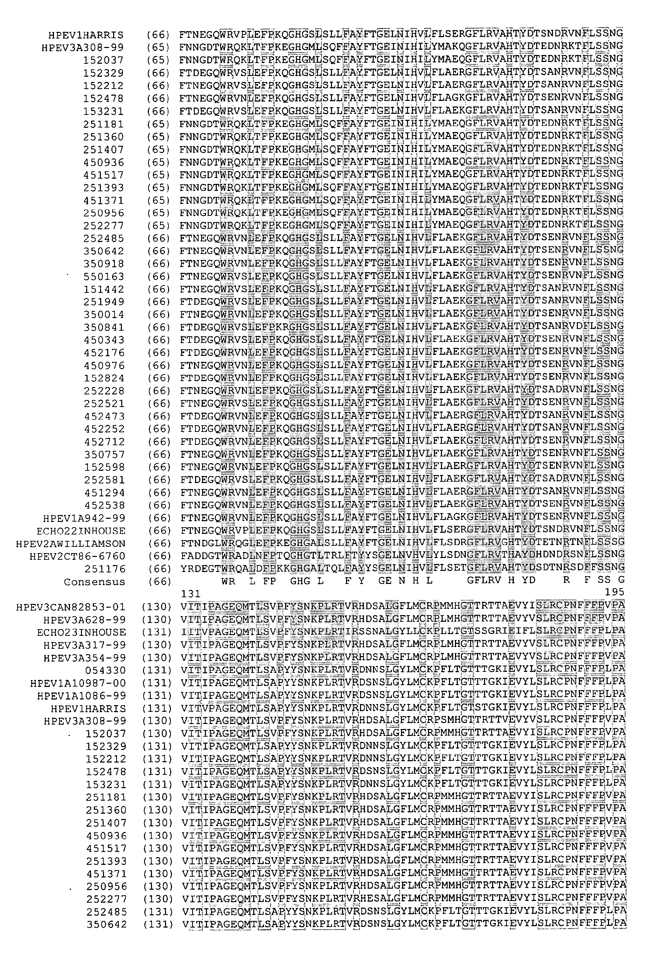

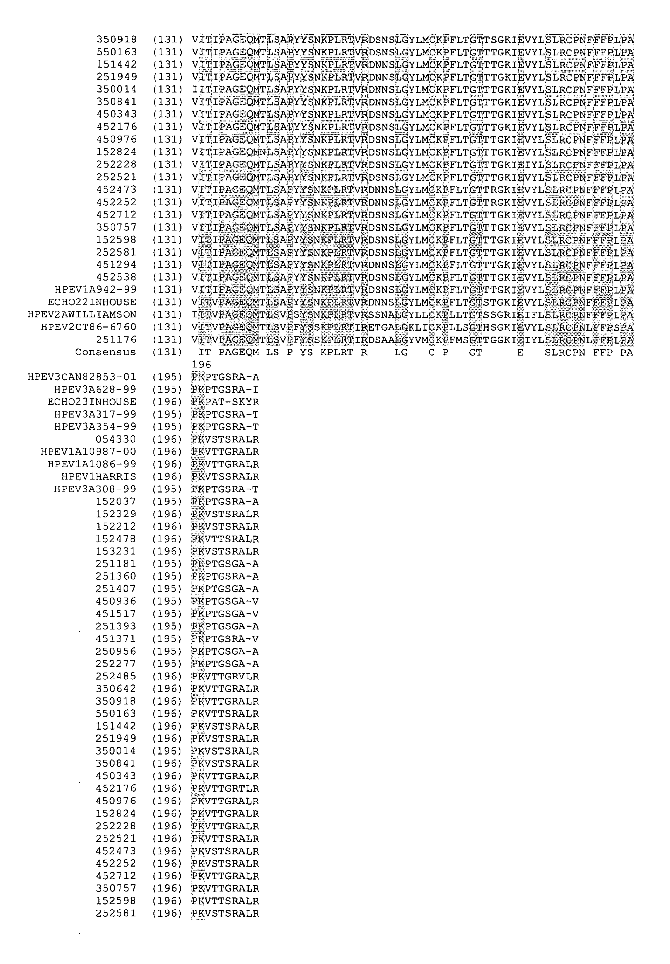

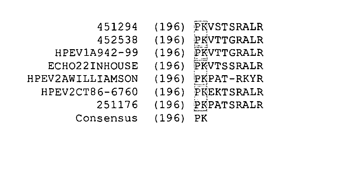

An amino acid sequence corresponding to the amino acid sequence located from position 60 to 126 in the HPEV 251176 VP1 amino acid sequence depicted in Figure 15 is particularly indicative for HPEV 251176. This amino acid sequence does not share extensive homology with any of the known Parechoviruses. An amino acid sequence which is more than 63% homologous to said amino acid sequence is indicative for HPEV251176. Further provided is therefore an isolated or recombinant virus comprising an amino acid sequence which is more than 63% homologous to an amino acid sequence located from position 60 to 126 in the HPEV 251176 VP1 amino acid sequence depicted in Figure 15, or a functional part, derivative and/or analogue of said virus. Preferably said homology is at least 65%, more preferably at least 70%, more preferably at least 80%, more preferably at least 90%, more preferably at least 95% and even more preferably at least 98%.

The amino acid sequence located from position 60 to 69 in the HPEV 251176 VP1 amino acid sequence depicted in Figure 15 is very particularly indicative for HPEV 251176. Other Parechoviral sequences are no more than 40% homologous to this sequence. An amino acid sequence which is more than 40% homologous to said amino acid sequence is therefore particularly indicative for HPEV251176. Further provided is therefore an isolated or recombinant virus comprising an amino acid sequence which is more than 40% homologous to an amino acid sequence located from position 60 to 69 in the HPEV 251176 VP1 amino acid sequence depicted in Figure 15, or a functional part, derivative and/or analogue of said virus. Preferably said homology is at least 50%, more preferably at least 60%, more preferably at least 70%, more preferably at least 80%, more preferably at least 90%, more preferably at least 95% and even more preferably at least 98%.

An amino acid sequence which is more than 82% homologous to an amino acid sequence as depicted in Figure 7 is also indicative for an HPEV 251176 amino acid sequence. A virus comprising such amino acid sequence is therefore also a member of the novel HPEV 251176 serotype of the present invention. One embodiment therefore provides an isolated or recombinant virus comprising an amino acid sequence which is more than 82% homologous to an amino acid sequence as depicted in Figure 7, or a functional part, derivative and/or analogue of said virus. Preferably said homology is at least 85%, more preferably at least 90%, more preferably at least 95% and even more preferably at least 98%. The higher the amount of homology, the better a strain is classified as belonging to a certain strain of the HPEV 251176 serotype.

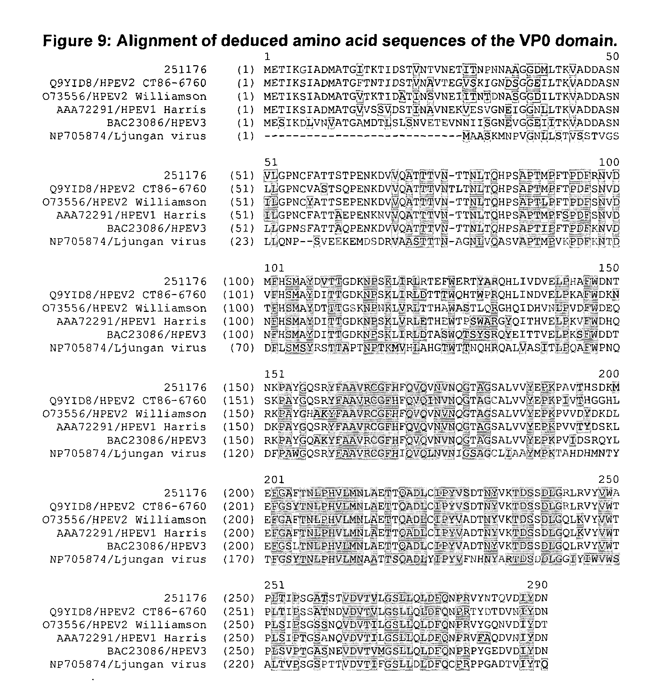

Furthermore, an amino acid sequence corresponding to the amino acid sequence located from position 123 to 205 in the HPEV 251176 VP0 amino acid sequence depicted in Figure 9 is particularly indicative for HPEV 251176. This amino acid sequence does not share extensive homology with any of the known Parechoviruses. An amino acid sequence which is more than 74.7% homologous to said amino acid sequence is indicative for HPEV251176. Further provided is therefore an isolated or recombinant virus comprising an amino acid sequence which is more than 74.7% homologous to an amino acid sequence located from position 123 to 205 in the HPEV 251176 VP0 amino acid sequence depicted in Figure 9, or a functional part, derivative and/or analogue of said virus. Preferably said homology is at least 80%, more preferably at least 90%, more preferably at least 95% and even more preferably at least 98%.

Furthermore, an amino acid sequence corresponding to the amino acid sequence located from position 24 to 40 in the HPEV 251176 VP0 amino acid sequence depicted in Figure 9 is particularly indicative for HPEV 251176. This amino acid sequence does not share extensive homology with any of the known Parechoviruses. An amino acid sequence which is more than 29.4% homologous to said amino acid sequence is indicative for HPEV251176. Further provided is therefore an isolated or recombinant virus comprising an amino acid sequence which is more than 29.4% homologous to an amino acid sequence located from position 24 to 40 in the HPEV 251176 VP0 amino acid sequence depicted in Figure 9, or a functional part, derivative and/or analogue of said virus. Preferably said homology is at least 30%, more preferably at least 40%, more preferably at least 50%, more preferably at least 70%, more preferably at least 90%, more preferably at least 95% and even more preferably at least 98%.

As outlined above, homology is preferably measured in a proteinaceous molecule other than conserved Parechoviral molecules such as the RNA dependent RNA polymerase encoded by the 3D gene.

The above mentioned amino acid sequences are particularly suitable for distinguishing HPEV251176 from other viruses. For instance, the presence of HPEV251176 is determined using a binding compound capable of specifically binding at least part of at least one of said amino acid sequences. Of course, many alternative methods for specifically detecting a given amino acid sequence are known in the art.

A functional part of a virus of the invention is defined as a part of said virus which has at least one same property as said virus in kind, not necessarily in amount. The functionality of said functional part varies with the application chosen for said functional part. For instance, if an immune response against a virus of the invention is desired, it is possible to use a functional part of said virus which is capable of eliciting an immune response which is specific for said virus. In this embodiment a functional part is used which has the same immunogenic properties in kind, not necessarily in amount. By immunogenic properties is meant the capability to induce an immune response in a host cell. Such functional part for instance comprises an epitope of said virus.

Alternatively, or additionally, a functional part of a virus of the invention is used that has a same infectivity property as said virus in kind, not necessarily in amount. In one embodiment said functional part is used in order to infect a host cell.

In one embodiment a functional part of a virus of the invention is used in order to generate a chimeric virus and/or a viral vector, for instance for vaccination and/or gene therapy purposes. A chimeric virus comprises at least part of a virus of the invention and at least part of another virus. Such virus is for instance suitable for testing effects of recombination on viral fitness, and/or for localization of HPEV251176-specific antigens. A viral vector of the invention also comprises at least part of a virus of the invention. Said vector is for instance suitable for vaccination purposes. In one embodiment, at least one sequence of a virus of the invention that is not essential for infectivity is deleted and replaced by another sequence of interest, for instance at least one (heterologous) gene of interest. Such vector is subsequently used in order to infect a host cell and to introduce said sequence of interest, for instance at least one (heterologous) gene of interest, into said host cell. Preferably, said (heterologous) gene of interest is expressed by said host cell. Such (chimeric) viruses and vectors comprise a functional part of HPEV 251176 and are thus within the scope of the present invention. Methods for generating recombinant and/or chimeric nucleic acid sequences, proteinaceous molecules, viruses, vectors and replicons are well known in the art and need no further explanation here.

A functional derivative of a virus of the invention is defined as a virus that has been altered such that at least one property of said virus is essentially the same in kind, not necessarily in amount. A derivative can be provided in many ways, for instance through conservative amino acid substitution.

A person skilled in the art is well able to generate an analogue of a virus of the invention. Such an analogue has essentially at least one same property of said virus in kind, not necessarily in amount. In one embodiment a chimeric virus is generated. Alternatively, or additionally, a virus comprising at least one chimeric proteinaceous molecule is produced. A chimeric proteinaceous molecule is defined as a molecule comprising at least one part that is derived from - and/or essentially identical to - a first proteinaceous molecule and at least one part that is derived from - and/or essentially identical to - a second proteinaceous molecule. For instance, HPEV 251176 is rendered more immunogenic by generating a cell surface associated chimeric protein (also called a cell surface associated fusion protein) comprising at least part of an HPEV 251176 surface protein and an immunogenic part that is not naturally bound to said surface protein. HPEV 251176 virus comprising such chimeric protein is preferably used for inducing an enhanced immune response in a host, for instance for vaccination purpose.

As used herein, the term "a virus of the invention" is also meant to encompass a functional part, derivative and/or analogue of said virus.

Now that the new HPEV 251176 serotype of the invention has been provided, as well as nucleic acid sequences and amino acid sequences thereof, it has become possible to generate and/or isolate a compound capable of specifically binding said virus or a functional part, derivative, analogue, nucleic acid sequence and/or amino acid sequence thereof. Such compound is for instance useful for detection of HPEV 251176 virus, or a functional part, derivative and/or analogue thereof, in a sample. This enables, amongst other things, diagnosis of infection. Such compound is furthermore suitable for many other purposes, such as isolation and/or purification of a virus of the invention. A compound of the invention, which for instance comprises an antibody or a T cell or a functional part, derivative and/or analogue thereof, is generated and/or isolated using a variety of known techniques. In one embodiment a virus of the invention - or a functional part, derivative and/or analogue thereof, preferably a nucleic acid sequence or an amino acid sequence thereof - is provided to a non-human animal and antibodies and/or T cells capable of specifically binding said virus, functional part, derivative, analogue, nucleic acid sequence and/or amino acid sequence are isolated. In one embodiment monoclonal antibodies specifically directed against a virus, functional part, derivative, analogue, nucleic acid sequence and/or amino acid sequence of the invention are produced. This is for instance performed by isolating spleen cells of said non-human animal and fusing said spleen cells with myeloma cells, resulting in hybridoma clones. These clones are screened for a clone which produces a specific antibody of interest. Said clone is preferably isolated and used for the generation of a cell line capable of producing monoclonal antibodies specifically directed against a virus, functional part, derivative, analogue, nucleic acid sequence and/or amino acid sequence of the invention.

In another embodiment, a phage display library is screened for the presence of a binding molecule capable of specifically binding a virus, functional part, derivative, analogue, nucleic acid sequence and/or amino acid sequence of the invention. Said binding molecule for instance comprises an antibody, a single chain antibody and/or a single domain antibody. After incubation of said phage display library with a virus, functional part, derivative, analogue, nucleic acid sequence and/or amino acid sequence of the invention bound phages are isolated and the attached binding molecule is identified and/or further purified using methods known in the art.

The art provides many alternative ways of generating and/or isolating a binding molecule, which methods are also suitable for the production of a compound of the invention. It is for instance possible to isolate an antibody of the invention from a sample derived from an individual infected with a HPEV 251176 virus of the invention. It is also possible to collect B-cells from said individual, select a B-cell capable of producing a desired antibody of the invention, and collect antibody produced by said B-cell. Preferably said B-cell is cultured. Alternatively, or additionally, an antibody of the invention produced by said B-cell is sequenced from this B-cell and generated artificially.

The invention thus provides an isolated, synthetic or recombinant compound capable of specifically binding a virus or a functional part, derivative and/or analogue according to the invention. Said compound is preferably capable of specifically binding an amino acid sequence of a virus or functional part, derivative or analogue according to the invention. Such compound is particularly suitable for detecting and/or isolating whole or at least partially disrupted virus particles. In one embodiment said compound is capable of specifically binding at least part of an amino acid sequence as depicted in Figure 5, Figure 6, Figure 7 and/or Figure 8, said part having at least 30 amino acid residues. More preferably, said compound is capable of specifically binding at least part of an amino acid sequence as depicted in Figure 5 and/or Figure 7, said part having at least 30 amino acid residues. Such compound is capable of specifically binding HPEV 251176 strain ECACC 05060901.

In another preferred embodiment said compound is capable of specifically binding a nucleic acid sequence of a virus or functional part, derivative or analogue according to the invention. Said nucleic acid sequence preferably comprises at least part of a nucleic acid sequence as depicted in Figure 5, Figure 6, Figure 7 and/or Figure 8, said part having at least 30 nucleotides. One preferred embodiment provides a compound of the invention capable of specifically recognizing a nucleic acid sequence as depicted in Figure 5 and/or Figure 7, said part having at least 30 nucleotides. Such compound is capable of specifically binding HPEV 251176 strain ECACC 05060901.

A compound of the invention preferably comprises a proteinaceous molecule, more preferably an antibody or a functional part, derivative and/or analogue thereof.

A functional part of an antibody is defined as a part which has the same immunogenic properties in kind, not necessarily in amount. By immunogenic properties is meant the capability to induce an immune response in a host cell. A functional part of an HPEV 251176-specific antibody is preferably capable of specifically binding an antigen of HPEV 251176. However, said functional part may bind such antigen to a different extend as compared to said whole antibody. A functional part of an antibody for instance comprises a single chain antibody, a single domain antibody or a FAB fragment. A functional derivative of an antibody is defined as an antibody which has been altered such that the immunogenic properties of said antibody are essentially the same in kind, not necessarily in amount. A derivative can be provided in many ways, for instance through conservative amino acid substitution.

A person skilled in the art is well able to generate analogous compounds of an antibody. This is for instance performed by generating a chimeric molecule comprising at least a functional part of an antibody of the invention and at least a part derived from another molecule. Said chimeric molecule of the invention for instance comprises a functional part of an antibody of the invention together with another binding moiety. An analogue has essentially the same immunogenic properties of said protein in kind, not necessarily in amount. As used herein, the term "antibody" is also meant to comprise a functional part, derivative and/or analogue of said antibody.

Once an antibody of the invention or a functional part, derivative and/or analogue thereof is obtained, it is possible to improve a desired property, such as its binding specificity and/or affinity. This is for instance done with a method wherein at least one amino acid residue is replaced by another residue, after which at least one characteristic of the resulting molecule is tested. In one embodiment an Ala-scan method is performed. This involves substitution of an original amino acid residue by alanine. Alternatively, or additionally, a replacement net mapping method is performed. In this embodiment, an original amino acid residue is replaced by any other amino acid residue. Preferably, a plurality of molecules is generated, wherein different amino acid residues are replaced. Subsequently at least one characteristic, preferably a binding characteristic, of the resulting molecules is tested. A molecule with a desired characteristic is subsequently identified and/or isolated. Said molecule is for instance used in order to isolate and/or identify a virus of the invention. Said molecule can also be used in another round of substitution and selection method.

There are many different ways in which a binding compound of the invention is generated. In a preferred embodiment the invention provides a method for producing a compound capable of specifically binding a virus or functional part, derivative or analogue according to the invention, the method comprising producing compounds capable of binding a virus or functional part, derivative and/or analogue according to the invention, and selecting a compound that is specific for said virus. It is for instance possible to generate a random or semi-random library, after which said library is screened for the presence of a compound capable of specifically binding a virus of the invention. It is also possible to generate a collection of compounds capable of binding a virus of the invention, and to screen said collection for a compound with a desired (improved) characteristic, such as an improved binding specificity and/or affinity.

A compound of the invention is capable of specifically binding HPEV 251176. Hence, a virus that is specifically bound by said compound is an HPEV 251176 virus provided by the invention. The invention therefore provides an isolated or recombinant virus which is immunoreactive with a compound according to the invention. A virus is immunoreactive with a compound of the invention if said compound comprises a binding region capable of specifically binding said virus to a higher extend as compared to the occurrence of nonspecific sticking of any virus. The invention further provides a composition of matter comprising isolated HPEV 251176 virus, and/or a functional part, derivative and/or analogue thereof. As explained hereinbefore, a certain HPEV 251176 strain may comprise several mutations compared to the HPEV 251176 strain ECACC 05060901, because the nucleotide sequences and amino acid sequences of various isolates of a virus species differ to some extend. These mutations comprise natural mutations and/or artificial mutations.

Now that a virus of the invention has been provided, a skilled person is capable of designing a primer and/or probe, capable of specifically hybridizing to a nucleic acid of a virus or functional part, derivative and/or analogue according to the invention. Such primer and/or probe is therefore also herewith provided. A primer of the invention is particularly suitable for amplifying a nucleic acid sequence of a virus of the invention. This is for instance useful for determining whether a sample comprises a virus of the invention. If present, nucleic acid of a virus of the invention is specifically amplified and the presence of an amplificate is visualized. Said amplificate is for instance visualised during an amplification reaction using a specific probe with an attached label. In this embodiment a signal of said label becomes detectable after said probe has hybridised to a complementary nucleic acid. Examples of such probes that enable real-time homogenous detection in amplification reactions are TaqMan (Heid et al; 1996) and Molecular Beacon probes (Tyagi et al; 1996 and Leone et al; 1998). Alternatively, or additionally, an amplified region is detected at the end of an amplification reaction by at least one probe that is specific for an amplified region.

A probe of the invention is particularly suitable for detection of the presence of (amplified) nucleic acid of a virus of the invention. In one embodiment, nucleic acid in a sample is amplified using at least one universal primer and/or at least one primer capable of amplifying at least two different nucleic acid sequences, where after amplified nucleic acid is incubated with a probe of the invention in order to determine whether nucleic acid of a virus of the invention has been amplified. It is of course also possible to perform a nucleic acid amplification reaction with at least one specific primer of the invention and to use a probe of the invention in order to screen for the presence of nucleic acid of a virus of the invention. In yet another embodiment, no nucleic acid amplification reaction is performed. Nucleic acid of (partly) unknown origin is incubated with a probe of the invention in order to determine whether nucleic acid of a virus of the invention is present. A primer and/or probe of the present invention is preferably capable of hybridizing to HPEV 251176 nucleic acid under stringent conditions. This enables distinction between closely related serotypes, because under stringent conditions hybridization takes only place if the sequences of the primer/probe and the nucleic acid sequence are exactly complementary over more than about 95% of the length of the probe (the exact percentage being dependent on, amongst other things, the GC percentage of the target nucleic acid and the cation concentration of the reaction mixture. The skilled person is well capable of assessing stringent conditions for a given amplification reaction).

Primers may be generated for sequences throughout the genome of HPEV 251176. However, if specific amplification of HPEV 251176 is desired, at least one primer is preferably chosen for a HPEV 251176 sequence which is at least 10% different from corresponding sequences in other Parechoviruses. Most preferably at least one primer is chosen for a HPEV 251176 sequence outside an UTR and/or the 3D gene. In a particularly preferred embodiment both primers are chosen outside the UTR and 3D gene regions. In a preferred embodiment a primer of the invention comprises sufficient sequence divergence from related parechoviruses, in order to provide specificity of HPEV 251176 sequences. In one embodiment a primer of the invention has at least one and preferably at least between 2-5 nucleotide differences compared to the sequence it intends to discriminate from, typically a related human parechovirus.

The length of a primer typically varies depending on the length of the sequence that is amplified and the reaction conditions. Typically, a primer of the invention has a length of about 10-50 nucleotides. Preferably, a primer of the invention has a length of about 15-40 nucleotides, more preferably between 15-25 nucleotides. A skilled person is well capable of determining the conditions needed for specific hybridisation of a nucleic acid to a nucleic acid of HPEV 251176. Typically the specificity increases with increasing size of overlap with the HPEV251176 nucleic acid and with increasing homology with the HPEV251176 nucleic acid.

A probe of the invention may comprise essentially the entire HPEV 251176 genome. Typically, the length of a probe of the invention is between 20 and 2000 nucleotides. Said probe preferably has a homology of at least 90%, more preferably at least 95%, most preferably at least 99% with a complementary strand of a nucleic acid of HPEV 251176. A probe of the invention is preferably comprises a label such as for instance ³²P, ³³P, ³⁵S, Cy 5 or Cy3. Said label may be either linked to said probe directly or indirectly via an intermediate.

A preferred embodiment provides a primer and/or probe, capable of specifically hybridizing to a nucleic acid sequence as depicted in Figure 5, Figure 6, Figure 7 and/or Figure 8. Most preferably a primer of probe is provided which is capable of specifically hybridizing to a nucleic acid sequence as depicted in Figure 5 and/or Figure 7. Said primer/probe is preferably capable of specifically amplifying/detecting HPEV 251176 strain ECACC 05060901. In one embodiment a primer of the invention comprises a consecutive stretch of between 10 and 50 nucleotides - preferably between 15 and 25 nucleotides - of a complementary strand of a sequence as depicted in figure 5 and/or 7. In a particularly preferred embodiment a primer and/or probe according to the invention comprises a sequence as depicted in Table 1.

Now that a virus of the invention has been provided, nucleic acid of said virus, or a part thereof, is preferably identified, isolated, purified and generated (for instance recombinantly). Said nucleic acid is obtained and used for a variety of purposes. Thus the invention further provides an isolated, synthetic or recombinant nucleic acid sequence comprising a stretch of at least 100 consecutive nucleotides of a nucleic acid of a virus of the invention, or a nucleic acid sequence that is at least 80%, preferably at least 90%, more preferably at least 95%, most preferably at least 98% homologous to a stretch of at least 100 consecutive nucleotides of a nucleic acid of a virus of the invention, wherein said stretch of at least 100 consecutive nucleotides comprises at least 50 nucleotides outside the UTR and 3D gene regions. Preferably said whole stretch of at least 100 nucleotides is outside the UTR and 3D gene regions. In a preferred embodiment a nucleic acid sequence according to the invention comprises a nucleic acid sequence that is at least 70%, more preferably at least 80%, more preferably at least 90%, most preferably at least 95%, homologous to a part of a sequence as depicted in Figure 5 and/or Figure 7, said part having at least 30 nucleic acid residues. In one embodiment a nucleic acid sequence according to the invention comprises at least part of a sequence as depicted in figure 5 or figure 7, said part having at least 30 nucleic acid residues.

One preferred embodiment provides an isolated, synthetic or recombinant nucleic acid sequence comprising a nucleic acid sequence that is complementary to a stretch of at least 100 consecutive nucleotides of a nucleic acid of a virus of the invention, or that is complementary to a nucleic acid sequence that is at least 80%, preferably at least 90%, more preferably at least 95%, most preferably at least 98% homologous to a stretch of at least 100 consecutive nucleotides of a nucleic acid of a virus of the invention, wherein said stretch of at least 100 consecutive nucleotides comprises at least 50 nucleotides outside the UTR and 3D gene regions.

Further provided is a nucleic acid sequence comprising a sequence which is more than 71% homologous to a nucleic acid sequence as depicted in Figure 5, or a functional part, derivative and/or analogue thereof. Preferably said homology is at least 80%, more preferably at least 90% and even more preferably at least 95%.

Further provided is a nucleic acid sequence comprising a sequence which is more than 88.5% homologous to at least part of a nucleic acid sequence as depicted in Figure 5, said part having at least 25 nucleic acid residues, or a functional part, derivative and/or analogue thereof. Preferably said homology is at least 90%, more preferably at least 95% and even more preferably at least 97%.

Further provided is a nucleic acid sequence comprising a sequence which is more than 53.8% homologous to a nucleic acid sequence located from position 517 to 542 in the HPEV 251176 VP1 nucleic acid sequence as depicted in Figure 20, or a functional part, derivative and/or analogue thereof. Preferably said homology is at least 60%, more preferably at least 70%, more preferably at least 80% and even more preferably at least 90%.

Further provided is a nucleic acid sequence comprising a sequence which is more than 87.1% homologous to at least part of a nucleic acid sequence as depicted in Figure 5, said part having at least 30 nucleic acid residues, or a functional part, derivative and/or analogue thereof. Preferably said homology is at least 90%, more preferably at least 95% and even more preferably at least 97%.

Further provided is a nucleic acid sequence comprising a sequence which is more than 51.6% homologous to a nucleic acid sequence located from position 238 to 268 in the HPEV 251176 VP1 nucleic acid sequence as depicted in Figure 20, or a functional part, derivative and/or analogue thereof. Preferably said homology is at least 60%, more preferably at least 70%, more preferably at least 80% and even more preferably at least 90%.

Further provided is a nucleic acid sequence comprising a sequence which is more than 83.9% homologous to at least part of a nucleic acid sequence as depicted in Figure 5, said part having at least 50 nucleic acid residues, or a functional part, derivative and/or analogue thereof. Preferably said homology is at least 90%, more preferably at least 95% and even more preferably at least 97%.

Further provided is a nucleic acid sequence comprising a sequence which is more than 56.9% homologous to a nucleic acid sequence located from position 221 to 271 in the HPEV 251176 VP1 nucleic acid sequence as depicted in Figure 20, or a functional part, derivative and/or analogue thereof. Preferably said homology is at least 60%, more preferably at least 70%, more preferably at least 80% and even more preferably at least 90%.

Further provided is a nucleic acid sequence comprising a sequence which is more than 78.2% homologous to at least part of a nucleic acid sequence as depicted in Figure 5, said part having at least 100 nucleic acid residues, or a functional part, derivative and/or analogue thereof. Preferably said homology is at least 80%, more preferably at least 85%, more preferably at least 90% and even more preferably at least 97%.

Further provided is a nucleic acid sequence comprising a sequence which is more than 63.4% homologous to a nucleic acid sequence located from position 221 to 321 in the HPEV 251176 VP1 nucleic acid sequence as depicted in Figure 20, or a functional part, derivative and/or analogue thereof. Preferably said homology is at least 70%, more preferably at least 80% and even more preferably at least 90%.

Further provided is a nucleic acid sequence comprising a sequence which is more than 75% homologous to a nucleic acid sequence as depicted in Figure 7. Preferably said homology is at least 80%, more preferably at least 90%, more preferably at least 95% and even more preferably at least 97%.

Further provided is a nucleic acid sequence comprising a sequence which is more than 96.2% homologous to at least part of a nucleic acid sequence as depicted in Figure 7, said part having at least 25 nucleic acid residues, or a functional part, derivative and/or analogue thereof. Preferably said homology is at least 97% and even more preferably at least 98%.

Further provided is a nucleic acid sequence comprising a sequence which is more than 34.6% homologous to a nucleic acid sequence located from position 75 to 100 in the HPEV 251176 VP0 nucleic acid sequence as depicted in Figure 14, or a functional part, derivative and/or analogue thereof. Preferably said homology is at least 40%, more preferably at least 50%, more preferably at least 60%, more preferably at least 70%, more preferably at least 80% and even more preferably at least 90%.

Further provided is a nucleic acid sequence comprising a sequence which is more than 93.3% homologous to at least part of a nucleic acid sequence as depicted in Figure 7, said part having at least 30 nucleic acid residues, or a functional part, derivative and/or analogue thereof. Preferably said homology is at least 95%, more preferably at least 97%, more preferably at least 98% and even more preferably at least 99%.

Further provided is a nucleic acid sequence comprising a sequence which is more than 41.9% homologous to a nucleic acid sequence located from position 75 to 105 in the HPEV 251176 VP0 nucleic acid sequence as depicted in Figure 14, or a functional part, derivative and/or analogue thereof. Preferably said homology is at least 50%, more preferably at least 60%, more preferably at least 70%, more preferably at least 80% and even more preferably at least 90%.

Further provided is a nucleic acid sequence comprising a sequence which is more than 90.4% homologous to at least part of a nucleic acid sequence as depicted in Figure 7, said part having at least 50 nucleic acid residues, or a functional part, derivative and/or analogue thereof. Preferably said homology is at least 95%, more preferably at least 97% and even more preferably at least 98%.

Further provided is a nucleic acid sequence comprising a sequence which is more than 52.9% homologous to a nucleic acid sequence located from position 66 to 110 in the HPEV 251176 VP0 nucleic acid sequence as depicted in Figure 14, or a functional part, derivative and/or analogue thereof. Preferably said homology is at least 60%, more preferably at least 70%, more preferably at least 80% and even more preferably at least 90%.

Further provided is a nucleic acid sequence comprising a sequence which is more than 89.1% homologous to at least part of a nucleic acid sequence as depicted in Figure 7, said part having at least 100 nucleic acid residues, or a functional part, derivative and/or analogue thereof. Preferably said homology is at least 90%, more preferably at least 95% and even more preferably at least 97%.

Further provided is a nucleic acid sequence comprising a sequence which is more than 64.4% homologous to a nucleic acid sequence located from position 39 to 139 in the HPEV 251176 VP0 nucleic acid sequence as depicted in Figure 14, or a functional part, derivative and/or analogue thereof. Preferably said homology is at least 65%, more preferably at least 70%, more preferably at least 80% and even more preferably at least 90%.

Further provided is a vector and/or a gene delivery vehicle comprising a nucleic acid sequence according to the present invention and a host cell comprising said vector.

Nucleic acid of a virus of the invention encodes various proteins. Now that a virus of the invention and nucleic acid thereof has been isolated, it is for instance used for expressing said proteins. These proteins are for instance expressed in cells producing a virus of the invention and/or in expression systems that are transformed with a nucleic acid of the invention encoding at least part of a HPEV 251176 protein. The invention thus further provides an amino acid sequence encoded by a nucleic acid sequence according to the invention. Preferably an isolated, synthetic or recombinant amino acid sequence of the invention comprises at least part of a sequence as depicted in Figure 5, Figure 6 or Figure 7, said part having at least 30 amino acid residues, or a functional part, derivative and/or analogue thereof. Most preferably an amino acid sequence of HPEV 251176 other than the 3D gene encoded RNA polymerase is provided. One preferred embodiment provides an isolated, synthetic or recombinant amino acid sequence comprising at least part of a VP1 and/or VP0 sequence as depicted in Figure 5 and/or Figure 7, said part having at least 30 amino acid residues, or a functional part, derivative and/or analogue thereof.

Many different variants of an amino acid sequence of the invention having at least one same function in kind, not necessarily in amount, are present in nature and are produced artificially, as explained before. The invention therefore further provides an isolated, synthetic or recombinant amino acid sequence comprising an amino acid sequence that is at least 80%, preferably at least 85%, more preferably at least 90%, most preferably at least 95% homologous to a sequence as depicted in Figure 5 and/or Figure 7 or a functional part, derivative and/or analogue thereof.

Further provided is an amino acid sequence comprising a sequence which is more than 63% homologous to an amino acid sequence located from position 60 to 126 in the HPEV 251176 VP1 amino acid sequence depicted in Figure 15. Preferably said homology is at least 65%, more preferably at least 70%, more preferably at least 80%, more preferably at least 90%, more preferably at least 95% and even more preferably at least 98%.

Further provided is an amino acid sequence comprising a sequence which is more than 40% homologous to an amino acid sequence located from position 60 to 69 in the HPEV 251176 VP1 amino acid sequence depicted in Figure 15. Preferably said homology is at least 50%, more preferably at least 60%, more preferably at least 70%, more preferably at least 80%, more preferably at least 90%, more preferably at least 95% and even more preferably at least 98%.

Further provided is an amino acid sequence comprising a sequence which is more than 74.7% homologous to an amino acid sequence located from position 123 to 205 in the HPEV 251176 VP0 amino acid sequence depicted in Figure 9. Preferably said homology is at least 80%, more preferably at least 90%, more preferably at least 95% and even more preferably at least 98%.

Further provided is an amino acid sequence comprising a sequence which is more than 29.4% homologous to an amino acid sequence located from position 24 to 40 in the HPEV 251176 VP0 amino acid sequence depicted in Figure 9. Preferably said homology is at least 30%, more preferably at least 40%, more preferably at least 50%, more preferably at least 70%, more preferably at least 90%, more preferably at least 95% and even more preferably at least 98%.

As a result of the fact that a given amino acid sequence can be encoded by a variety of codons, an amino acid sequence of the invention is encoded by a variety of different nucleic acid sequences. The invention therefore further provides a nucleic acid sequence encoding an amino acid sequence according to the invention.

A virus or a functional part, derivative and/or analogue according to the invention is suitable for determining whether a sample comprises a virus of the invention. A complementary strand of at least part of a nucleic acid sequence of said virus or functional part, derivative and/or analogue, said part having at least 7 nucleotides, more preferably at least 10 nucleotides, is for instance used in a hybridization reaction with nucleic acid of a sample. If said complementary strand appears to hybridize to nucleic acid present in said sample, preferably under stringent conditions, it indicates that nucleic acid of a virus of the invention is present in said sample. A primer and/or probe of the invention is also suitable for determining whether a sample comprises nucleic acid of a virus of the invention. If said primer and/or probe appears to hybridize to nucleic acid present in said sample, preferably under stringent conditions, it indicates that nucleic acid of a virus of the invention is present in said sample. Furthermore, a compound of the invention capable of specifically binding a virus, nucleic acid sequence and/or amino acid sequence of the invention is also suitable for detecting at least part of a virus of the invention in a sample. For instance, in one embodiment virus particles, viral nucleic acid and/or viral amino acid sequences are detected via a binding assay using a binding compound of the invention. After incubation of a compound of the invention with a sample, unbound constituents of said sample are washed away and if bound (parts of a) virus of the invention are present, they are visualised using any well known method of the art.

The invention thus further provides a method for detecting a virus and/or a functional part, derivative, analogue and/or nucleic acid sequence according to the invention in a sample, comprising hybridizing and/or amplifying a nucleic acid of said virus or functional part, derivative or analogue with a primer, probe and/or nucleic acid sequence according to the example and detecting hybridized and/or amplified product. A use of a virus, functional part, derivative, analogue, compound, primer, probe and/or nucleic acid sequence according to the invention for detecting a virus according to the invention in a sample is also herewith provided.

It is also possible to determine whether an individual comprises a compound, for instance an antibody and/or a T cell, capable of specifically binding at least part of a virus of the invention. The presence of such binding compound indicates that said individual has been infected by an HPEV 251176 virus of the present invention. A virus or functional part, derivative, analogue and/or an amino acid sequence according to the invention is particularly suitable for determining whether a sample comprises a binding compound specific for a virus of the invention. This is for instance in one embodiment performed using the same kind of binding assay as described above: a virus, functional part, derivative, analogue and/or amino acid sequence of the invention is incubated with a sample, unbound constituents are washed away and a visualisation reaction is performed in order to determine whether compounds capable of specifically binding a virus and/or amino acid sequence of the invention are bound to said virus, functional part, derivative, analogue and/or amino acid sequence. A use of a virus, functional part, derivative, analogue and/or an amino acid sequence according to the invention for detecting a compound capable of specifically binding a virus and/or amino acid sequence of the invention in a sample is therefore also herewith provided

In a preferred embodiment said method is performed in order to determine whether a sample of an individual comprises a T cell and/or an antibody capable of specifically binding a virus of the invention or a nucleic acid sequence or an amino acid sequence thereof. Additionally, or alternatively, a method of the invention is preferably used in order to determine whether a sample of an individual comprises a ligand such as an IgG, IgM and/or IgA that is specific for a virus of the invention. The presence of such T cell, antibody and/or ligand indicates that said individual has been infected by an HPEV 251176 virus. In one embodiment it is determined whether an individual has been infected by HPEV 251176 strain ECACC 05060901.

HPEV 251176 is associated with fever, infections of the gastrointestinal or the upper respiratory tract, neonatal sepsis and meningitis, especially in immunocompromised individuals. In one embodiment the invention therefore further provides an isolated, synthetic and/or recombinant virus of the invention or a functional part, derivative and/or analogue thereof comprising the capability of inducing an HPEV 251176 related disease and/or HPEV 251176 related symptom in a subject. Said subject preferably comprises a human individual.

Now that a virus of the invention has been provided, it has become possible to prepare an immunogenic composition, a vaccine and/or a medicament. For instance, a whole virus of the invention is used. In order to at least partly prevent replication and spreading of the virus, involving HPEV 251176 related disease, said administered virus is preferably attenuated or inactivated. Administration of a virus of the invention to a subject will elicit an immune response in said subject. If said subject is a non-human animal, said immune response is preferably used for obtaining a compound capable of specifically binding a virus, nucleic acid sequence and/or amino acid sequence of the invention.

In one embodiment an immune response in a subject against HPEV 251176 provides said subject with at least partial protection against a virus of the invention. Hence, if said subject is subsequently infected by a virus of the invention, replication and/or spreading of said virus is at least in part counteracted by the subject's immune response. It is also possible to provide a subject that is already suffering from an HPEV 251176-related disease with an HPEV 251176 virus of the invention in order to enhance the subject's immune response. In one embodiment a nucleic acid sequence and/or an amino acid sequence of a HPEV 251176 virus is used in order to elicit and/or enhance an immune response in a subject and, hence, to better prevent and/or counteract an HPEV 251176-related disease. Additionally, or alternatively, a compound capable of specifically binding a virus, functional part, derivative, analogue, nucleic acid sequence and/or amino acid sequence of the invention is used. Said compound is suitable for (temporary) use as a vaccine and/or medicament. As long as said compound is circulating in an individual, it is capable of at least in part counteracting a virus of the invention.

The invention therefore provides a virus, functional part, derivative, analogue, compound, a nucleic acid sequence and/or amino acid sequence according to the invention for use as a vaccine or medicament. Said vaccine or medicament is particularly suitable for preventing and/or counteracting a disease that is involved with the presence of a Parechovirus. One embodiment therefore provides a use of a virus, functional part, derivative, analogue, compound, nucleic acid sequence and/or amino acid sequence according to the invention for the preparation of a vaccine or medicament against a Parechoviral genus related disease. Most preferably said Parechoviral related disease comprises an HPEV 251176 related disease. A use of a virus, functional part, derivative, analogue, compound, nucleic acid sequence and/or amino acid sequence according to the invention for the preparation of a vaccine or medicament against an HPEV 251176 related disease is therefore also herewith provided.

An immunogenic composition comprising a virus, functional part, derivative, analogue, compound, nucleic acid sequence and/or amino acid sequence according to the invention is also herewith provided, as well as a medicament comprising a virus, functional part, derivative, analogue, compound, nucleic acid sequence and/or amino acid sequence according to the invention. In one preferred embodiment an immunogenic composition of the invention comprises a vaccine capable of providing at least partial protection against an HPEV 251176 related disease. Most preferably said vaccine is capable of providing full protection against an HPEV 251176 related disease,

which means that subsequent infection with a virus of the invention does essentially not result in an HPEV 251176 related disease.

Immunogenic compositions are generated in a variety of ways. One way is to culture an HPEV 251176 virus, for example on Vero-, HEL- and/or tertiary monkey kidney cells, and to use attenuated and/or inactivated virus harvested from the culture. In one embodiment at least one isolated or synthetic epitope of HPEV 251176 is used. Such epitope is for instance produced using a conventional peptide synthesis method. Known methods for the generation of Parechovirus vaccines are for instance adapted in order to produce an immunogenic composition comprising HPEV 251176 or a functional part, derivative, analogue, amino acid and/or nucleic acid thereof. An immunogenic composition, vaccine and/or medicament of the invention preferably comprises a suitable adjuvant, carrier and/or diluent. Suitable adjuvants, carriers and diluents are well known in the art. For instance, Freund's Complete Adjuvant (FCA), Montanide ISA Adjuvants [Seppic, Paris, France], Ribi's Adjuvants (Ribi ImmunoChem Research, Inc., Hamilton, MT), Hunter's TiterMax (CytRx Corp., Norcross, GA), Aluminum Salt Adjuvants (e.g. Alhydrogel - Superfos of Denmark) or Gerbu Adjuvant [Gerbu Biotechnik GmbH, Gaiberg, Germany) are suitable adjuvants. A suitable carrier for instance comprises a saline solution.

An immunogenic composition and/or a medicament is particularly suitable for treating an infected individual who is suffering from, or at risk of suffering from, an HPEV 251176 related disease. The invention therefore provides a method for treating an individual suffering from, or at risk of suffering from, a Parechovirus 251176 related disease, comprising administering to said individual an immunogenic composition or a medicament according to the invention. In one embodiment, an immunogenic composition and/or a medicament of the present invention is used together with a known antiviral medicament such as Pleconaril and/or Ribavirin. Said known medicament is for instance administered before and/or after administration of an immunogenic composition or a medicament of the present invention. In one embodiment said known medicament is administered together with an immunogenic composition or a medicament of the present invention.

An immunogenic composition of the invention is furthermore particularly suitable for at least in part preventing an HPEV 251176 related disease. The invention therefore furthermore provides a method for at least partial prophylaxis of a Parechovirus 251176 related disease, comprising administering an immunogenic composition according to the invention to an individual.

Dose ranges of a virus, functional part, derivative, analogue, compound, nucleic acid sequence and/or amino acid sequence according to the invention to be used in a therapeutical application as described herein before are designed on the basis of rising dose studies in the clinic in clinical trials for which rigorous protocol requirements exist.

A virus, functional part, derivative, analogue, compound, nucleic acid sequence and/or amino acid sequence according to the invention is also suitable for diagnosis of a Parechoviral genus related disease, especially an HPEV 251176 related disease. As already explained above, a virus, functional part, derivative, analogue, compound, nucleic acid sequence and/or amino acid sequence according to the invention is suitable for detecting at least part of a virus (for instance a nucleic acid sequence and/or an amino acid sequence derived from said virus) in a sample. The presence of at least part of a virus of the invention in a sample derived from an individual indicates that said individual suffers from, or is at risk of suffering from, a Parechoviral genus related disease. The invention therefore provides a use of a virus, functional part, derivative, analogue, compound, primer, probe, nucleic acid sequence and/or amino acid sequence according to the invention for diagnosis of a Parechoviral genus related disease. Preferably said Parechoviral genus related disease comprises a Parechovirus 251176 related disease.

As mentioned before, a virus of the invention as well as a nucleic acid sequence and an amino acid sequence derived thereof are particularly suitable for detection of the presence of a virus of the invention in a sample. A compound capable of specifically binding a virus of the invention or an amino acid sequence or nucleic acid sequence derived thereof is also suitable for detecting HPEV 251176. This enables diagnosis of a Parechoviral genus related disease, especially an HPEV 251176 related disease. The invention therefore provides a diagnostic kit comprising a virus, functional part, derivative, analogue, binding compound, primer, probe, nucleic acid sequence and/or amino acid sequence according to the invention.

One embodiment provides a method for determining whether an individual suffers from a Parechovirus genus related disease, preferably a Parechovirus 251176 related disease, comprising obtaining a sample from said individual and detecting a Parechovirus, preferably a Parechovirus 251176 virus, or a functional part, derivative or analogue thereof in said sample with a method and/or a diagnostic kit according to the invention.

The invention is further illustrated by the following examples. The examples do not limit the scope of the invention in any way.

### Examples

### Sequencing of the genome of Parechovirus 251176

Pairs of suitably oriented consensus primers (Table 1), derived from full genome alignment of all known Parechoviruses, were used to amplify partially overlapping RT-PCR fragments. The collection of cDNA fragments collectively represented the complete HPEV251176 genome with the exception of the 5'-and 3'-untranlated regions. The 3'-end of the genome was amplified using a combination of JZH and HPEVx6556F following cDNA synthesis primed with the JHZ-tagged oligo-dT (Table 1) oligonucleotides. The 5'-end was obtained using primers GSP1 and 2 according to the 5'-RACE protocol referred to in the Materials and Methods section.
RT-PCR product containing plasmids were sequenced with the BigDyeTM Terminator Cycle Sequencing Kit (Applied Biosystems, Foster City, California), initially using its -21 M13 and M13 reverse primers. Electrophoresis of sequencing reaction mixtures was performed with an Applied Biosystems 377 automated sequencer, following the manufacturer's protocols. The ends of the sequences thus obtained are used to deduce additional "walking primers" to generate additional overlapping sequence data. The complete genome sequence is deduced by assembling these partially overlapping nucleotide sequences into a single contig using for example the Vector NTI suite 7 software package.

### Materials and Methods

### Nucleic extraction and RT-PCR

Nucleic acid extraction from clinical faeces samples for screening and RT-PCRs were performed as previously described¹. The Parechovirus VP1 domains were amplified with primer pair VP1_echoF₁/R₁ and the 5'-UTR with the nested primer pairs A0013/A0014 and A0015/A0016. The thermal cycling program for the VP1 domain was 3 min 94°C followed by 35 cycles of 30 sec 94°C, 30 sec 42°C and 30 sec 60°C. A final cycle of 5 min 72°C was performed before cooling the mixture to 4°C. The first PCR in the 5 min-UTR amplification go after the thermal cycling protocol of 5 min 95°C followed by 35 cycles of 1 min 95°C, 1 min 55°C and 2 min 72°C and a final step of 10 min at 72°C. Five microliters of the first PCR reaction were transferred to a nested PCR and subjected to 5 min 95°C, 25 cycles of 1 min 95°C, 1 min 55°C and 2 min 72°C and the cycling scheme ended with 10 min at 72°C prior to cooling to 4°C.

**Table 1: RT-PCR primers**

| Primer | Sequence | Amplicon |
|---|---|---|
| A0013 | GCYCACACAGCCATCCTCTAGTAAG | 357 bp |
| A0014 | GGYTTGGCCCRCTAGACGTT | |
| A0015 | AGCATCCYANTGCCAGCGGA | 222 bp |
| A0016 | GCCCCAGATCAGATCCAYAGTG | |
| VP1_echoF₁ | CCAAAATTCRTGGGGTTC | 691 bp |
| VP1_echoR₁ | AAACCYCTRTCTAAATAWGC | |
| VP1_echoF₀ | TTYTCMACWTGGATGAGGAARAC | |
| VP1_echoR₀ | CCWGTYAAWGCTGTYTTRAAAATRTC | |
| HPEVx 6522F | AGATTGGCACTTYATGATTAATGC | |
| HPEVx6545R | GCATTAATCATRAAGTGCCAATCT | |
| HPEVx 6556F | TACAATTATGARATGGAYTAYTCYCA | |
| HPEVx6581R | TGRGARTARTCCATYTCATAATTGTA | |
| HPEVx 4951F | CAAATGGARGCYTTYATNGAACC | |
| HPEVx 4861F | GATGCWGTNTCWTACATYAAGCA | |
| HPEVx 4883R | TGCTTRATGTAWGANACWGCATC | |
| HPEVx 6522F | AGATTGGCACTTYATGATTAATGC | |
| HPEVx6545R | GCATTAATCATRAAGTGCCAATCT | |
| GSP1 | TCAGATCCAYAGTGTCACTTGTTAC | 5'-UTR |
| GSP2 | GCATCCTTCGTGGGCCTTACAACTA | |
| JZHoligodT | GCTATCATCACAATGGACTTTTTTTTTTTTTTTTTTV | 3'-UTR |
| JZH1 | GCTATCATCACAATGGAC | |

For sequence analysis of the full-length Parechovirus 251176 genome, RNA was extracted from cell culture supernatant with Machery-Nagel NucleoSpin RNA II kit (BIOKE) according to the manufacturers' instructions. Using combinations of the consensus primers in Table 1, partially overlapping PCR fragments were generated, covering the complete genome. The 3' of the parechoviral genome was amplified with primer pair HPEVx 6522F/JZH1 following cDNA synthesis with a JZH-tagged oligo-dT. The 5' end of the genome was amplified with the GSP1 and 2 primers with the Invitrogen 5' RACE for Rapid Amplification of cDNA Ends, Version 2.0 according to the instruction of its manufacturer.
RT-PCR product containing plasmids were sequenced with the BigDyeTM Terminator Cycle Sequencing Kit (Applied Biosystems, Foster City, California), initially using its -21 M13 and M13 reverse primers. Electrophoresis of sequencing reaction mixtures was performed with an Applied Biosystems 377 automated sequencer, following the manufacturer's protocols. The ends of the sequences thus obtained are used to deduce additional "walking primers" to generate additional overlapping sequence data. The complete genome sequence is deduced by assembling these partially overlapping nucleotide sequences into a single contig using for example the Vector NTI suite 7 software package.

### Phylogenetic analysis

Sequences were aligned using Clustal-W¹² included in the Vector NTI suite 7 software package (InforMax, Bethesda, U.S.A.). Phylogenetic analyses were performed by the Neighbour-Joining (NJ) method⁹ as implemented in the MEGA 3.0 software package⁶. Kimura's 2-parameter distances⁵ were estimated for the nucleotide sequences, and P-distances were used for amino acid sequences. Thousand bootstrap replicates were analyzed. The use of other methods for distance estimation did not influence the tree topology. Gaps introduced for optimal alignment were not considered informative and hence excluded from the analyses. Similarity plots of these alignments were generated using the SimPlot v2.5 software⁷.

### Virus propagation in cell culture

Clinical faecal samples were resuspended (30% v/v) in medium B (25 g/L Nutrient Broth No. 2 Oxoid, 250 u/mL Penicillin, 250 µg/mL Streptomycin, 1.5 µg/mL Amphotericin B). The growth medium was removed from tubes containing cultured Vero-, HEL and tertiary monkey kidney cells with a heat-sterilized platinum needle medium-sucking device. Faeces, stored in medium B, were centrifuged at 2500 RPM for 10 min and 1 mL of the resulting supernatant was filtered through a 45 µM syringe-mounted filter. After inoculation, the closed tubes were gently inverted to ensure contact between the patient material and the cells attached to the glass wall. The tubes incubated for 30 min at 37°C. MEM Hanks 8% FCS (Gibco) solution was equilibrated at room temperature and 0.375 µg/mL Amphotericin B and 10 mg/mL Neomycin (Gibco) were added. To each tube 1 mL MEM Hanks 8% FCS was added and the tubes were incubated at 37°C. Twice a week for at least two weeks, the cells were microscopically observed and any virus-specific cytopathic effect (CPE) was scored.

### Results

To identify new viruses, clinical faeces samples collected between December 2000 and January 2005 in the Academic Medical Centre (AMC) of the University of Amsterdam were routinely inoculated on Vero-, HEL- and tertiary monkey kidney cells and analysed with an enterovirus RT-PCR¹. Samples that exhibited a cytopathic effect (CPE) in cell culture, but remained negative in the enterovirus RT-PCR were screened with consensus primers targeting the Parechovirus 5'-UTR and VP1 regions.

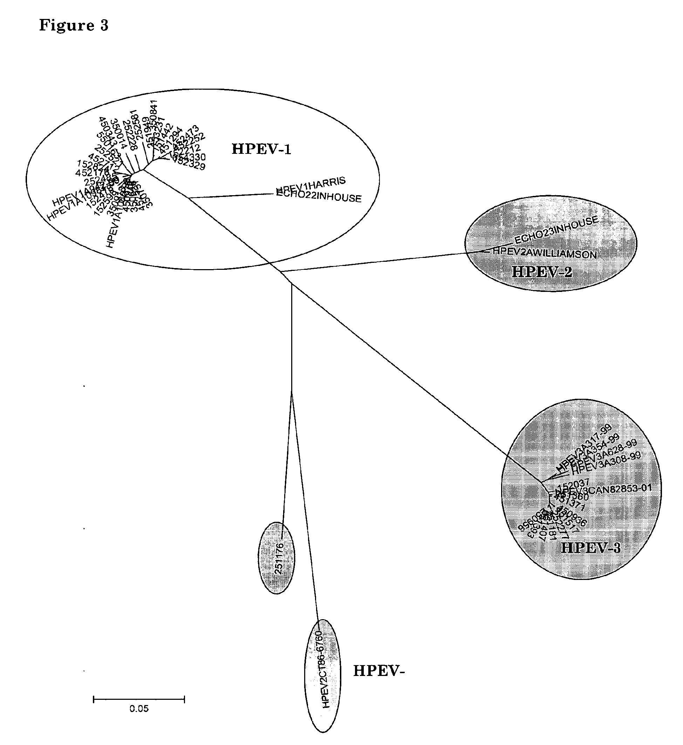

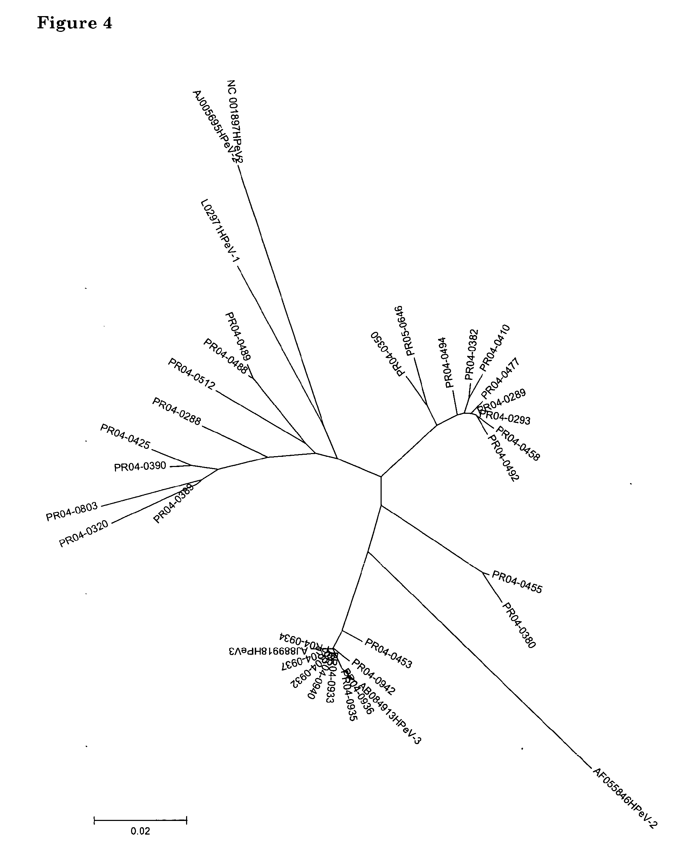

All PCR products corresponding to the predicted sizes were cloned and sequenced. The resulting contigs were aligned and subjected to phylogenetic analysis using the corresponding domains from HPEV-1, 2 and 3 as references (Fig. 3 and 4). However, both 5'-UTR and VP1 based phylogenetic trees revealed clusters that were not compatible with the current taxonomy, demonstrating the discovery of a novel Parechovirus serotype(s).

### Brief description of the drawings

**Figure 1: Genome structure HPEV-1, 2 and 3**
   Parechoviruses have the typical Picornavirus genomic RNA containing a large single open reading frame flanked by non-translated 5' and 3'-regions. The resulting polyprotein is proteolytically processed by the 3C encoded protease (3Cₚᵣₒ). VP0, VP3 and VP1 constitute the structural proteins. Many of the parechovirus non-structural proteins could also be identified by using sequence alignments. Both the RNA-dependent RNA-polymerase (3Dₚₒₗ) and 3Cₚᵣₒ, the trypsin family protease responsible for most processing of the picornavirus polyprotein, are easily recognized and the critical motifs thought to be involved in catalytic activity (YGDD and GXCGG, respectively) are conserved in the currently known family members. Polypeptide 2C, with proposed helicase activity in other picornaviruses, is also relatively well conserved between HPEV1, 2 and 3 and other representatives of the family. On the other hand, 2A, 2B, and 3A exhibit only limited identity with those of other picornaviruses, thus making their precise identification in the polyprotein by sequence alignment more difficult.
**Figure 2: Phylogenetic analysis of the genus Parechovirus**
   The reference genomes are indicated with their GenBank accession number and serotype name. The Parechovirus genus currently contains two species: human Parechovirus and Ljunganvirus infecting bank voles (*Clethrionomys glareolus*). Two human Parechovirus serotypes, HPEV-1³ and HPEV-2 were formerly classified in the enterovirus genus as echovirus 22 and 23⁸, respectively. The fact that one of the HPEV-2 serotypes (AJ005695) seems more closely related to the HPEV-1 both in the 5'-UTR as well as in the rest of the genome (data not shown) hints that Parechovirus taxonomy will be revised in the future. Recently, a third serotype, associated with gastroenteritis, exanthema or respiratory illness, has been proposed under the name Parechovirus 3 (HPEV-3)⁴
**Figure 3: VP1 based phylogenetic tree**
   The deduced amino acid sequences of most AMC clinical isolates cluster with one of type strains of HPEV-1, 2 or 3. However, isolate 251176 is only distantly related to the Echo 23 (HPEV-2) Connecticut isolate.
**Figure 4: 222 bp 5'-UTR based phylogenetic tree**
   Using a nested RT-PCR assay, 222 bp 5'-UTR fragments were amplified from 235 faeces samples collected between collected between October 2003 and January 2004. In total 21 samples yielded amplicons of the predicted size. This screening revealed more Parechovirus-specific amplicons the VP1 screen and also here deviant nucleotide sequences were obtained. Unfortunately, the lower virus titers in samples PR04-0380 and PR04-488 impede cloning of additional fragments of these potentially new Parechoviruses.
**Figure 5:** Sequences of partial Parechovirus 251176 VP1 region

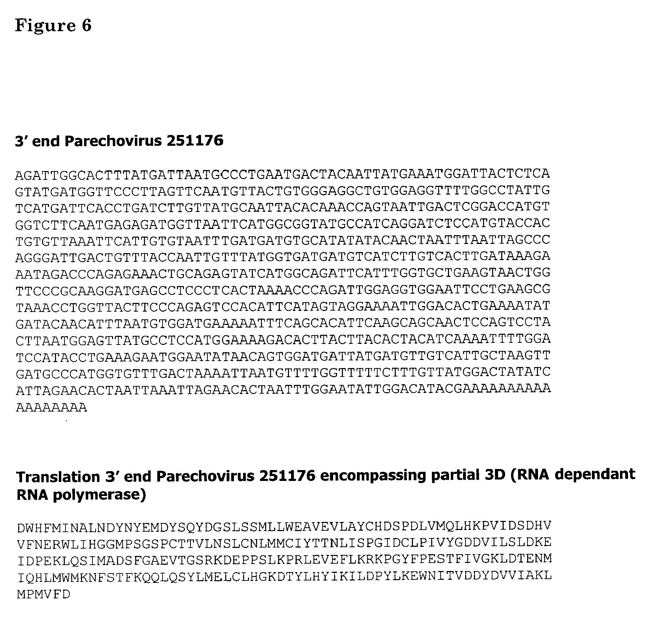

**Figure 6:** Sequences of 3' end Parechovirus 251176
**Figure 7:** Sequences of Parechovirus 251176 VP0 domain.

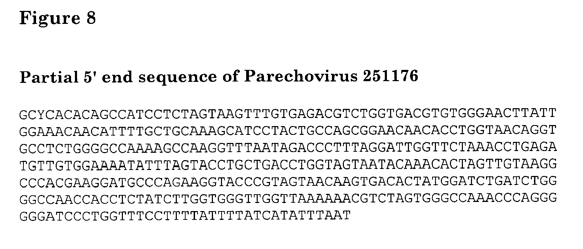

**Figure 8:** 5' end of Parechovirus 251176
**Figure 9:** Alignment of deduced amino acid sequences of the VP0 domain

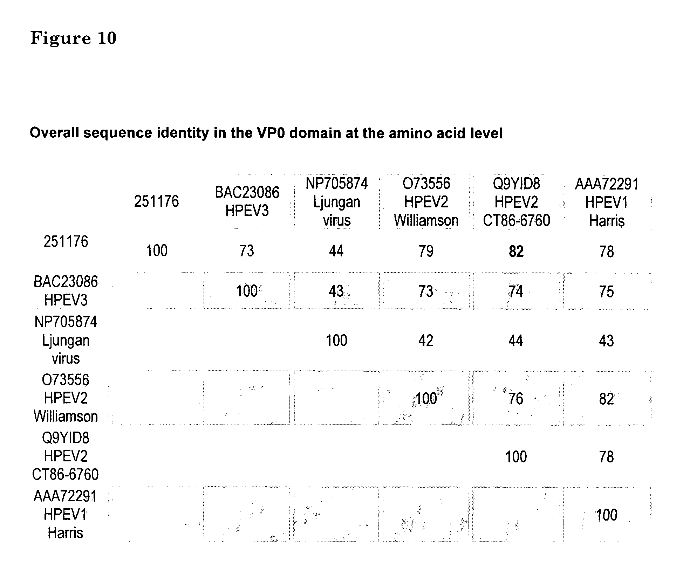

**Figure 10:** Overall sequence identity in the VP0 domain at the amino acid level
**Figure 11:** Alignment cDNA sequences Parechovirus VP0 domain
**Figure 12:** Overall sequence identity Parechovirus VP0 domain at the DNA level
**Figure 13:** Variation in sequence identity at the VP0 DNA level between 251176 and closest relative
**Figure 14:** Alignment VP0 cDNA HPEV251176 and AF055846 HPEV2
**Figure 15:** Alignment deduced Parechovirus VP1 amino acid sequences

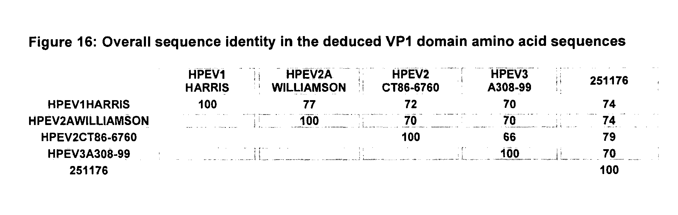

**Figure 16:** Overall sequence identity in the deduced VP1 domain amino acid sequences
**Figure 17:** Alignment of VP1 encoding PCR products
**Figure 18:** Overall sequence identity of Parechovirus VP1 encoding domains
**Figure 19:** Variation in sequence identity at the VP1 cDNA level between 251176 and closest relative
**Figure 20:** Alignment VP1 encoding cDNA PCR products HPEV251176 and AF055846 HPEV2

### References

1. Beld, M., R. Minnaar, J. Weel, C. Sol, M. Damen, A. H. van der, P. Wertheim-van Dillen, A. van Breda, and R. Boom. 2004. Highly sensitive assay for detection of enterovirus in clinical specimens by reverse transcription-PCR with an armored RNA internal control. J. Clin. Microbiol. 42:3059-3064.
2. Boivin, G., Y. Abed, and F. D. Boucher. 2005. Human parechovirus 3 and neonatal infections. Emerg. Infect. Dis. 11:103-105.
3. Hyypia, T., C. Horsnell, M. Maaronen, M. Khan, N. Kalkkinen, P. Auvinen, L. Kinnunen, and G. Stanway. 1992. A distinct picornavirus group identified by sequence analysis. Proc. Natl. Acad. Sci. U. S. A 89:8847-8851.
4. Ito, M., T. Yamashita, H. Tsuzuki, N. Takeda, and K. Sakae. 2004. Isolation and identification of a novel human parechovirus. J. Gen. Virol. 85:391-398.
5. Kimura, M. 1980. A simple method for estimating evolutionary rates of base substitutions through comparative studies of nucleotide sequences. J. Mol. Evol. 16:111-120.
6. Kumar, S., K. Tamura, and M. Nei. 1994. MEGA: Molecular Evolutionary Genetics Analysis software for microcomputers. Comput. Applic. Biosci. 10:189-191.
7. Lole, K. S., R. C. Bollinger, R. S. Paranjape, D. Gadkari, S. S. Kulkarni, N. G. Novak, R. Ingersoll, H. W. Sheppard, and S. C. Ray. 1999. Full-length human immunodeficiency virus type 1 genomes from subtype C-infected seroconverters in India, with evidence of intersubtype recombination. J. Virol. 73:152-160.
8. Oberste, M. S., K. Maher, and M. A. Pallansch. 1998. Complete sequence of echovirus 23 and its relationship to echovirus 22 and other human enteroviruses. Virus Res. 56:217-223.
9. Saitou, N. and M. Nei. 1987. The neighbor-joining method: a new method for reconstructing phylogenetic trees. Mol. Biol. Evol. 4:406-425.
10. Stanway, G. and T. Hyypia. 1999. Parechoviruses. J. Virol. 73:5249-5254.
11. Stanway, G., N. Kalkkinen, M. Roivainen, F. Ghazi, M. Khan, M. Smyth, O. Meurman, and T. Hyypia. 1994. Molecular and biological characteristics of echovirus 22, a representative of a new picornavirus group. J. Virol. 68:8232-8238.
12. Thompson, J. D., D. G. Higgins, and T. J. Gibson. 1994. Improved sensitivity of profile searches through the use of sequence weights and gap excision. Comput. Appl. Biosci. 10:19-29.
13. Heid CA, Stevens J, Livak KJ, Williams PM: Real time quantitative PCR. Genome Res. 6: 986-994, 1996
14. Tyagi S, Kramer FR: Molecular beacons: probes that fluoresce upon hybridization. Nat.Biotechnol. 14: 303-308, 1996
15. Leone G, van Schijndel H, Van Gemen B, Kramer FR, Schoen CD: Molecular beacon probes combined with amplification by NASBA enable homogeneous, real-time detection of RNA. Nucleic Acids Res. 26: 2150-2155, 1998

## Claims

1. An isolated or recombinant virus comprising a nucleic acid sequence as depicted in Figure 5, Figure 6, Figure 7 and/or Figure 8, or a functional part, derivative and/or analogue of said virus.

2. An isolated or recombinant virus comprising an amino acid sequence as depicted in Figure 5, Figure 6 and/or Figure 7, or a functional part, derivative and/or analogue of said virus.

3. An isolated or recombinant virus comprising a nucleic acid encoding an amino acid sequence as depicted in Figure 5, Figure 6 and/or Figure 7, or a functional part, derivative and/or analogue of said virus.

4. An isolated or recombinant virus comprising a nucleic acid sequence which is more than 71% homologous to a nucleic acid sequence as depicted in Figure 5, or a functional part, derivative and/or analogue of said virus.

5. An isolated or recombinant virus comprising a nucleic acid sequence which is more than 88.5% homologous to at least part of a nucleic acid sequence as depicted in Figure 5, said part having at least 25 nucleic acid residues, or a functional part, derivative and/or analogue of said virus.

6. An isolated or recombinant virus comprising a nucleic acid sequence which is more than 53.8% homologous to a nucleic acid sequence located from position 517 to 542 in the HPEV 251176 VP1 nucleic acid sequence as depicted in Figure 20, or a functional part, derivative and/or analogue of said virus.

7. An isolated or recombinant virus comprising a nucleic acid sequence which is more than 87.1% homologous to at least part of a nucleic acid sequence as depicted in Figure 5, said part having at least 30 nucleic acid residues, or a functional part, derivative and/or analogue of said virus.

8. An isolated or recombinant virus comprising a nucleic acid sequence which is more than 51.6% homologous to a nucleic acid sequence located from position 238 to 268 in the HPEV 251176 VP1 nucleic acid sequence as depicted in Figure 20, or a functional part, derivative and/or analogue of said virus.

9. An isolated or recombinant virus comprising a nucleic acid sequence which is more than 83.9% homologous to at least part of a nucleic acid sequence as depicted in Figure 5, said part having at least 50 nucleic acid residues, or a functional part, derivative and/or analogue of said virus.

10. An isolated or recombinant virus comprising a nucleic acid sequence which is more than 56.9% homologous to a nucleic acid sequence located from position 221 to 271 in the HPEV 251176 VP1 nucleic acid sequence as depicted in Figure 20, or a functional part, derivative and/or analogue of said virus.

11. An isolated or recombinant virus comprising a nucleic acid sequence which is more than 78.2% homologous to at least part of a nucleic acid sequence as depicted in Figure 5, said part having at least 100 nucleic acid residues, or a functional part, derivative and/or analogue of said virus.

12. An isolated or recombinant virus comprising a nucleic acid sequence which is more than 63.4% homologous to a nucleic acid sequence located from position 221 to 321 in the HPEV 251176 VP1 nucleic acid sequence as depicted in Figure 20, or a functional part, derivative and/or analogue of said virus.

13. An isolated or recombinant virus comprising a nucleic acid sequence which is more than 75% homologous to a nucleic acid sequence as depicted in Figure 7, or a functional part, derivative and/or analogue of said virus.

14. An isolated or recombinant virus comprising a nucleic acid sequence which is more than 96.2% homologous to at least part of a nucleic acid sequence as depicted in Figure 7, said part having at least 25 nucleic acid residues, or a functional part, derivative and/or analogue of said virus.

15. An isolated or recombinant virus comprising a nucleic acid sequence which is more than 34.6% homologous to a nucleic acid sequence located from position 75 to 100 in the HPEV 251176 VP0 nucleic acid sequence as depicted in Figure 14, or a functional part, derivative and/or analogue of said virus.

16. An isolated or recombinant virus comprising a nucleic acid sequence which is more than 93.3% homologous to at least part of a nucleic acid sequence as depicted in Figure 7, said part having at least 30 nucleic acid residues, or a functional part, derivative and/or analogue of said virus.

17. An isolated or recombinant virus comprising a nucleic acid sequence which is more than 41.9% homologous to a nucleic acid sequence located from position 75 to 105 in the HPEV 251176 VP0 nucleic acid sequence as depicted in Figure 14, or a functional part, derivative and/or analogue of said virus.

18. An isolated or recombinant virus comprising a nucleic acid sequence which is more than 90.4% homologous to at least part of a nucleic acid sequence as depicted in Figure 7, said part having at least 50 nucleic acid residues, or a functional part, derivative and/or analogue of said virus.

19. An isolated or recombinant virus comprising a nucleic acid sequence which is more than 52.9% homologous to a nucleic acid sequence located from position 66 to 110 in the HPEV 251176 VP0 nucleic acid sequence as depicted in Figure 14, or a functional part, derivative and/or analogue of said virus.

20. An isolated or recombinant virus comprising a nucleic acid sequence which is more than 89.1% homologous to at least part of a nucleic acid sequence as depicted in Figure 7, said part having at least 100 nucleic acid residues, or a functional part, derivative and/or analogue of said virus.

21. An isolated or recombinant virus comprising a nucleic acid sequence which is more than 64.4% homologous to a nucleic acid sequence located from position 39 to 139 in the HPEV 251176 VP0 nucleic acid sequence as depicted in Figure 14, or a functional part, derivative and/or analogue of said virus.

22. An isolated or recombinant virus comprising an amino acid sequence which is more than 79% homologous to an amino acid sequence as depicted in Figure 5, or a functional part, derivative and/or analogue of said virus.

23. An isolated or recombinant virus comprising an amino acid sequence which is more than 63% homologous to an amino acid sequence located from position 60 to 126 in the HPEV 251176 VP1 amino acid sequence depicted in Figure 15, or a functional part, derivative and/or analogue of said virus.

24. An isolated or recombinant virus comprising an amino acid sequence which is more than 40% homologous to an amino acid sequence located from position 60 to 69 in the HPEV 251176 VP1 amino acid sequence depicted in Figure 15, or a functional part, derivative and/or analogue of said virus.

25. An isolated or recombinant virus comprising an amino acid sequence which is more than 82% homologous to an amino acid sequence as depicted in Figure 7, or a functional part, derivative and/or analogue of said virus.

26. An isolated or recombinant virus comprising an amino acid sequence which is more than 74.7% homologous to an amino acid sequence located from position 123 to 205 in the HPEV 251176 VP0 amino acid sequence depicted in Figure 9, or a functional part, derivative and/or analogue of said virus.

27. An isolated or recombinant virus comprising an amino acid sequence which is more than 29.4% homologous to an amino acid sequence located from position 24 to 40 in the HPEV 251176 VP0 amino acid sequence depicted in Figure 9, or a functional part, derivative and/or analogue of said virus.

28. An isolated, synthetic or recombinant compound capable of specifically binding a virus or functional part, derivative or analogue according to any one of claims 1-27.

29. An isolated, synthetic or recombinant compound capable of specifically binding an amino acid sequence of a virus or functional part, derivative or analogue according to any one of claims 1-27.

30. An isolated, synthetic or recombinant compound capable of specifically binding a nucleic acid sequence of a virus or functional part, derivative or analogue according to any one of claims 1-27.

31. An isolated, synthetic or recombinant compound capable of specifically binding at least part of a nucleic acid sequence as depicted in Figure 5 and/or Figure 7.

32. An isolated, synthetic or recombinant compound capable of specifically binding at least part of an amino acid sequence as depicted in Figure 5 and/or Figure 7.

33. A compound according to any one of claims 28-32 which is a proteinaceous molecule.

34. A method for producing a compound capable of specifically binding a virus or functional part, derivative or analogue according to any one of claims 1-27, the method comprising:
- producing compounds capable of binding a virus or functional part, derivative or analogue according to any one of claims 1-27, and
- selecting a compound that is specific for said virus.

35. An isolated or recombinant virus which is immunoreactive with a compound according to any one of claims 28-33.

36. A primer and/or probe, capable of specifically hybridizing to a nucleic acid of a virus or functional part, derivative and/or analogue according to any one of claims 1-27 or 35.

37. A primer and/or probe, capable of specifically hybridizing to a nucleic acid sequence as depicted in Figure 5 and/or Figure 7.

38. A primer and/or probe according to claim 36 or 37, which is capable of hybridizing to said nucleic acid under stringent conditions.

39. A primer and/or probe according to any one of claims 36-38, comprising a sequence as depicted in Table 1.

40. An isolated, synthetic or recombinant nucleic acid sequence comprising at least part of a sequence as depicted in Figure 5 and/or Figure 7, or a complementary strand thereof, said part having at least 30 nucleic acid residues, or a functional part, derivative and/or analogue thereof.

41. An isolated, synthetic or recombinant nucleic acid sequence comprising a nucleic acid sequence that is at least 70% homologous to a nucleic acid sequence according to claim 40.

42. An isolated, synthetic and/or recombinant nucleic acid sequence comprising a stretch of at least 100 consecutive nucleotides of a nucleic acid sequence according to claim 40 or 41.

43. An amino acid sequence encoded by a nucleic acid sequence according to any one of claims 40-42.

44. An isolated, synthetic or recombinant amino acid sequence comprising at least part of a sequence as depicted in Figure 5, Figure 6 or Figure 7, said part having at least 30 amino acid residues, or a functional part, derivative and/or analogue thereof.

45. An isolated, synthetic or recombinant amino acid sequence comprising an amino acid sequence that is at least 80% homologous to an amino acid sequence according to claim 43 or 44.

46. A nucleic acid sequence encoding at least part of an amino acid sequence according to any one of claims 43-45, said part having at least 30 amino acids.

47. Use of a virus or functional part, derivative and/or analogue according to any one of claims 1-27 or 35, a compound according to any one of claims 28-33, a primer or probe according to any one of claims 36-39, and/or a nucleic acid sequence according to claims 40-42 or 46 for detecting a virus according to any one of claims 1-27 or 35 in a sample.

48. Use of a virus or functional part, derivative and/or analogue according to any one of claims 1-27 or 35, and/or an amino acid sequence according to any one of claims 43-45 for detecting a compound capable of specifically binding a virus according to any one of claims 1-27 or 35 in a sample.

49. Use according to claim 48, wherein said compound comprises a specific ligand and/or antibody of said virus.

50. Use according to claim 48 or 49, wherein said virus comprises Parechovirus 251176.

51. A virus or functional part, derivative or analogue according to any one of claims 1-27 or 35, a compound according to any one of claims 28-33, a nucleic acid sequence according to any one of claims 40-42 or 46, and/or an amino acid sequence according to any one of claims 43-45 for use as a vaccine or medicament.

52. Use of a virus or functional part, derivative or analogue according to any one of claims 1-27 or 35, a compound according to any one of claims 28-33, a nucleic acid sequence according to any one of claims 40-42 or 46, and/or an amino acid sequence according to any one of claims 43-45 for the preparation of a vaccine or medicament against a Parechoviral genus related disease.

53. Use of a virus or functional part, derivative and/or analogue according to any one of claims 1-27 or 35, a compound according to any one of claims 28-33, a primer or probe according to any one of claims 36-39, a nucleic acid sequence according to any one of claims 40-42 or 46, and/or an amino acid sequence according to any one of claims 43-45 for diagnosis of a Parechoviral genus related disease.

54. Use according to claim 52 or 53, wherein said Parechoviral genus related disease comprises a Parechovirus 251176 related disease.

55. An immunogenic composition comprising a virus or functional part, derivative or analogue according to any one of claims 1-27 or 35, a compound according to any one of claims 28-33, a nucleic acid sequence according to any one of claims 40-42 or 46, and/or an amino acid sequence according to any one of claims 43-45.

56. A medicament comprising a virus or functional part, derivative or analogue according to any one of claims 1-27 or 35, a compound according to any one of claims 28-33, a nucleic acid sequence according to any one of claims 40-42 or 46, and/or an amino acid sequence according to any one of claims 43-45.

57. A method for detecting a virus or functional part, derivative or analogue according to any one of claims 1-27 or 35 in a sample, comprising hybridizing and/or amplifying a nucleic acid of said virus or functional part, derivative or analogue with a primer and/or probe according to any one of claims 36-39 and/or a nucleic acid sequence according to any one of claims 40-42 or 46 and detecting hybridized and/or amplified product.

58. A diagnostic kit comprising a virus or functional part, derivative and/or analogue according to any one of claims 1-27 or 35, a compound according to any one of claims 28-33, a primer or probe according to any one of claims 36-39, a nucleic acid sequence according to any one of claims 40-42 or 46, and/or an amino acid sequence according to any one of claims 43-45.

59. A method for treating an individual suffering from, or at risk of suffering from, a Parechovirus 251176 related disease, comprising administering to said individual an immunogenic composition or medicament according to claim 55 or 56.

60. A method for determining whether an individual suffers from a Parechovirus 251176 related disease, comprising obtaining a sample from said individual and detecting a Parechovirus 251176 virus or functional part, derivative or analogue thereof in said sample with a method according to claim 57 and/or a diagnostic kit according to claim 58.

61. A vector and/or a gene delivery vehicle comprising a nucleic acid sequence according to any one of claims 40-42 or 46.

62. A virus according to any one of claims 1-27 or 35, comprising the isolate HPEV 251176 ECACC 05060901, or a functional part, derivative and/or analogue of said virus.

63. A virus derived from the isolate HPEV 251176 ECACC 05060901, or a functional part, derivative and/or analogue of said virus.

64. Composition of matter comprising isolated HPEV 251176 strain ECACC 05060901.
